# EUROPÄISCHE PATENTSCHRIFT

(11) **EP 0 584 046 B1**
(45) Veröffentlichungstag und Bekanntmachung des Hinweises auf die Patenterteilung: **07.10.1998**
(21) Anmeldenummer: 93810573.1
(22) Anmeldetag: 11.08.1993
(51) Int. Cl.: C07C 271/12, A01N 47/12, C07D 295/205, A01N 47/16, C07C 271/28, A01N 47/20, C07C 271/64, C07C 271/14, C07C 271/16, C07C 271/66, C07C 271/24, C07C 333/04, C07C 313/32, C07C 313/06, C07C 311/53, C07C 381/06, C07C 323/20, C07C 43/295, C07C 43/23, C07C 323/12

(54) **Carbamidsäurederivate und ihre Verwendung als Pestizide**
Carbamic acid amide derivatives and their use as pesticides
Dérivés d'amide carbamique et leur application comme pesticides

(30) Priorität: 20.08.1992 CH 2590/92
(43) Veröffentlichungstag der Anmeldung: 23.02.1994
(73) Patentinhaber: Novartis AG, 4058 Basel (CH)
(72) Erfinder: Karrer, Friedrich Dr., CH-4800 Zofingen (CH)

(56) Entgegenhaltungen:
- EP-A- 0 072 475
- EP-A- 0 129 513
- EP-A- 0 169 169
- EP-A- 0 460 472
- DE-A- 3 832 656
- US-A- 4 625 048

## Beschreibung

Die Erfindung betrifft Verbindungen der Formel 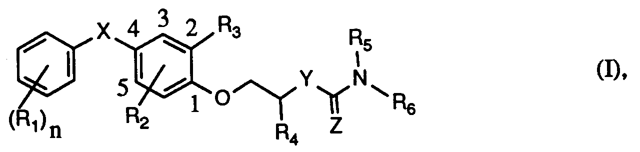 worin
R₁ Halogen, C₁-C₃-Alkyl, Halogen-C₁-C₃-alkyl, C₁-C₃-Alkoxy, Halogen-C₁-C₃-alkoxy oder Cyano und/oder zwei an benachbarte C-Atome des Phenylrings gebundene Substituenten R₁ gemeinsam -O-CH₂-O-;
R₂ Wasserstoff, Halogen oder Methyl;
R₃ Fluor, Chlor, Brom oder C₁-C₃-Alkyl;
R₄ Wasserstoff oder C₁-C₃-Alkyl;
entweder R₅ Wasserstoff, C₁-C₄-Alkyl, 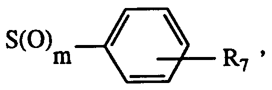 -COCO-R₈, -CO-R₉ oder S(O)ₘ-N(R₁₀)-COO-R₁₁ und
R₆ C₁-C₆-Alkyl, Halogen-C₁-C₄-alkyl, C₂-C₆-Alkenyl, Halogen-C₃-C₄-alkenyl, C₃-C₅-Alkinyl, Halogen-C₃-C₅-alkinyl, C₁-C₃-Alkoxy-C₁-C₃-alkyl, C₃-C₆-Cycloalkyl, unsubstituiertes Phenyl oder Phenyl welches ein- bis dreifach substituiert ist mit gleichen oder verschiedenen Substituenten ausgewählt aus der Gruppe bestehend aus Halogen, C₁-C₄-Alkyl und C₁-C₄-Alkoxy;
oder R₅ und R₆ gemeinsam -(CH₂)₄- oder -(CH₂)₅-;
R₇ Wasserstoff, Halogen oder C₁-C₃-Alkyl;
R₈ C₁-C₈-Alkoxy oder -N(R₁₂)₂;
R₉ C₁-C₄-Alkyl, C₃-C₆-Cycloalkyl, -N(R₁₃)₂ oder C₁-C₄-Alkoxy;
R₁₀ C₁-C₃-Alkyl;
R₁₁ C₁-C₆-Alkyl;
R₁₂ unabhängig voneinander C₁-C₈-Alkyl;
R₁₃ unabhängig voneinander C₁-C₄-Alkyl;
m die Zahl 0, 1 oder 2;
n die Zahl 0, 1, 2 oder 3, wobei, wenn n 2 oder 3 ist, die Reste R₁ gleich oder verschieden sein können;
X O, S, CH₂, CO oder -O-CH₂-;
Y O oder S; und
Z O oder S bedeuten,
mit Ausnahme von 1-(2-Fluor-4-phenoxy-phenoxy)-2-ethylaminocarbonyloxy-ethan, jeweils in freier Form oder in Salzform, ein Verfahren zur Herstellung und die Verwendung dieser Verbindungen, Schädlingsbekämpfungsmittel, deren Wirkstoff aus diesen Verbindungen, in freier Form oder in agrochemisch verwendbarer Salzform, ausgewählt ist, ein Verfahren zur Herstellung Verwendung dieser Mittel, mit diesen Mitteln behandeltes pflanzliches Vermehrungsgut, ein Verfahren zur Bekämpfung von Schädlingen, Zwischenprodukte, in freier Form oder in Salzform, zur Herstellung dieser Verbindungen und ein Verfahren zur Herstellung dieser Zwischenprodukte.

In der Literatur werden gewisse Carbamidsäurederivate als Wirkstoffe in Schädlingsbekämpfungsmitteln vorgeschlagen. So werden strukturell abweichende Carbamidsäureester zum Beispiel in US-A-4'625'048 und EP-A-0,072,475 genannt. Die biologischen Eigenschaften dieser bekannten Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften, insbesondere zur Bekämpfung von Insekten und Vertretern der Ordnung Akarina, zur Verfügung zu stellen, wobei diese Aufgabe erfindungsgemäss durch die Bereitstellung der vorliegenden Verbindungen der Formel I gelöst wird.

Verbindungen der Formel I, welche mindestens ein basisches Zentrum aufweisen, können Säureadditionssalze bilden. Diese werden beispielsweise mit starken anorganischen Säuren, wie Mineralsäuren, z. B. Perchlorsäure, Schwefelsäure, Salpetersäure, salpetrige Säure, einer Phosphorsäure oder einer Halogenwasserstoffsäure, mit starken organischen Carbonsäuren, wie gegebenenfalls, z. B. durch Halogen, substituierten C₁-C₄-Alkancarbonsäuren, z. B. Essigsäure, wie gegebenenfalls ungesättigten Dicarbonsäuren, z. B. Oxal-, Malon-, Bernstein-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten C₁-C₄-Alkan- oder Aryl-sulfonsäuren, z. B. Methan- oder p-Toluolsulfonsäure, gebildet. Verbindungen I mit mindestens einer aciden Gruppe können Salze mit Basen bilden. Geeignete Salze mit Basen sind beispielsweise Metallsalze, wie Alkali- oder Erdalkalimetallsalze, z. B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete Salze, die beispielsweise für die Isolierung bzw. Reinigung von freien Verbindungen der Formel I oder deren agrochemisch verwendbaren Salzen eingesetzt werden. Unter dem Ausdruck "Verbindungen der Formel I" sind somit vorstehend und nachfolgend sowohl die freien Verbindungen der Formel I als auch ihre Salze zu verstehen.

Die vor- und nachstehend verwendeten Allgemeinbegriffe haben, sofern nicht abweichend definiert, die nachfolgend aufgeführten Bedeutungen.

Halogen - als Substituent per se sowie als Strukturelement von anderen Gruppen und Verbindungen, wie von Halogenalkyl, Halogenalkoxy, Halogenalkenyl und Halogenalkinyl - ist Fluor, Chlor, Brom oder Iod, insbesondere Fluor, Chlor oder Brom, vor allem Fluor oder Chlor.

Kohlenstoffhaltige Gruppen und Verbindungen enthalten, sofern nicht abweichend definiert, jeweils 1 bis und mit 8, vorzugsweise 1 bis und mit 4, vor allem 1 bis und mit 3, insbesondere 1 oder 2, Kohlenstoffatome.

Cycloalkyl ist Cyclopropyl, Cyclobutyl, Cyclopentyl oder Cyclohexyl.

Alkyl - als Gruppe per se sowie als Strukturelement von anderen Gruppen und Verbindungen, wie von Halogenalkyl, Alkoxy, Halogenalkoxy und Alkoxyalkyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl, Butyl, Pentyl, Hexyl, Heptyl oder Octyl, oder verzweigt, z. B. Isopropyl, Isobutyl, sek.-Butyl, tert.-Butyl, Isopentyl, Neopentyl, Isohexyl oder Isooctyl.

Alkenyl und Alkinyl - als Gruppen per se sowie als Strukturelemente von anderen Gruppen und Verbindungen, wie von Halogenalkenyl und Halogenalkinyl, - sind geradkettig oder verzweigt und enthalten jeweils zwei oder vorzugsweise eine ungesättigte Kohlenstoff-Kohlenstoff-Bindung(en). Beispielhaft genannt seien Vinyl, Prop-1-en-3-yl, 2-Methylprop-1-en-3-yl, But-2-en-1-yl, But-2-en-3-yl, Prop-1-in-3-yl, But-2-in-1-yl und But-3-in-1-yl.

Halogensubstituierte kohlenstoffhaltige Gruppen und Verbindungen, wie Halogenalkyl, Halogenalkoxy, Halogenalkenyl und Halogenalkinyl, können teilweise halogeniert oder perhalogeniert sein, wobei im Falle von Mehrfach-Halogenierung die Halogensubstituenten gleich oder verschieden sein können. Beispiele für Halogenalkyl - als Gruppe per se sowie als Strukturelement von anderen Gruppen und Verbindungen, wie von Halogenalkoxy, - sind das ein- bis dreifach durch Fluor, Chlor und/oder Brom substituierte Methyl, wie CHF₂, CHCl₂, CH₂Cl oder CF₃; das ein- bis fünffach durch Fluor, Chlor und/oder Brom substituierte Ethyl, wie CH₂CH₂Cl, CH₂CH₂F, CHClCH₃, CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl, wie CH₂CHBrCH₂Br, CF₂CHFCF₃, CH₂CF₂CF₃, CF₂CF₂CF₃ oder CH(CF₃)₂; und das ein- bis neunfach durch Fluor, Chlor und/oder Brom substituierte Butyl oder eines seiner Isomeren, wie CF(CF₃)CHFCF₃, CF₂(CF₂)₂CF₃ oder CH₂(CF₂)₂CF₃. Beispiele für Halogenalkenyl sind 1-Chlorprop-1-en-3-yl, 2-Chlorprop-1-en-3-yl, 2,3-Dichlorprop-1-en-3-yl, 2,3-Dibromprop-1-en-3-yl, CH₂CH=CHCH₂Cl, CH₂CH=CHCH₂F und CH₂CH=CHCHF₂. Beispiele für Halogenalkinyl sind 2-Chlorprop-1-in-3-yl, 3,3-Dichlorprop-1-in-3-yl, 3,3-Dibromprop-1-in-3-yl, 3-Chlorprop-1-in-3-yl, 3-Fluorprop-1-in-3-yl und 4,4,4-Trifluorbut-2-in-1-yl.

In Alkoxyalkyl ist eine an den restlichen Teil der Verbindung der Formel I gebundene Alkylgruppe durch eine Alkoxygruppe substituiert, wobei beide Kohlenstoffketten unabhängig voneinander gerade oder verzweigt sein können; Beispiele sind Methoxymethyl, 1- und 2-Methoxyethyl, 1- und 2-Ethoxyethyl, Ethoxymethyl, Propoxymethyl, 2-Methoxyprop-1-yl und 2-Propoxyethyl.

Phenylreste sind unsubstituiert oder können einen bis drei Substituenten tragen, welche gleich oder verschieden sind. Beispiele sind 4-Chlorphenyl, 4-Methoxyphenyl, 4-Fluorphenyl, 3,4-Dichlorphenyl oder 3-Chlor-4-Methylphenyl.

Bevorzugte Ausführungsformen im Rahmen der Erfindung - unter Berücksichtigung der vorstehend genannten Massgabe - sind:
(1) Eine Verbindung der Formel I, worin
   R₁ Halogen, C₁-C₃-Alkyl, Halogen-C₁-C₃-alkyl, C₁-C₃-Alkoxy, Halogen-C₁-C₃-alkoxy oder Cyano und/oder zwei an benachbarte C-Atome des Phenylrings gebundene Substituenten R₁ gemeinsam -O-CH₂-O-;
   R₂ Wasserstoff, Halogen oder Methyl;
   R₃ Chlor, Brom oder C₁-C₃-Alkyl;
   R₄ Wasserstoff oder C₁-C₃-Alkyl;
   R₅ Wasserstoff, C₁-C₄-Alkyl, 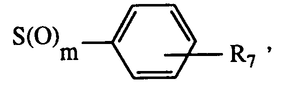 -COCO-R₈, -CO-R₉ oder S(O)ₘ-N(R₁₀)-COO-R₁₁;
   R₆ C₁-C₆-Alkyl; Halogen-C₁-C₄-alkyl; C₃-C₆-Alkenyl, Halogen-C₃-C₄-alkenyl, C₃-C₅-Alkinyl, Halogen-C₃-C₅-alkinyl, C₁-C₃-Alkoxy-C₁-C₃-alkyl oder C₃-C₆-Cycloalkyl;
   R₇ Wasserstoff, Halogen oder C₁-C₃-Alkyl;
   R₈ C₁-C₈-Alkoxy oder -N(R₁₂)₂;
   R₉ C₁-C₄-Alkyl, C₃-C₆-Cycloalkyl, -N(R₁₃)₂ oder C₁-C₄-Alkoxy;
   R₁₀ C₁-C₃-Alkyl;
   R₁₁ C₁-C₆-Alkyl;
   R₁₂ unabhängig voneinander C₁-C₈-Alkyl;
   R₁₃ unabhängig voneinander C₁-C₄-Alkyl;
   m die Zahl 0, 1 oder 2;
   n die Zahl 0, 1, 2 oder 3, wobei, wenn n 2 oder 3 ist, die Reste R₁ gleich oder verschieden sein können;
   X O, S, CH₂ oder CO;
   Y O oder S; und
   Z O oder S bedeuten;
(2) Eine Verbindung der Formel I, worin (R₁)ₙ(R₁)₀, Monofluor, Monochlor, Monobrom, Difluor, Dichlor, Monochlor-monofluor, Monobrom-monofluor, Monomethyl, Monoethyl, Dimethyl, Monomethoxy, Monocyano, Monotrifluormethyl, Monotrifluormethoxy oder Monomethylendioxy,
   insbesondere (R₁)₀, 2-, 3- oder 4-Fluor, 3- oder 4-Chlor, 4-Brom, 2,4- oder 3,5-Difluor, 3,4- oder 3,5-Dichlor, 3-Chlor-4-fluor, 4-Brom-2-fluor, 3- oder 4-Methyl, 3- oder 4-Ethyl, 3,5-Dimethyl, 3- oder 4-Methoxy, 4-Cyano, 3- oder 4-Trifluormethyl, 3- oder 4-Trifluor-methoxy oder 3,4-Methylendioxy,
   vor allem (R₁)₀, 3- oder 4-Fluor, 3-Chlor, 3,5-Difluor oder 3,4-Dichlor,
   insbesondere (R₁)₀, 3-Chlor oder 3,5-Difluor, ist;
(3) Eine Verbindung der Formel I, worin R₂ Wasserstoff, Chlor oder Methyl, insbesondere Wasserstoff, 5-Chlor oder 5-Methyl, vor allem Wasserstoff, ist;
(4) Eine Verbindung der Formel I, worin R₃ Chlor, Brom oder Methyl, insbesondere Chlor oder Brom, vor allem Chlor, ist;
(5) Eine Verbindung der Formel I, worin R₃ Fluor ist;
(6) Eine Verbindung der Formel I, worin R₄ Wasserstoff ist;
(7) Eine Verbindung der Formel I, worin R₅ Wasserstoff, C₁-C₄-Alkyl, - S-C₆H₄-Cl, -COCO-R₈ und R₈ C₁-C₄-Alkoxy, oder R₅ -CO-R₉ und R₉ C₁-C₄-Alkyl, C₁-C₄-Alkoxy oder C₃-C₆-Cycloalkyl ist,
   insbesondere worin R₅ Wasserstoff ist;
(8) Eine Verbindung der Formel I, worin R₆ C₁-C₆-Alkyl, Halogen-C₁-C₃-alkyl, C₂-C₄-Alkenyl, Halogen-C₃-C₄-alkenyl, C₃-C₄-Alkinyl, C₁-C₂-Alkoxy-C₁-C₂-alkyl oder C₃-C₆-Cycloalkyl, besonders C₁-C₃-Alkyl, Halogen-C₁-C₂-alkyl oder C₃-C₄-Alkenyl, vor allem C₁-C₂-Alkyl oder Chlor-C₁-C₂-alkyl, insbesondere Ethyl, ist;
(9) Eine Verbindung der Formel I, worin R₅ und R₆ gemeinsam -(CH₂)₄- oder -(CH₂)₅-bedeuten;
(10) Eine Verbindung der Formel I, worin X O, CH₂, CO oder -O-CH₂-, vor allem O, CH₂ oder CO,
   insbesondere O oder CH₂, vor allem O, ist;
(11) Eine Verbindung der Formel I, worin Y O ist;
(12) Eine Verbindung der Formel I, worin Z O ist;
(13) Eine Verbindung der Formel I, worin (R₁)ₙ (R₁)₀, 2-, 3- oder 4-Fluor, 3- oder 4-Chlor, 4-Brom, 2,4- oder 3,5-Difluor, 3,4- oder 3,5-Dichlor, 3-Chlor-4-fluor, 4-Brom-2-fluor, 3- oder 4-Methyl, 3- oder 4-Ethyl, 3,5-Dimethyl, 3- oder 4-Methoxy, 4-Cyano, 3- oder 4-Trifluormethyl, 3- oder 4-Trifluormethoxy oder 3,4-Methylendioxy, R₂ Wasserstoff, Chlor oder Methyl, R₃ Fluor, Chlor, Brom oder Methyl, R₄ Wasserstoff, entweder R₅ Wasserstoff, C₁-C₄-Alkyl 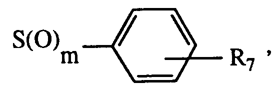 -COCO-R₈ oder -CO-R₉ und R₆ C₁-C₆-Alkyl, Halogen-C₁-C₃-alkyl, C₂-C₄-Alkenyl, C₃-C₄-Alkinyl, C₁-C₂-Alkoxy-C₁-C₂-alkyl oder einfach mit Halogen substituiertes Phenyl; oder R₅ und R₆ gemeinsam -(CH₂)₄- oder -(CH₂)₅-; X O, CH₂, CO oder -O-CH₂-; Y O oder S, Z O oder S, R₇ Wasserstoff oder Halogen; R₈ C₁-C₄-Alkoxy ; R₉ C₁-C₄-Alkyl oder C₃-C₆-Cycloalkyl und m 0 oder 1 bedeuten;
(14) Eine Verbindung der Formel I, worin (R₁)ₙ (R₁)₀, 3- oder 4-Fluor, 3-Chlor, 3,5-Difluor oder 3,4-Dichlor, R₂ Wasserstoff, R₃ Chlor, R₄ Wasserstoff, entweder R₅ Wasserstoff und R₆ C₁-C₃-Alkyl, Halogen-C₁-C₂-alkyl, C₂-C₄-Alkenyl, C₃-C₄-Alkinyl, C₁-C₂-Alkoxy-C₁-C₂-alkyl oder einfach mit Chlor substituiertes Phenyl; oder R₅ und R₆ gemeinsam -(CH₂)₄-; X O, CH₂, CO oder -O-CH₂-; Y O oder S und Z O oder S bedeuten;
(15) Eine Verbindung der Formel I, worin (R₁)ₙ (R₁)₀, R₂ Wasserstoff, R₃ Fluor, R₄ Wasserstoff, R₅ Wasserstoff, R₆ C₁-C₃-Alkyl und
   X, Y und Z jeweils O bedeuten;
(16) Eine Verbindung der Formel I, worin (R₁)ₙ (R₁)₀, 3-Chlor oder 3,5-Difluor, R₂ Wasserstoff, R₃ Chlor, R₄ Wasserstoff, R₅ Wasserstoff, R₆ C₁-C₂-Alkyl oder Chlor-C₁-C₂-alkyl und X, Y und Z jeweils O bedeuten.

Besonders bevorzugt sind im Rahmen der Erfindung die in den Beispielen H3 bis H9 genannten Verbindungen der Formel I.

Namentlich bevorzugt sind im Rahmen der Erfindung
(a) 1-[2-Chlor-4-(3-chlorphenoxy)-phenoxy]-2-ethylaminocarbonyloxy-ethan,
(b) 1-(2-Chlor-4-phenoxy-phenoxy)-2-ethylaminocarbonyloxy-ethan und
(c) 1-[2-Chlor-4-(3,5-difluorphenoxy)-phenoxy]-2-ethylaminocarbonyloxy-ethan.

Als weiterer Gegenstand der Erfindung ist das Verfahren zur Herstellung der Verbindungen der Formel I, mit Ausnahme von 1-(2-Fluor-4-phenoxy-phenoxy)-2-ethylaminocarbonyloxy-ethan, jeweils in freier Form oder in Salzform, z.B. dadurch gekennzeichnet, dass man
a) eine Verbindung der Formel 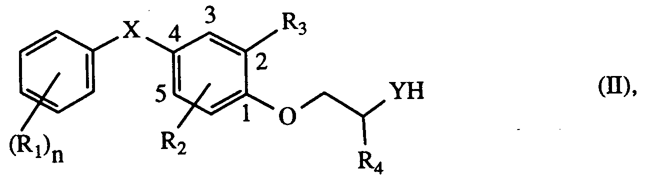 worin R₁, R₂, R₃, R₄, n, X und Y die für die Formel I angegebenen Bedeutungen haben, oder ein Salz davon, vorzugsweise in Gegenwart einer Base, mit einer Verbindung der Formel

   L-C(=Z)-N(R₅)R₆ (III),

   die bekannt ist oder in Analogie zu entsprechenden bekannten Verbindungen hergestellt werden kann und worin R₅, R₆ und Z die für die Formel I angegebenen Bedeutungen haben und L für eine Abgangsgruppe steht, oder einem Salz davon umsetzt, oder
b) zur Herstellung einer Verbindung der Formel I, worin R₅ Wasserstoff ist, oder eines Salzes davon eine Verbindung der Formel (II) oder ein Salz davon, vorzugsweise in Gegenwart eines Acylierungskatalysators, mit einer Verbindung der Formel

   R₆N=C=Z (IV),

   die bekannt ist oder in Analogie zu entsprechenden bekannten Verbindungen hergestellt werden kann und worin R₆ und Z die für die Formel I angegebenen Bedeutungen haben, umsetzt, oder
c) eine Verbindung der Formel 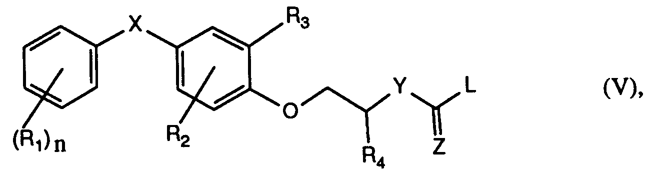 worin R₁, R₂, R₃, R₄, n, X, Y und Z die für die Formel I angegebenen Bedeutungen haben und L für eine Abgangsgruppe steht, oder ein Salz davon, vorzugsweise in Gegenwart einer Base, mit einer Verbindung der Formel

   H-N(R₅)R₆ (VI),

   die bekannt ist oder in Analogie zu entsprechenden bekannten Verbindungen hergestellt werden kann und worin R₅ und R₆ die für die Formel I angegebenen Bedeutungen haben, oder einem Salz davon umsetzt, oder
d) eine Verbindung der Formel 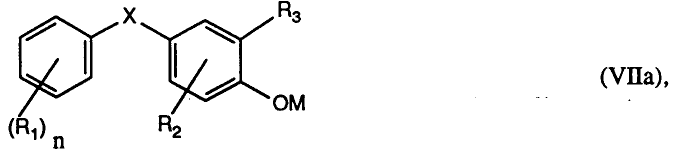 die bekannt ist oder in Analogie zu entsprechenden bekannten Verbindungen hergestellt werden kann und worin R₁, R₂, R₃, n und X die für die Formel I angegebenen Bedeutungen haben und M für ein Kation, vorzugsweise ein Alkalimetallion, steht, mit einer Verbindung der Formel 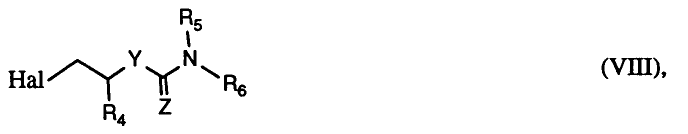 die bekannt ist oder in Analogie zu entsprechenden bekannten Verbindungen hergestellt werden kann und worin R₄, R₅, R₆, Y und Z die in Formel I angegebene Bedeutung besitzen und Hal ein Halogenatom, vorzugsweise Brom, bedeutet, vorzugsweise in Gegenwart einer Base umsetzt,
   und/oder, wenn erwünscht, eine verfahrensgemäss oder auf andere Weise erhältliche Verbindung der Formel I, in freier Form oder in Salzform, in eine andere Verbindung der Formel I überführt, ein verfahrensgemäss erhältliches Gemisch von Isomeren auftrennt und das gewünschte Isomere isoliert und/oder eine verfahrensgemass oder auf andere Weise erhältliche freie Verbindung der Formel I in ein Salz oder ein verfahrensgemäss oder auf andere Weise erhältliches Salz einer Verbindung der Formel I in die freie Verbindung der Formel I oder in ein anderes Salz überführt.

Für vor- und nachstehend aufgeführte Ausgangsmaterialien gilt im Hinblick auf deren Salze das vorstehend für Salze von Verbindungen der Formel I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs-oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsgemisches, vorzugsweise von etwa -20°C bis etwa +150°C, und, falls erforderlich, in einem geschlossenen Gefäss, unter erhöhtem oder vermindertem Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen der Formel I, in freier Form oder in Salzform, verwendet werden, sind bekannt oder können nach an sich bekannten Methoden, z. B. gemäss den nachstehenden Angaben, hergestellt werden.

### Variante a):

Geeignete Abgangsgruppen L in den Verbindungen der Formel III sind z. B. Hydroxy, C₁-C₈-Alkoxy, Halogen-C₁-C₈-alkoxy, C₁-C₈-Alkanoyloxy, Mercapto, C₁-C₈-Alkylthio, Halogen-C₁-C₈-alkylthio, C₁-C₈-Alkansulfonyloxy, Halogen-C₁-C₈-alkansulfonyloxy, Benzolsulfonyloxy, Toluolsulfonyloxy und Halogen.

Geeignete Basen zur Erleichterung der HL-Abspaltung sind z. B. Alkalimetall- oder Erdalkalimetall-hydroxide, -hydride, -amide, -alkanolate, -acetate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, -methanolat, -acetat, -carbonat, Kalium-tert.-butanolat, -hydroxid, -carbonat, -hydrid, Lithiumdiisopropylamid, Kalium-bis(trimethylsilyl)-amid, Calciumhydrid, Triethylamin, Diisopropyl-ethyl-amin, Triethylendiamin, Cyclohexylamin, N-Cyclohexyl-N,N-dimethyl-amin, N,N-Diethylanilin, Pyridin, 4-(N,N-Dimethylamino)pyridin, 4-Pyrrolidin-1-ylpyridin, Chinuclidin, N-Methylmorpholin, Benzyltrimethyl-ammoniumhydroxid sowie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa - 20°C bis etwa +180°C, bevorzugt von etwa -10°C bis etwa +130°C, in vielen Fällen im Bereich zwischen etwa 0°C und der Rückflusstemperatur des Reaktionsgemisches.

### Variante b):

Geeignete Acylierungskatalysatoren sind z. B. tertiäre organische Basen und zinnorganische Verbindungen. Als tertiäre organische Basen kommen z. B. tertiäre Amine und tertiäre basische Heterocyclen, wie Trimethylamin, Triethylamin, Diisopropyl-ethyl-amin, Tetramethylethylendiamin, N-Cyclohexyl-N,N-dimethyl-amin, N,N-Diethylanilin, Pyridin, 4-(N,N-Dimethylamino)pyridin, 4-Pyrrolidin- 1-ylpyridin, N-Methylmorpholin, Chinuclidin, 1,5-Diazabicyclo[5.4.0]undec-5-en (DBU), 1,4-Diazabicyclo[2.2.2]octan und Gemische davon, und als zinnorganische Verbindungen z. B. Dialkylzinndialkanoate, wie Dibutylzinndiacetat, in Betracht.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäureethylester; Ether, wie Diethylether, Dipropylether, Diisopropylether, Dibutylether, tert.-Butylmethylether, Ethylenglykoldimethylether, Dimethoxydiethylether, Tetrahydrofuran oder Dioxan; Ketone, wie Aceton, Methylethylketon oder Methylisobutylketon; Amide, wie N,N-Dimethylformamid, N,N-Diethylformamid, N,N-Dimethylacetamid, N-Methylpyrrolidon oder Hexamethylphosphorsäuretriamid; Nitrile, wie Acetonitril oder Propionitril; und Sulfoxide, wie Dimethylsulfoxid. Wird die Umsetzung in Gegenwart einer tertiären organischen Base ausgeführt, können auch im Ueberschuss eingesetzte Basen, wie Triethylamin, Pyridin, N-Methylmorpholin oder N,N-Diethylanilin, als Lösungs- oder Verdünnungsmittel dienen.

Die Umsetzung erfolgt vorteilhaft in einem Temperaturbereich von etwa -20°C bis etwa +180°C, bevorzugt von etwa -10°C bis etwa +130°C, in vielen Fällen im Bereich zwischen etwa 0°C und der Rückflusstemperatur des Reaktionsgemisches.

Gemäss einer bevorzugten Ausführungsform der Variante b) arbeitet man in Gegenwart eines Acylierungskatalysators, z. B. eines Trialkylamins, wie in Gegenwart von Triethylamin, und/oder einer bicyclischen organischen Base, wie in Gegenwart von 1,4-Diazabicyclo[2.2.2]octan, in einem Ether, wie Tetrahydrofuran, und bei einer Temperatur zwischen Raumtemperatur und 80°C.

### Variante c):

Geeignete Abgangsgruppen L in den Verbindungen V sind z. B. von der unter der Variante a) angegebenen Art.

Geeignete Basen zur Erleichterung der HL-Abspaltung sind z. B. von der unter der Variante a) angegebenen Art.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Geeignete Lösungs- oder Verdünnungsmittel sind z. B. von der unter der Variante a) angegebenen Art.

Die Umsetzung erfolgt vorteilhaft in einem Temperaturbereich von etwa - 20°C bis etwa +180°C, bevorzugt von etwa -10°C bis etwa +130°C, in vielen Fällen im Bereich zwischen etwa 0°C und der Rückflusstemperatur des Reaktionsgemisches.

### Variante d):

Geeignete Kationen in den Verbindungen der Formel VIIa sind z.B. solche von Alkali- und Erdalkalimetallen, vorzugsweise von Alkalimetallen, insbesondere Natrium- und Kaliumionen.

Geeignete Basen zur Bildung einer Verbindung der Formel VIIa aus einer Verbindung der Formel 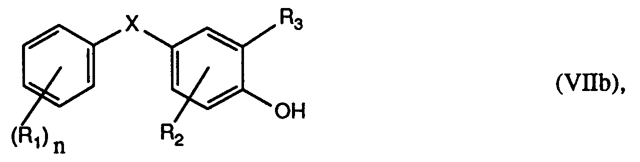 die bekannt ist oder in Analogie zu entsprechenden bekannten Verbindungen hergestellt werden kann und worin R₁, R₂, R₃, n und X die für die Formel I angegebenen Bedeutungen haben, sind z. B. von der unter der Variante a) angegebenen Art, insbesondere kommen Natrium-hydroxid, -hydrid, -amid, -methanolat, -acetat, -carbonat, Kalium-tert.-butanolat, -hydroxid, -carbonat, -hydrid, Lithiumdiisopropylamid, Kalium-bis-(trimethylsilyl)-amid, Calciumhydrid, besonders bevorzugt Natriummethanolat und Kalium-tert.-butanolat, in Frage. Es ist auch möglich, eine Verbindung der Formel VIIb mit einer geeigneten Base in ein Salz der Formel VIIa überzuführen und in situ zu einer Verbindung der Formel I weiter zu verarbeiten. Dabei wird die Base vorzugsweise im Ueberschuss eingesetzt, aber auch ein stöchiometrisches Mengenverhältnis ist möglich.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Geeignete Lösungs- oder Verdünnungsmittel sind z. B. von der unter der Variante a) angegebenen Art.

Die Umsetzung erfolgt vorteilhaft in einem Temperaturbereich von etwa -20°C bis etwa +180°C, bevorzugt von etwa -10°C bis etwa +130°C, in vielen Fällen im Bereich zwischen etwa 20°C und der Rückflusstemperatur des Reaktionsgemisches.

Die in der Variante c) als Edukte eingesetzten Verbindungen V, in freier Form oder in Salzform, können in Analogie zu bekannten Verfahren hergestellt werden, z. B. durch Umsetzung einer Verbindung der Formel II oder eines Salzes davon, vorzugsweise in analoger Weise wie unter der Variante a) für die Umsetzung einer Verbindung der Formel II mit einer Verbindung der Formel III beschrieben, mit einer Verbindung der Formel

Z=C(L₁)L₂ (IX),

die bekannt ist oder in Analogie zu entsprechenden bekannten Verbindungen hergestellt werden kann und worin Z die für die Formel I angegebene Bedeutung hat und L₁ und L₂ unabhängig voneinander jeweils für eine Abgangsgruppe stehen, wobei solche Abgangsgruppen z. B. von der unter der Variante a) für Abgangsgruppen L angegebenen Art sind.

Gemäss der Variante c) ist es auch möglich, die Zwischenprodukte V, in freier Form oder in Salzform, in situ aus den Verbindungen der Formel II und IX herzustellen und ohne Isolierung, also im Sinne eines "Eintopfverfahrens", mit einer Verbindung der Formel VI oder einem Salz davon zu den Verbindungen der Formel I weiterumzusetzen.

Die in den Varianten a) und b) als Edukte für die Herstellung der Verbindungen der Formel I und in der Variante c) als Edukte für die Herstellung der Zwischenprodukte der Formel V eingesetzten Verbindungen der Formel II, mit Ausnahme von 2-(2-Fluor-4-phenoxy-phenoxy)ethanol, jeweils in freier Form oder in Salzform, sind neu und bilden ebenfalls einen Gegenstand der Erfindung. Besonders bevorzugt sind im Rahmen der Erfindung die in den Beispielen H1 und H9 genannten Verbindungen der Formel II.

Als weiterer Gegenstand der Erfindung ist das Verfahren zur Herstellung der Verbindungen der Formel II, mit Ausnahme von 2-(2-Fluor-4-phenoxy-phenoxy)ethanol, jeweils in freier Form oder in Salzform, z.B. dadurch gekennzeichnet, dass man
e) eine Verbindung der Formel (VIIa) oder (VIIb), vorzugsweise in einem inerten Lösungs- oder Verdünnungsmittel, z.B. einem Lösungs- oder Verdünnungsmittel von der unter der Variante b) angegebenen Art, und in Gegenwart eines Alkylierungskatalysators, z. B. eines tertiären Amins, wie in Gegenwart von Triethylamin, mit einer Verbindung der Formel 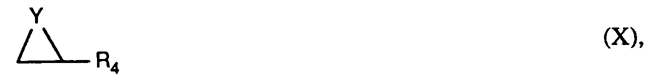 die bekannt ist oder in Analogie zu entsprechenden bekannten Verbindungen hergestellt werden kann und worin R₄ und Y die für die Formel I angegebenen Bedeutungen haben, umsetzt oder
f) zur Herstellung einer Verbindung der Formel II, worin R₄ Wasserstoff und Y O ist, oder eines Salzes davon eine Verbindung der Formel (VIIa) oder (VIIb), vorzugsweise in der Schmelze und in Gegenwart eines Alkylierungskatalysators, z. B. eines quaternären Ammoniumsalzes, wie in Gegenwart von Tetraethylammoniumchlorid, mit 2-Oxo-1,3-dioxolan umsetzt, oder
g) zur Herstellung einer Verbindung der Formel II, worin R₄ Wasserstoff und Y O ist, oder eines Salzes davon eine Verbindung der Formel 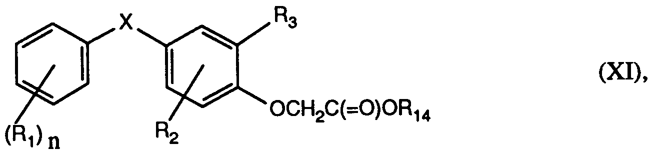 die bekannt ist oder in Analogie zu entsprechenden bekannten Verbindungen hergestellt werden kann und worin R₁, R₂, R₃, n und X die für die Formel I angegebenen Bedeutungen haben und R₁₄ C₁-C₈-Alkyl ist, vorzugsweise in einem inerten Lösungs- oder Verdünnungsmittel, z. B. in einem Ether, wie Diethylether oder Tetrahydrofuran, mit einem Reduktionsmittel, z. B. mit einem komplexen Metallhydrid, wie Lithiumaluminiumhydrid, umsetzt
   und/oder, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel II in ein Salz oder ein verfahrensgemäss oder auf andere Weise erhältliches Salz einer Verbindung der Formel II in die freie Verbindung der Formel II oder in ein anderes Salz überführt.

Eine verfahrensgemäss oder auf andere Weise erhältliche Verbindung der Formel I bzw. II kann in an sich bekannter Weise in eine andere Verbindung der Formel I bzw. II überführt werden, indem man einen oder mehrere Substituenten der Ausgangsverbindung der Formel I bzw. II in üblicher Weise durch (einen) andere(n) erfindungsgemässe(n) Substituenten ersetzt.

Beispielsweise können:
- in den Verbindungen der Formel I Wasserstoffsubstituenten R₅ gegen Alkyl-, Mercapto-, Sulfinyl-, Sulfonyl- oder Carbonyl-Gruppen R₅ ausgetauscht werden;
- in den Verbindungen der Formel I Mercaptogruppen R₅ zu Sulfinyl- oder Sulfonylgruppen R₅ oder Sulfinylgruppen R₅ zu Sulfonylgruppen R₅ oxidiert werden; oder
- Verbindungen der Formel II, worin Y O ist, in Verbindungen der Formel II, worin Y S ist, überführt werden.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden.

Salze von Verbindungen der Formel I bzw. II können in an sich bekannter Weise hergestellt werden. So erhält man beispielsweise Säureadditionssalze von Verbindungen der Formeln I bzw. II durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen der Formeln I bzw. II können in üblicher Weise in die freien Verbindungen der Formeln I bzw. II überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen der Formeln I bzw. II können in an sich bekannter Weise in andere Salze von Verbindungen der Formeln I bzw. II umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z. B. Silberchlorid, unlöslich ist und damit aus dem Reaktionsgemisch ausscheidet.

Je nach Verfahrensweise bzw. Reaktionsbedingungen können die Verbindungen der Formeln I bzw. II mit salzbildenden Eigenschaften in freier Form oder in Form von Salzen erhalten werden.

Die Verbindungen der Formeln I bzw. II, in freier Form oder in Salzform, können in Form eines der möglichen Isomeren oder als Gemisch derselben, z. B. je nach Anzahl, absoluter und relativer Konfiguration von im Molekül auftretenden asymmetrischen Kohlenstoffatomen und/oder je nach Konfiguration von im Molekül auftretenden nichtaromatischen Doppelbindungen, als reine Isomere, wie Antipoden und/oder Diastereomere, oder als Isomerengemische, wie Enantiomerengemische, z. B. Racemate, Diastereomerengemische oder Racematgemische, vorliegen; die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen der Formeln I bzw. II, in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie einer Carbonsäure, z. B. Campher-, Wein- oder Äpfelsäure, oder einer Sulfonsäure, z. B. Camphersulfonsäure, und Trennung des auf diese Weise erhaltenen Diastereomerengemisches, z.B. auf Grund ihrer verschiedenen Löslichkeiten durch fraktionierte Kristallisation, in die Diastereomeren, aus denen das gewünschte Enantiomere durch Einwirkung geeigneter, z.B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere oder Diastereomere, oder Isomerengemisch, z. B. Enantiomerengemisch oder Diastereomerengemisch, sofern die einzelnen Komponenten unterschiedliche biologische Wirksamkeit besitzen.

Die Verbindungen der Formeln I bzw. II, in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen der Formel I bzw. deren Salzen führen.

Die Erfindung betrifft insbesondere die in den Beispielen H1 bis H8 beschriebenen Herstellungsverfahren.

Erfindungsgemäss für die Herstellung der Verbindungen der Formel I bzw. ihrer Salze verwendete Ausgangsstoffe und Zwischenprodukte, jeweils in freier Form oder in Salzform, die neu sind, ein Verfahren zu ihrer Herstellung und ihre Verwendung als Ausgangsstoffe und Zwischenprodukte für die Herstellung der Verbindungen der Formel I sind ebenfalls Gegenstand der Erfindung; insbesondere betrifft dies die Verbindungen der Formel II.

Die erfindungsgemassen Verbindungen der Formel I sind auf dem Gebiet der Schädlingsbekämpfung bei günstiger Warmblüter-, Fisch- und Pflanzenverträglichkeit bereits bei niedrigen Anwendungskonzentrationen präventiv und/oder kurativ wertvolle Wirkstoffe mit einem sehr günstigen bioziden Spektrum. Die erfindungsgemässen Wirkstoffe sind gegen alle oder einzelne Entwicklungsstadien von normal sensiblen, aber auch von resistenten, tierischen Schädlingen, wie Insekten und Vertetern der Ordnung Akarina, wirksam. Die insektizide und/oder akarizide Wirkung der erfindungsgemässen Wirkstoffe kann sich dabei direkt, d. h. in einer Abtötung der Schädlinge, welche unmittelbar oder erst nach einiger Zeit, beispielsweise bei einer Häutung, eintritt, oder indirekt, z. B. in einer verminderten Eiablage und/oder Schlupfrate, zeigen, wobei die gute Wirkung einer Abtötungsrate (Mortalität) von mindestens 50 bis 60% entspricht.

Zu den erwähnten tierischen Schädlingen gehören beispielsweise:
aus der Ordnung Lepidoptera zum Beispiel
   Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
   Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Oryzaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
   Blatta spp., Blattella spp., Gryllotalpa spp., Leucophaea maderae, Locusta spp., Periplaneta spp. und Schistocerca spp.;
aus der Ordnung Isoptera zum Beispiel
   Reticulitermes spp.;
aus der Ordnung Psocoptera zum Beispiel
   Liposcelis spp.;
aus der Ordnung Anoplura zum Beispiel
   Haematopinus spp., Linognathus spp., Pediculus spp., Pemphigus spp. und Phylloxera spp.;
aus der Ordnung Mallophaga zum Beispiel
   Damalinea spp. und Trichodectes spp.;
aus der Ordnung Thysanoptera zum Beispiel
   Frankliniella spp., Hercinothrips spp., Taeniothrips spp., Thrips palmi, Thrips tabaci und Scirtothrips aurantii;
aus der Ordnung Heteroptera zum Beispiel
   Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
   Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma lanigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
   Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
   Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.; aus der Ordnung Siphonaptera zum Beispiel Ceratophyllus spp. und Xenopsylla cheopis;
aus der Ordnung Thysanura zum Beispiel
   Lepisma saccharina und
aus der Ordnung Acarina zum Beispiel
   Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Mit den erfindungsgemässen Wirkstoffen kann man insbesondere an Pflanzen, vor allem an Nutz- und Zierpflanzen in der Landwirtschaft, im Gartenbau und im Forst, oder an Teilen, wie Früchten, Blüten, Laubwerk, Stengeln, Knollen oder Wurzeln, solcher Pflanzen auftretende Schädlinge des erwähnten Typus bekämpfen, d. h. eindämmen oder vernichten, wobei zum Teil auch später zuwachsende Pflanzenteile noch gegen diese Schädlinge geschützt werden.

Als Zielkulturen kommen insbesondere Getreide, wie Weizen, Gerste, Roggen, Hafer, Reis, Mais oder Sorghum; Rüben, wie Zucker- oder Futterrüben; Obst, z. B. Kern-, Stein- und Beerenobst, wie Aepfel, Birnen, Pflaumen, Pfirsiche, Mandeln, Kirschen oder Beeren, z. B. Erdbeeren, Himbeeren oder Brombeeren; Hülsenfrüchte, wie Bohnen, Linsen, Erbsen oder Soja; Oelfrüchte, wie Raps, Senf, Mohn, Oliven, Sonnenblumen, Kokos, Rizinus, Kakao oder Erdnüsse; Gurkengewächse, wie Kürbisse, Gurken oder Melonen; Fasergewächse, wie Baumwolle, Flachs, Hanf oder Jute; Citrusfrüchte, wie Orangen, Zitronen, Pampelmusen oder Mandarinen; Gemüse, wie Spinat, Kopfsalat, Spargel, Kohlarten, Möhren, Zwiebeln, Tomaten, Kartoffeln oder Paprika; Lorbeergewächse, wie Avocado, Cinnamonium oder Kampfer; sowie Tabak, Nüsse, Kaffee, Eierfrüchte, Zuckerrohr, Tee, Pfeffer, Weinreben, Hopfen, Bananengewächse, Naturkautschukgewächse und Zierpflanzen in Betracht.

Die erfindungsgemässen Wirkstoffe eignen sich besonders zur Bekämpfung von Adoxo-phyes reticulana, Cydia pomonella, Heliothis virescens, Lobesia botrana und Nilaparvata lugens in Citrus-, Obst- und Reis-Kulturen.

Weitere Anwendungsgebiete der erfindungsgemässen Wirkstoffe sind der Schutz von Vorräten und Lagern und von Material sowie im Hygienesektor insbesondere der Schutz von Haus- und Nutztieren vor Schädlingen des erwähnten Typus.

Die Erfindung betrifft daher auch Schädlingsbekämpfungsmittel, wie, je nach angestrebten Zielen und gegebenen Verhältnissen zu wählende, emulgierbare Konzentrate, Suspensionskonzentrate, direkt versprüh- oder verdünnbare Lösungen, streichfähige Pasten, verdünnte Emulsionen, Spritzpulver, lösliche Pulver, dispergierbare Pulver, benetzbare Pulver, Stäubemittel, Granulate oder Verkapselungen in polymeren Stoffen, welche - mindestens - einen der erfindungsgemässen Wirkstoffe enthalten.

Der Wirkstoff wird in diesen Mitteln in reiner Form, ein fester Wirkstoff z. B. in einer speziellen Korngrösse, oder vorzugsweise zusammen mit - mindestens - einem der in der Formulierungstechnik üblichen Hilfsstoffe, wie Streckmitteln, z. B. Lösungsmitteln oder festen Trägerstoffen, oder wie oberflächenaktiven Verbindungen (Tensiden), eingesetzt.

Als Lösungsmittel können z. B. in Frage kommen: gegebenenfalls partiell hydrierte aromatische Kohlenwasserstoffe, bevorzugt die Fraktionen C₈ bis C₁₂ von Alkylbenzolen, wie Xylolgemische, alkylierte Naphthaline oder Tetrahydronaphthalin, aliphatische oder cycloaliphatische Kohlenwasserstoffe, wie Paraffine oder Cyclohexan, Alkohole, wie Ethanol, Propanol oder Butanol, Glykole sowie deren Ether und Ester, wie Propylenglykol, Dipropylenglykolether, Ethylenglykol oder Ethylenglykolmono-methyl- oder -ethyl-ether, Ketone, wie Cyclohexanon, Isophoron oder Diacetonalkohol, stark polare Lösungsmittel, wie N-Methylpyrrolid-2-on, Dimethylsulfoxid oder N,N-Dimethylformamid, Wasser, gegebenenfalls epoxidierte Pflanzenöle, wie gegebenenfalls epoxidiertes Raps-, Rizinus-, Kokosnuss- oder Sojaöl, und Silikonöle.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, und als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen, je nach Art des zu formulierenden Wirkstoffs, nichtionische, kationische und/oder anionische Tenside oder Tensidgemische mit guten Emulgier-, Dispergier- und Netzeigenschaften in Betracht. Die nachstehend aufgeführten Tenside sind dabei nur als Beispiele anzusehen; in der einschlägigen Literatur werden viele weitere in der Formulierungstechnik gebräuchliche und erfindungsgemäss geeignete Tenside beschrieben.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind wasserlösliche, 20 bis 250 Ethylenglykolether- und 10 bis 100 Propylenglykolether-gruppen enthaltende, Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten. Als Beispiele seien Nonylphenolpolyethoxyethanole, Ricinusölpolyglykolether, Polypropylen-Polyethylenoxid-Addukte, Tributylphenoxypolyethoxyethanol, Polyethylenglykol und Octylphenoxypolyethoxyethanol erwähnt. Ferner kommen Fettsäureester von Polyoxyethylensorbitan, wie das Polyoxyethylensorbitan-trioleat, in Betracht.

Bei den kationischen Tensiden handelt es sich vor allem um quarternäre Ammoniumsalze, welche als Substituenten mindestens einen Alkylrest mit 8 bis 22 C-Atomen und als weitere Substituenten niedrige, gegebenenfalls halogenierte, Alkyl-, Benzyl- oder niedrige Hydroxyalkylreste aufweisen. Die Salze liegen vorzugsweise als Halogenide, Methylsulfate oder Ethylsulfate vor. Beispiele sind das Stearyl-trimethyl-ammoniumchlorid und das Benzyl-di-(2-chlorethyl)-ethyl-ammoniumbromid.

Geeignete anionische Tenside können sowohl wasserlösliche Seifen als auch wasserlösliche synthetische oberflächenaktive Verbindungen sein. Als Seifen eignen sich die Alkali-, Erdalkali- und gegebenenfalls substituierten Ammoniumsalze von höheren Fettsäuren (C₁₀-C₂₂), wie die Natrium- oder Kalium-Salze der Oel- oder Stearinsäure, oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch synthetische Tenside verwendet, insbesondere Fettsulfonate, Fettsulfate, sulfonierte Benzimidazolderivate oder Alkylarylsulfonate. Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäure-esters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8 bis 22 C-Atomen. Alkylarylsulfonate sind zum Beispiel die Natrium-, Calcium- oder Triethanolammoniumsalze der Dodecylbenzolsulfonsäure, der Dibutylnaphthalinsulfonsäure oder eines Naphthalinsulfonsäure-Formaldehyd-Kondensationsproduktes. Ferner kommen auch entsprechende Phosphate, wie Salze des Phosphorsäureesters eines p-Nonylphenol-(4-14)-Ethylenoxid-Adduktes oder Phospholipide, in Frage.

Die Mittel enthalten in der Regel 0,1 bis 99%, insbesondere 0,1 bis 95%, Wirkstoff und 1 bis 99,9%, insbesondere 5 bis 99,9%, - mindestens - eines festen oder flüssigen Hilfsstoffes, wobei in der Regel 0 bis 25%, insbesondere 0,1 bis 20%, der Mittel Tenside sein können (% bedeutet jeweils Gewichtsprozent). Während als Handelsware eher konzentrierte Mittel bevorzugt werden, verwendet der Endverbraucher in der Regel verdünnte Mittel, die wesentlich geringere Wirkstoffkonzentrationen aufweisen. Bevorzugte Mittel setzen sich insbesondere folgendermassen zusammen (% = Gewichtsprozent):

| Emulgierbare Konzentrate: | |
|---|---|
| Wirkstoff: | 1 bis 90%, vorzugsweise 5 bis 20% |
| Tensid: | 1 bis 30%, vorzugsweise 10 bis20 % |
| Lösungsmittel: | 5 bis 98%, vorzugsweise 70 bis 85% |

| Stäubemittel: | |
|---|---|
| Wirkstoff: | 0,1 bis 10%, vorzugsweise 0,1 bis 1% |
| fester Trägerstoff: | 99,9 bis 90%, vorzugsweise 99,9 bis 99% |

| Suspensionskonzentrate: | |
|---|---|
| Wirkstoff: | 5 bis 75%, vorzugsweise 10 bis 50% |
| Wasser: | 94 bis 24%, vorzugsweise 88 bis 30% |
| Tensid: | 1 bis 40%, vorzugsweise 2 bis 30% |

| Benetzbare Pulver: | |
|---|---|
| Wirkstoff: | 0,5 bis 90%, vorzugsweise 1 bis 80% |
| Tensid: | 0,5 bis 20%, vorzugsweise 1 bis 15% |
| fester Trägerstoff: | 5 bis 99%, vorzugsweise 15 bis 98% |

| Granulate: | |
|---|---|
| Wirkstoff: | 0,5 bis 30%, vorzugsweise 3 bis 15% |
| fester Trägerstoff: | 99,5 bis 70%, vorzugsweise 97 bis 85% |

Die Wirkung der erfindungsgemässen Mittel lässt sich durch Zusatz von anderen insektiziden und/oder akariziden Wirkstoffen wesentlich verbreitern und an gegebene Umstände anpassen. Als Wirkstoff-Zusätze kommen dabei z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate. Die erfindungsgemässen Mittel können auch weitere feste oder flüssige Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Düngemittel oder andere Wirkstoffe zur Erzielung spezieller Effekte, z. B. Bakterizide, Fungizide, Nematizide, Molluskizide oder selektive Herbizide, enthalten.

Die erfindungsgemässen Mittel werden in bekannter Weise hergestellt, bei Abwesenheit von Hilfsstoffen z. B. durch Mahlen, Sieben und/oder Pressen eines festen Wirkstoffs oder Wirkstoffgemisches, z. B. auf eine bestimmte Korngrösse, und bei Anwesenheit von mindestens einem Hilfsstoff z. B. durch inniges Vermischen und/oder Vermahlen des Wirkstoffs oder Wirkstoffgemisches mit dem (den) Hilfsstoff(en). Diese Verfahren zur Herstellung der erfindungsgemässen Mittel und die Verwendung der Verbindungen der Formel I zur Herstellung dieser Mittel bilden ebenfalls einen Gegenstand der Erfindung.

Die Anwendungsverfahren für die Mittel, also die Verfahren zur Bekämpfung von Schädlingen des erwähnten Typus, wie, je nach angestrebten Zielen und gegebenen Verhältnissen zu wählendes, Versprühen, Vernebeln, Bestauben, Bestreichen, Beizen, Streuen oder Giessen, und die Verwendung der Mittel zur Bekämpfung von Schädlingen des erwähnten Typus sind weitere Gegenstände der Erfindung. Typische Anwendungskonzentrationen liegen dabei zwischen 0,1 und 1000 ppm, bevorzugt zwischen 0,1 und 500 ppm, Wirkstoff. Die Aufwandmengen pro Hektar betragen im allgemeinen 1 bis 2000 g Wirkstoff pro Hektar, insbesondere 10 bis 1000 g/ha, vorzugsweise 20 bis 600 g/ha.

Ein bevorzugtes Anwendungsverfahren auf dem Gebiet des Pflanzenschutzes ist das Aufbringen auf das Blattwerk der Pflanzen (Blattapplikation), wobei sich Applikationsfrequenz und Aufwandmenge auf den Befallsdruck des jeweiligen Schädlings ausrichten lassen. Der Wirkstoff kann aber auch durch das Wurzelwerk in die Pflanzen gelangen (systemische Wirkung), indem man den Standort der Pflanzen mit einem flüssigen Mittel tränkt oder den Wirkstoff in fester Form in den Standort der Pflanzen, z. B. in den Boden, einbringt, z. B. in Form von Granulat (Bodenapplikation). Bei Wasserreiskulturen kann man solche Granulate dem überfluteten Reisfeld zudosieren.

Die erfindungsgemässen Mittel eignen sich auch für den Schutz von pflanzlichem Vermehrungsgut, z. B. Saatgut, wie Früchten, Knollen oder Körnern, oder Pflanzenstecklingen, vor tierischen Schädlingen. Das Vermehrungsgut kann dabei vor dem Ausbringen mit dem Mittel behandelt, Saatgut z. B. vor der Aussaat gebeizt, werden. Die erfindungsgemässen Wirkstoffe können auch auf Samenkörner aufgebracht werden (Coating), indem man die Körner entweder in einem flüssigen Mittel tränkt oder sie mit einem festen Mittel beschichtet. Das Mittel kann auch beim Ausbringen des Vermehrungsguts auf den Ort der Ausbringung, z. B. bei der Aussaat in die Saatfurche, appliziert werden. Diese Behandlungsverfahren für pflanzliches Vermehrungsgut und das so behandelte pflanzliche Vermehrungsgut sind weitere Gegenstände der Erfindung.

Die folgenden Beispiele dienen der Erläuterung der Erfindung. Sie schränken die Erfindung nicht ein. Temperaturen sind in Grad Celsius, Mischungsverhältnisse von Lösungsmitteln in Volumenteilen angegeben.

### Herstellungsbeispiele

### Beispiel H1: 2-[2-Chlor-4-(3-chlorphenoxy)-phenoxy]ethanol (Verbindung Nr. 10.1.6).

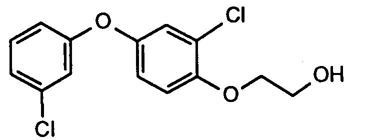

Ein Gemisch aus 7.7 g 2-Chlor-4-(3-chlorphenoxy)-phenol, 3.17 g 2-Oxo-1,3-dioxolan und 1.3 g Tetraethylammoniumchlorid wird zusammengeschmolzen, die Schmelze in einer Stickstoffatmosphäre unter Rühren 8 Stunden auf 130° erhitzt, das Reaktionsgemisch nach dem Abkühlen in 150 g Diethylether aufgenommen und die etherische Phase mehrmals mit Wasser gewaschen, über Natriumsulfat getrocknet und zur Trockne eingedampft. Der Rückstand wird chromatographisch [Kieselgel; Diethylether/Hexan (1:1)] gereinigt. Man erhält so die Titelverbindung, die einen Brechungsindex n_{D}²⁰ von 1.5995 aufweist.

### Beispiel H2: Chlorkohlensäure-2-(2-chlor-4-phenoxy)-phenoxy-ethylester

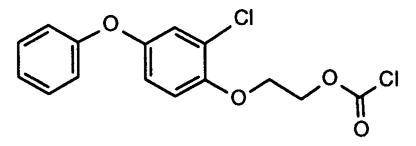

Man leitet in eine Mischung von 39.7 g 2-(2-chlor-4-phenoxy)-phenoxy-ethanol in 150 ml Toluol unter Rühren bei 55°C innert 2 bis 3 Stunden 19.8 g Phosgen ein. Man rührt bei 50°C weitere 14 Stunden. Das Lösungsmittel wird am Rotationsverdampfer entfernt und der Rückstand aus n-Hexan umkristallisiert. Man erhält das Titelprodukt mit einem Schmelzpunkt von 84-85.5°C.

### Beispiel H3: 1-[2-Chlor-4-(3-chlorphenoxy)-phenoxy]-2-ethylaminocarbonyloxy-ethan (Tabelle 1, Verbindung Nr. 1.2.6).

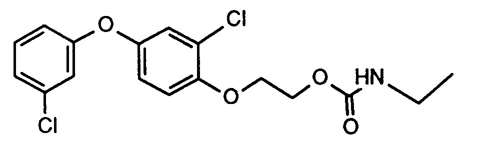

Zu einer Lösung von 4.49 g 2-[2-Chlor-4-(3-chlorphenoxy)-phenoxy]ethanol in 40 ml Tetrahydrofuran gibt man 0.1 ml Triethylamin, 15 mg 1,4-Diazabicyclo[2.2.2]octan und 1.3 g Ethylisocyanat zu. Das Reaktionsgemisch wird unter Rühren 18 Stunden auf 55°C erhitzt und dann im Vakuum zur Trockene eingedampft. Der Rückstand wird chromatographisch [Kieselgel; Diethylether/Hexan (2:3)] gereinigt. Man erhält so die Titelverbindung, die bei 73-74°C schmilzt.

### Beispiel H4: 1-[2-Chlor-4-(4-fluorphenoxy)-phenoxy]-2-ethylaminocarbonyloxy-ethan (Verbindung 1.2.4).

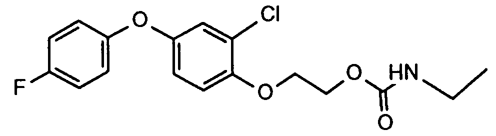

Zu 2.86 g 2-Chlor-4-(4-fluorphenoxy)-phenol in 10 ml Dimethylsulfoxid tropft man unter Rühren bei 10-15°C eine Lösung von 1.41 g K-tert.-Butylat in 20 ml Dimethylsulfoxid. Man tropft anschliessend 2.92 g Ethylcarbaminsäure -2-bromethylester in 10 ml Dimethylsulfoxid bei Raumtemperatur zu und rührt dann 18 Stunden bei 40°C. Dann wird das Reaktionsgemisch auf 300 ml Eiswasser gegossen und mehrmals mit Ether extrahiert. Die vereinigten Etherphasen werden mit wenig Wasser neutral gewaschen, über Natriumsulfat getrocknet und das Lösungsmittel im Vacuum abgedampft. Das Rohprodukt wird an Kieselgel mit Diethylether/Hexan (1:9) als Laufmittel gereinigt. Man erhält die Titelverbindung mit einem Schmelzpunkt von 42-43°C.

### Beispiel H5: 1-[2-Chlor-4-phenoxy-phenoxy]-2-N-pyrrolidinocarbonyloxy-ethan (Verbindung 1.121.1)

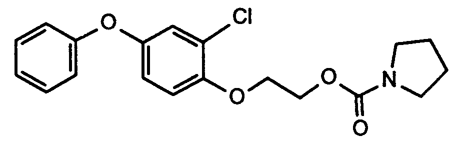

Zu einer Mischung von 2.2 g Pyrrolidin in 20 ml Dichlormethan tropft man unter Rühren bei 5-10°C eine Lösung von 4 g Chlorkohlensäure-2-(2-chlor-4-phenoxy)-phenoxy-ethylester in 20 ml Dichlormethan. Nach drei Stunden Nachrühren bei Raumtemperatur wird das Reaktionsgemisch dreimal-mit je 50 ml 1N Salzsäure und dann dreimal mit Wasser gewaschen, über Natriumsulfat getrocknet und das Lösungsmittel abgedampft. Der Rückstand wird mit n-Hexan verrührt, filtriert und mit kaltem Hexan gewaschen. Man erhält die Titelverbindung mit einem Schmelzpunkt von 79-81°C.

### Beispiel H6: 1-[2-Chlor-4-phenoxy-phenoxy]-2-[N-(4-chlor-phenyl)-aminocarbonyloxy]-ethan (Verbindung 1.124.1)

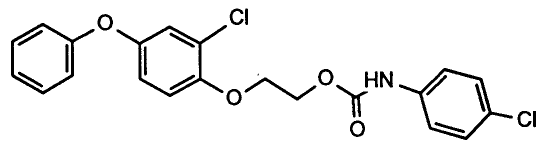

Zu 3.95 g 4-Chloranilin in 20 ml Dichlormethan werden unter Rühren bei 5-10°C 4 g Chlorkohlensäure-2-(2-chlor-4-phenoxy)-phenoxy-ethylester in 20 ml Dichlormethan getropft. Nach zwei Stunden Rühren bei Raumtemperatur wird das Reaktionsgemisch mit 100 ml Dichlormethan versetzt, dreimal mit 1N Salzsäure und dreimal mit Wasser gewaschen. Die organische Phase wird über Natriumsulfat getrocknet und das Lösungsmittel im Vacuum abgedampft. Das Rohprodukt wird mit n-Hexan verrührt, filtriert und mit kaltem Hexan gewaschen. Man erhält die Titelverbindung mit einem Schmelzpunkt von 155-155.5°C.

### Beispiel H7: 1-[2-Chlor-4-(3-chlorphenoxy)-phenoxy]-2-[N-ethyl-N-(4-chlorphenylsulfenyl)-aminocarbonyloxy]-ethan (Verbindung 1.68.6)

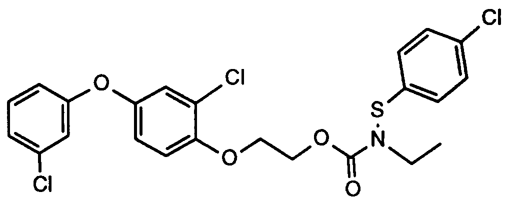

Zu einer Mischung von 3.7 g 1-[2-Chlor-4-(3-chlorphenoxy)-phenoxy]-2-ethylamino-carbonyloxy-ethan, 10 ml Pyridin und 10 ml Toluol tropft man unter Rühren bei 0-5°C innert ca. 30 Minuten eine Lösung von 2 g 4-Chlorphenylsulfenylchlorid in 10 ml Toluol und rührt weitere 16 Stunden bei Raumtemperatur. Das Reaktionsgemisch wird mit 100 ml Ether verdünnt, dreimal mit eiskalter 1N Salzsäure und dreimal mit Wasser gewaschen, die organische Phase über Natriumsulfat getrocknet und das Lösungsmittel im Vacuum abgedampft. Das Rohprodukt wird an Kieselgel mit Diethylether/n-Hexan (1:3) als Fliessmittel gereinigt. Man erhält die Titelverbindung mit einem Brechungsindex n^{D}₂₀ von 1.6209.

### Beispiel H8: 1-[2-Chlor-4-(3-chlorphenoxy)-phenoxy]-2-(N-ethyl-N-acetyl-aminocarbonyloxy)ethan (Verbindung 1.80.6)

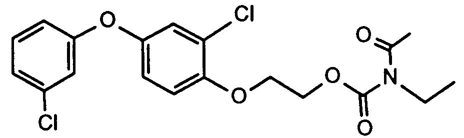

Zu einer Mischung von 5.55 g 1-[2-Chlor-4-(3-chlorphenoxy)-phenoxy]-2-ethylamino-carbonyloxy-ethan, 3.6 g Pyridin, 0.15 g Dimethylaminopyridin und 60 ml Toluol werden bei 10-20°C 4.7 g Acetylchlorid getropft. Dann wird das Gemisch bei Rückflusstemperatur 50 Stunden lang gerührt. Man giesst die Reaktionsmasse auf 300 ml Eiswasser und extrahiert dreimal mit Diethylether. Die vereinigten Etherphasen werden mit 1N Salzsäure und dreimal mit Wasser gewaschen, über Natriumsulfat getrocknet und das Lösungsmittel im Vacuum abgedampft. Das Rohprodukt wird an Kieselgel mit Diethylether/n-Hexan (2:7) als Fliessmittel gereinigt. Man erhält die Titelverbindung mit einem Brechungsindex n^{D}₂₀ von 1.5640.

### Beispiel H9:

In analoger Weise wie in den Beispielen H1 bis H8 beschrieben können auch die anderen in den Tabellen 1 bis 14 aufgeführten Verbindungen hergestellt werden. In den Tabellen 7, 8, 9, 13 und 14 offenbart jede Zeile genau eine Verbindung bestimmter Konstitution. In den Tabellen 1 bis 6 und 10 bis 12 offenbart hingegen jede einzelne Zeile nicht nur eine einzige Verbindung bestimmter Konstitution. Vielmehr gibt in der Spalte "Verbindungen" dieser Tabellen der Ausdruck "1-2", "1-7" bzw. "1-26", der in jeder Zeile nach dem zweiten Punkt in dieser Spalte steht, durch die dem Bindestrich folgende Zahl an, wieviele Verbindungen unterschiedlicher Konstitution durch die betreffende Zeile offenbart werden. Der Ausdruck "1-2" bedeutet demnach beispielsweise, dass die betreffende Zeile zwei Einzelverbindungen unterschiedlicher Konstitution offenbart. So werden z. B. in der mit "5.1.1-2" beginnenden Zeile von Tabelle 5 die beiden voneinander konstitutionsverschiedenen Verbindungen 5.1.1 und 5.1.2 offenbart, während der Ausdruck "4.2.1-7" für die sieben jeweils voneinander konstitutionsverschiedenen Verbindungen 4.2.1, 4.2.2, ...,4.2.6 und 4.2.7 steht und der Ausdruck "1.8.1-26" für die 26 jeweils voneinander konstitutionsverschiedenen Verbindungen 1.8.1, 1.8.2, ..., 1.8.25 und 1.8.26 steht. Die in der betreffenden Zeile offenbarten Einzelverbindungen sind jeweils einzig in der Weise voneinander konstitutionsverschieden, dass jede von ihnen eine andere Bedeutung der Variablen (R₁)ₙ aufweist. Jede Zahl in dem Ausdruck "1-2" (1 bis 2), also 1 und 2, jede Zahl in dem Ausdruck "1-7" (1 bis 7), also 1,2, ..., 6 und 7, und jede Zahl in dem Ausdruck "1-26" (1 bis 26), also 1, 2, .., 25 und 26, steht jeweils, einheitlich in allen Zeilen aller Tabellen 1 bis 6 und 9 bis 11, für eine bestimmte Bedeutung von (R₁)ₙ, nämlich 1 für (R₁)₀, 2 für 4-Chlor, 3 für 3-Fluor, 4 für 4-Fluor, 5 für 3,5-Difluor, 6 für 3-Chlor, 7 für 3,4-Dichlor, 8 für 2-Fluor, 9 für 3,5-Dichlor, 10 für 3-Chlor-4-fluor, 11 für 2,4-Difluor, 12 für 4-Brom-2-fluor, 13 für 4-Brom, 14 für 3-Methyl, 15 für 4-Methyl, 16 für 3-Ethyl, 17 für 4-Ethyl, 18 für 4-Cyano, 19 für 3-Trifluormethyl, 20 für 3,5-Dimethyl, 21 für 4-Trifluormethyl, 22 für 3-Methoxy, 23 für 4-Methoxy, 24 für 3,4-Methylendioxy, 25 für 3-Trifluormethoxy und 26 für 4-Trifluormethoxy. Durch entsprechende Wahl der Zahl nach dem zweiten Punkt in der Spalte "Verbindungen" erfolgt somit, in Verbindung mit den in der betreffenden Zeile aufgeführten Bedeutungen der übrigen Variablen, eine eindeutige Zuordnung einer bestimmten Verbindungsnummer zu einer einzigen konstitutionsdefinierten Einzelverbindung mit einer bestimmten Bedeutung aller Variablen einschliesslich (R₁)ₙ. So ist z. B. die Verbindung 3.78.23 diejenige Verbindung der Formel I, worin R₁ 4-Methoxy, R₂ Wasserstoff, R₃ Chlor, R₄ Wasserstoff, R₅ COCON(C₄H₉)₂, R₆ Ethyl, n 1, X Methylen, Y O und Z O bedeuten, und die Verbindung 12.14.2 ist diejenige Verbindung der Formel II, worin R₁ 4-Chlor, R₂ 5-Methyl, R₃ Methyl, R₄ Wasserstoff, n 1, X C(=O) und Y O bedeuten. In der Spalte "Physikalische Daten" der Tabelle 14 bezeichnen die angegebenen Temperaturen jeweils den Schmelzpunkt der betreffenden Verbindung und "n_{D}^{T}" steht für den Brechungsindex der betreffenden Verbindung bei der Temperatur T°C.

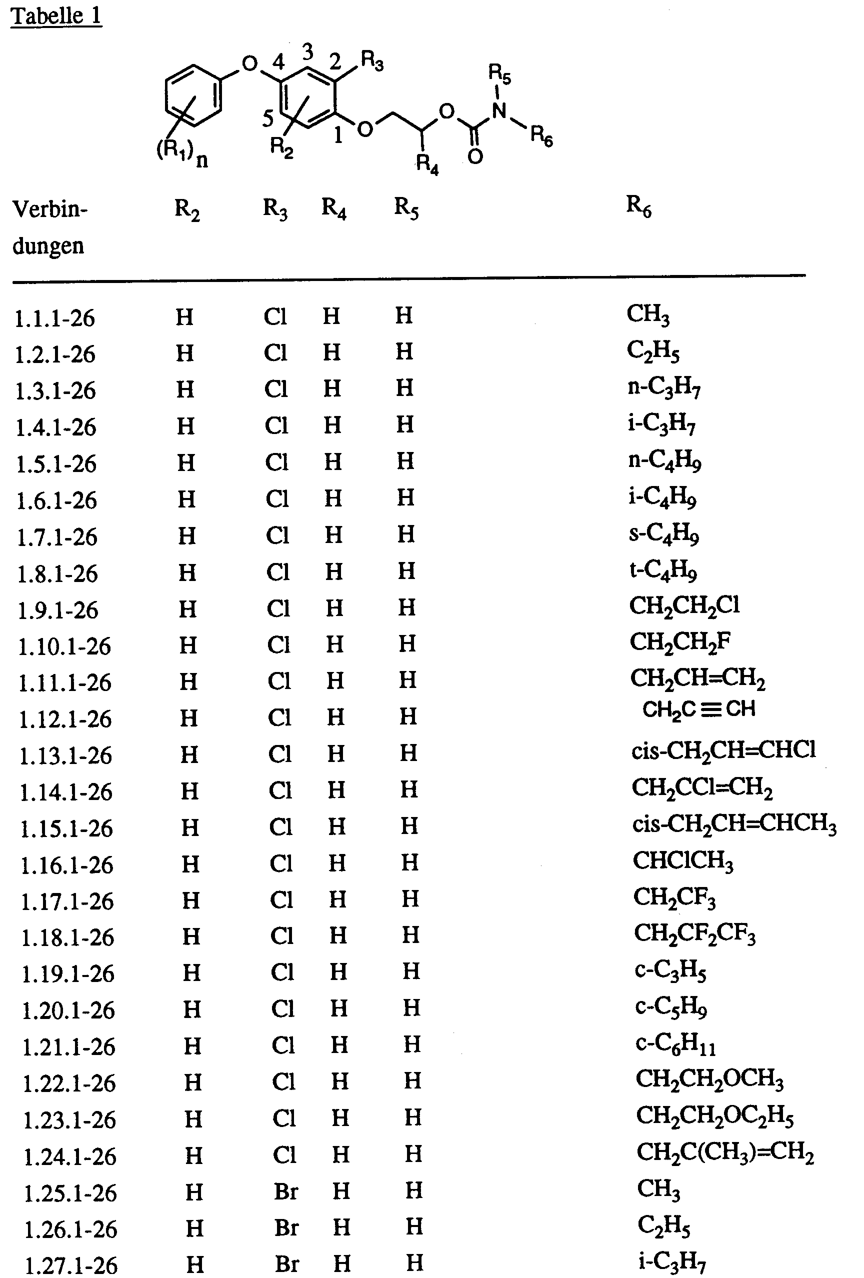

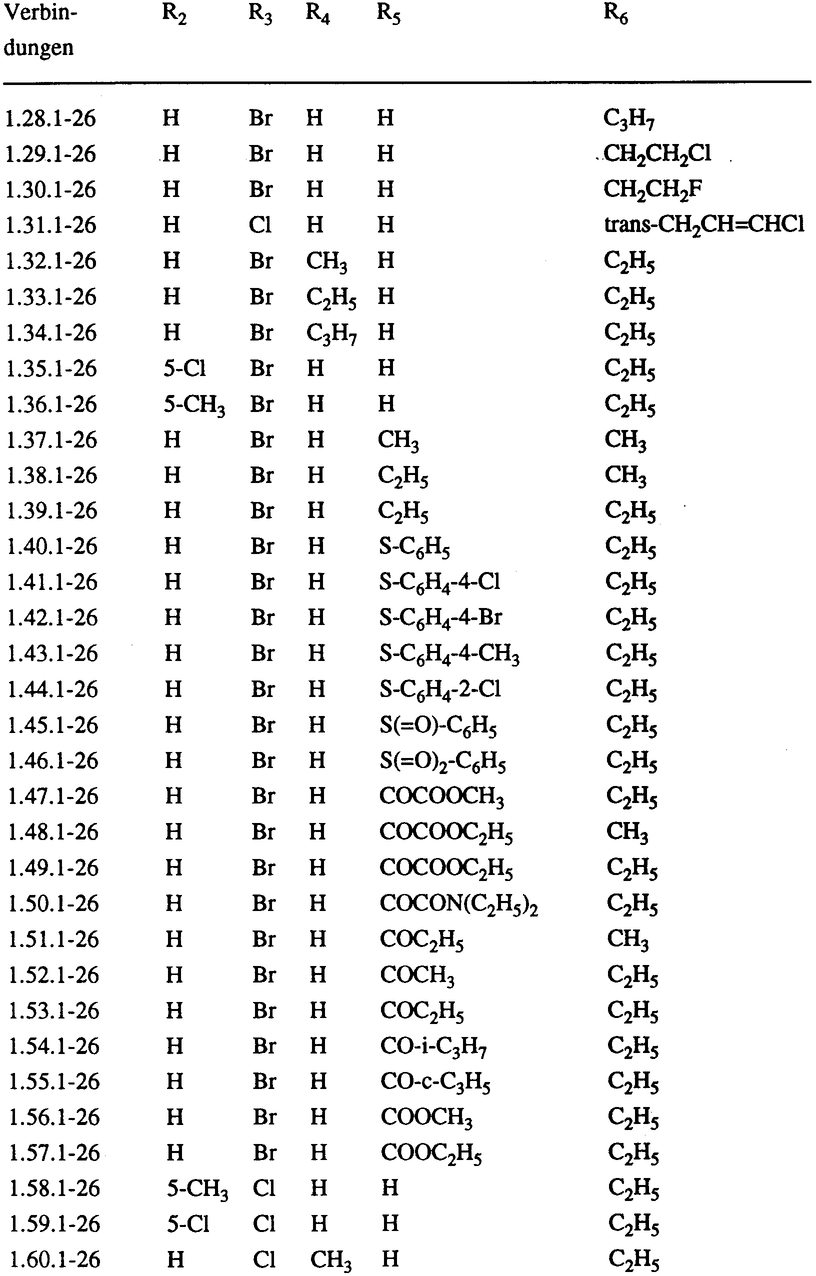

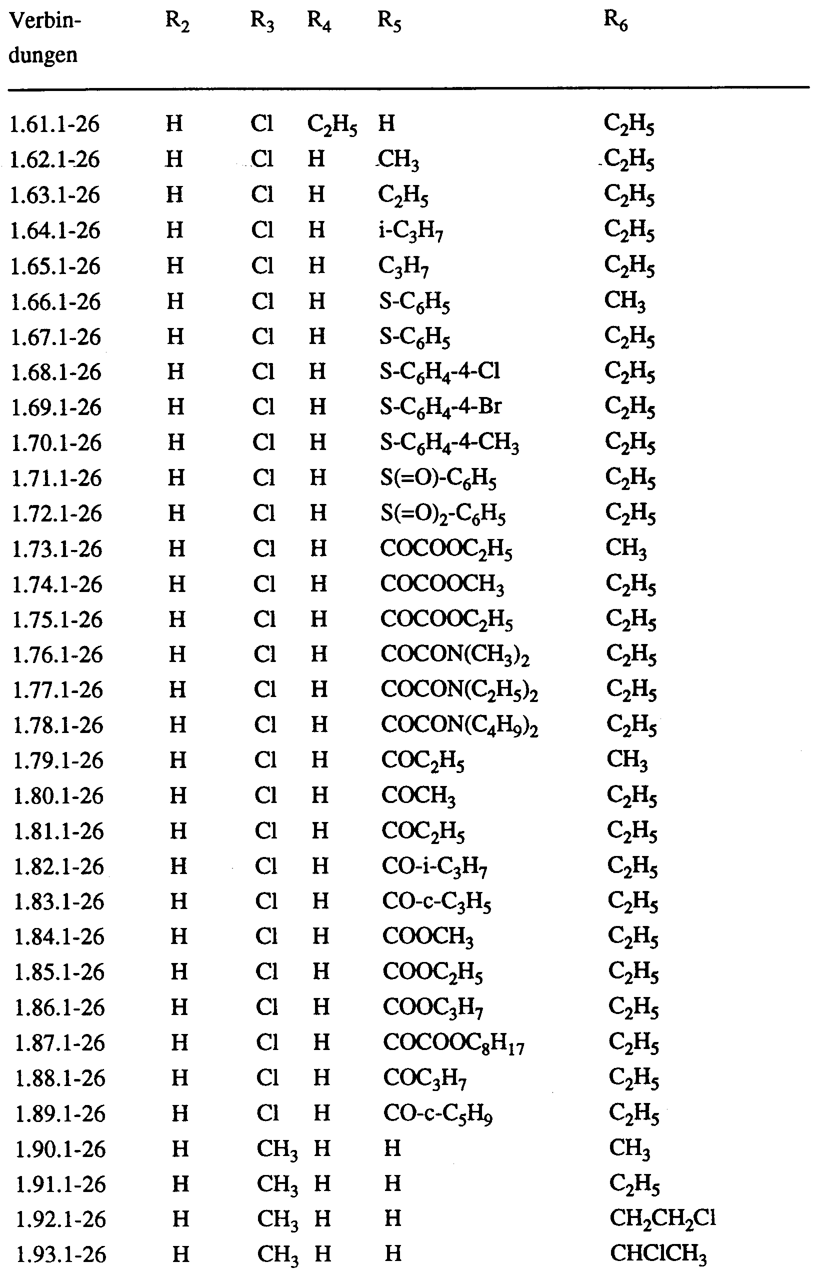

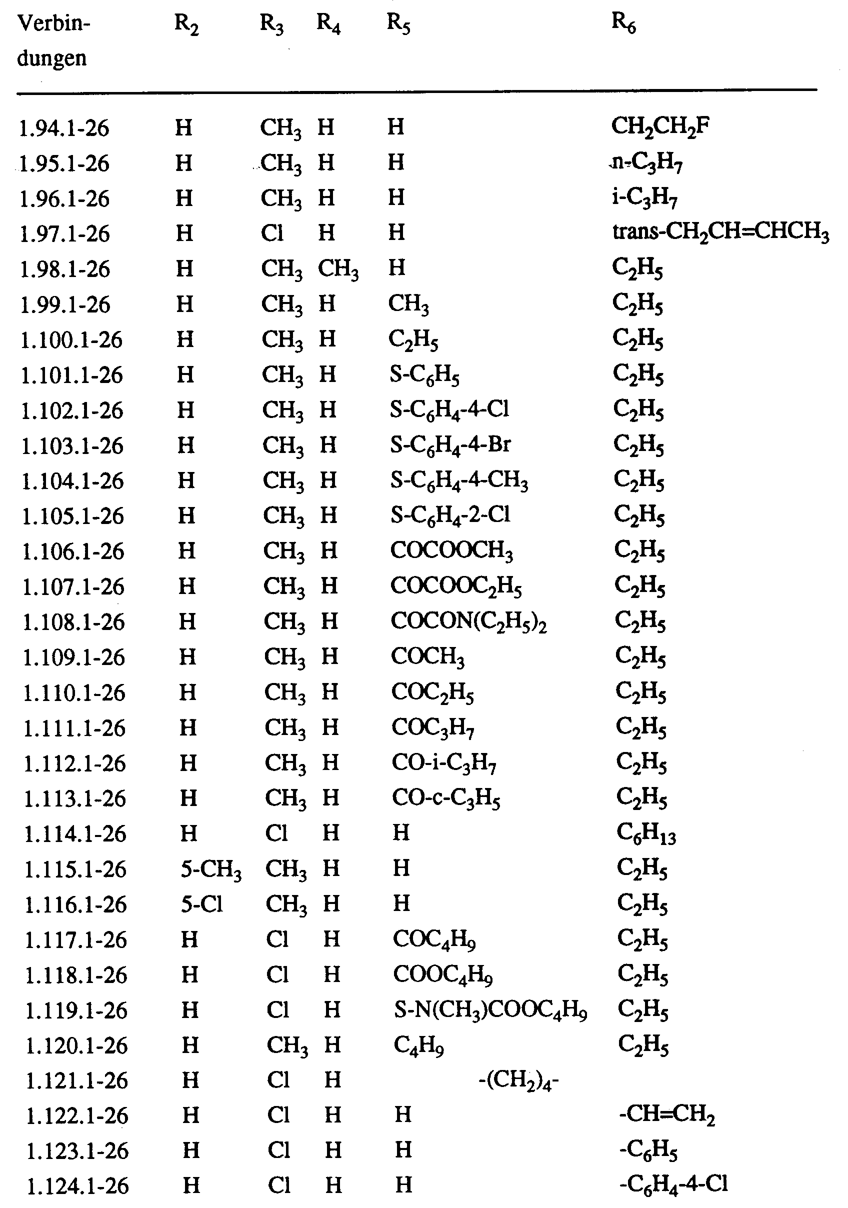

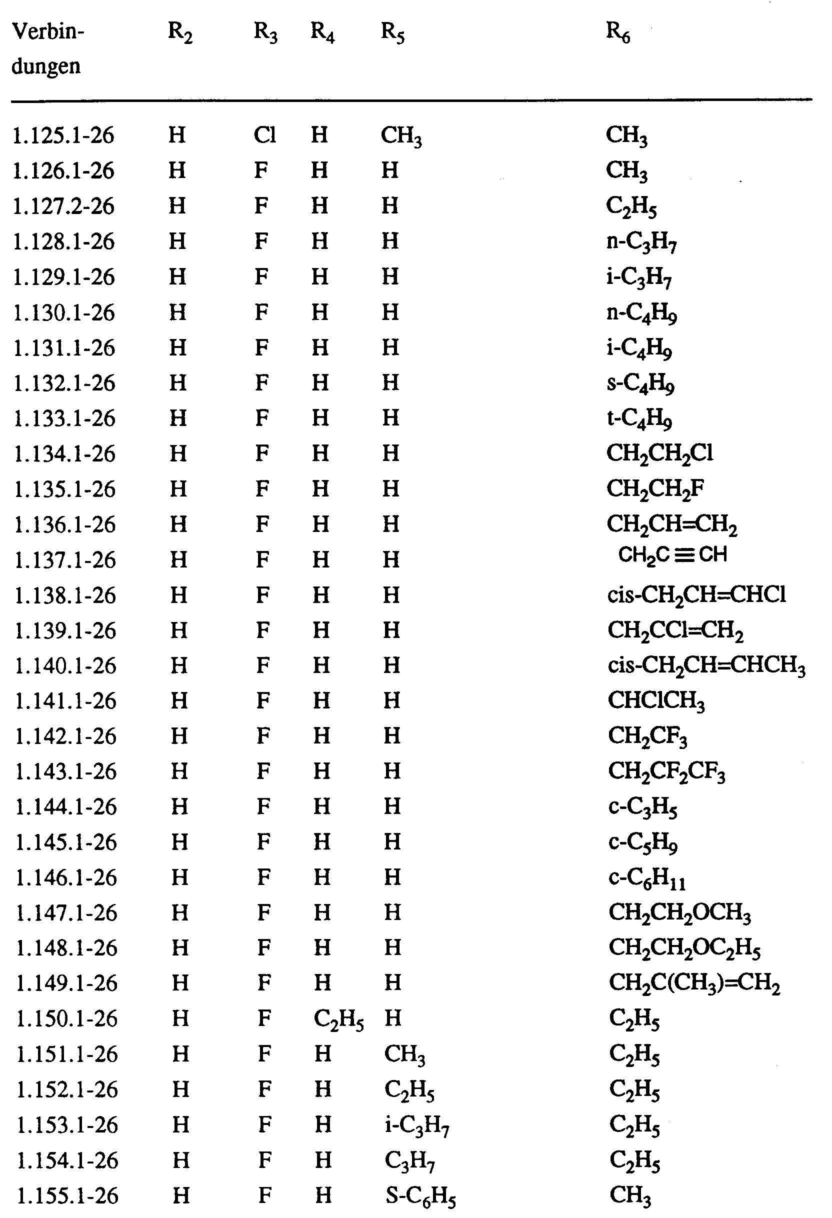

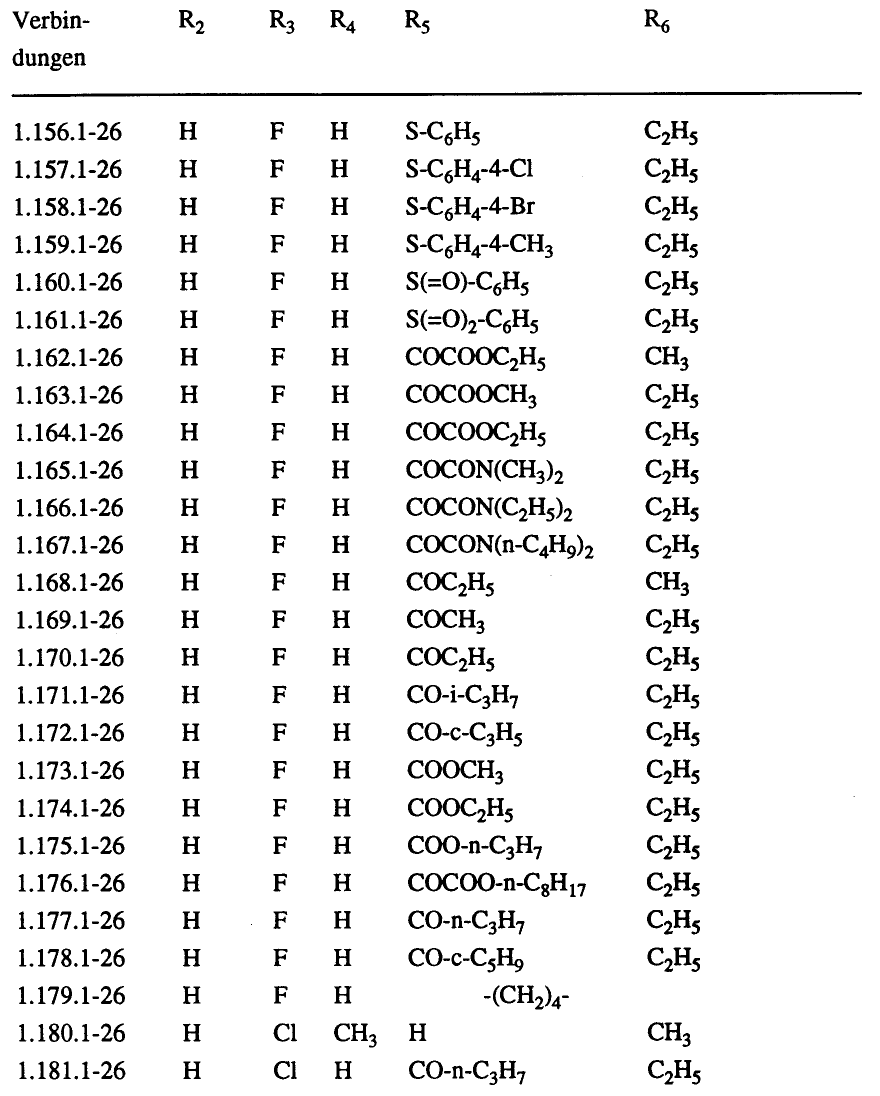

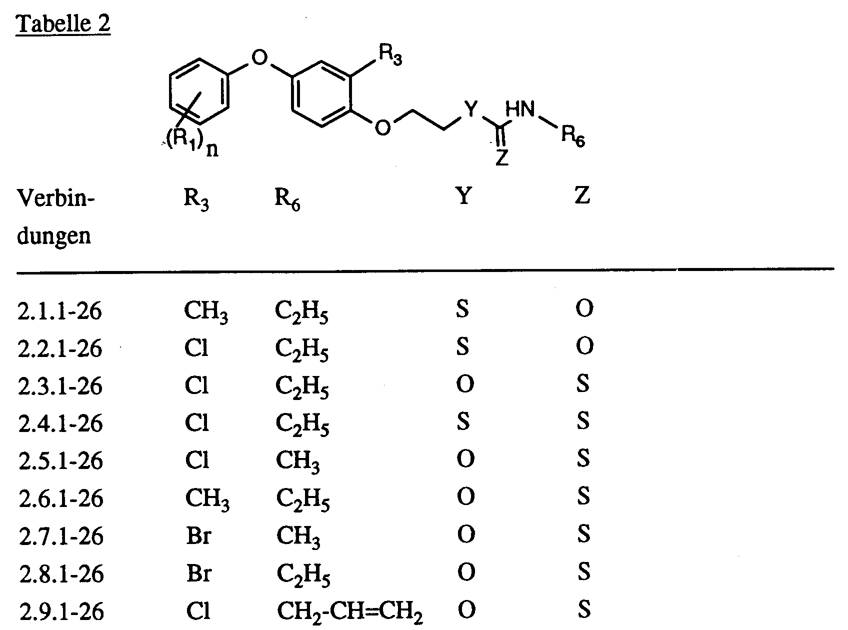

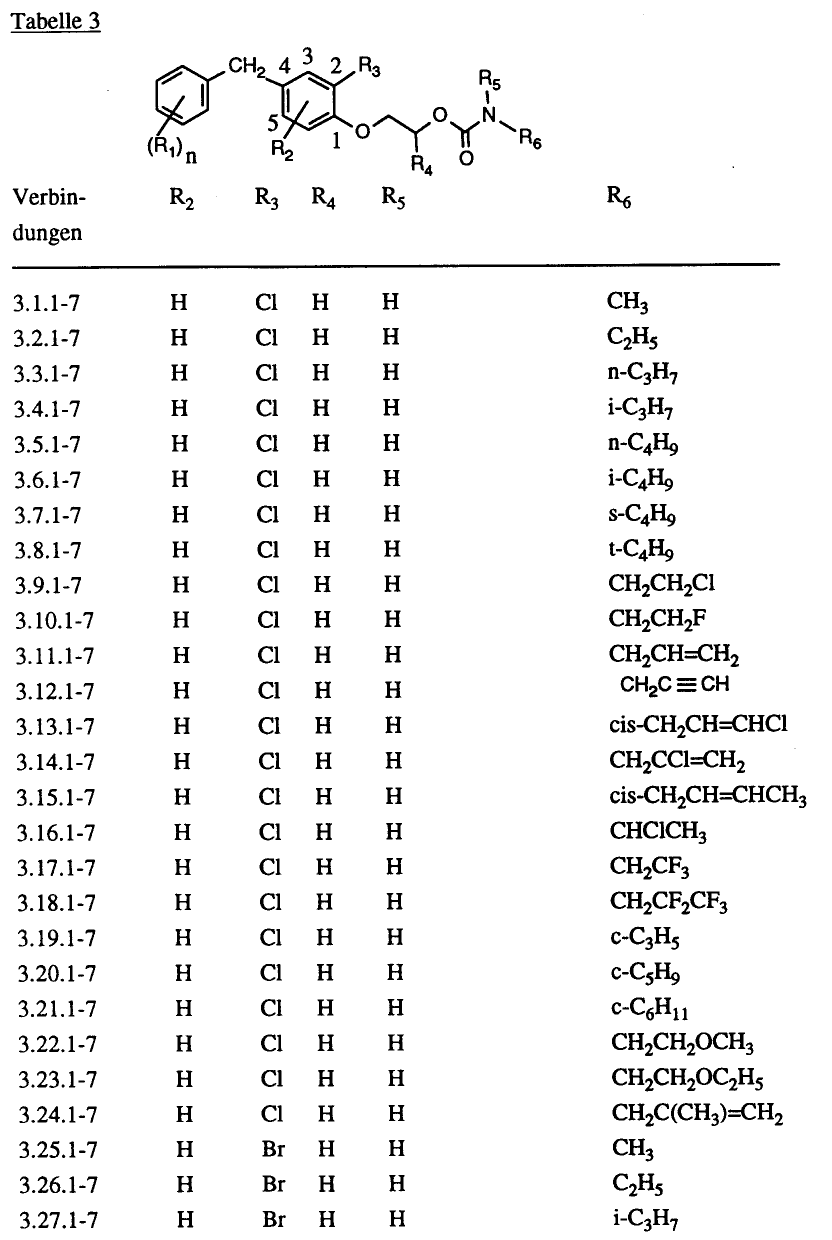

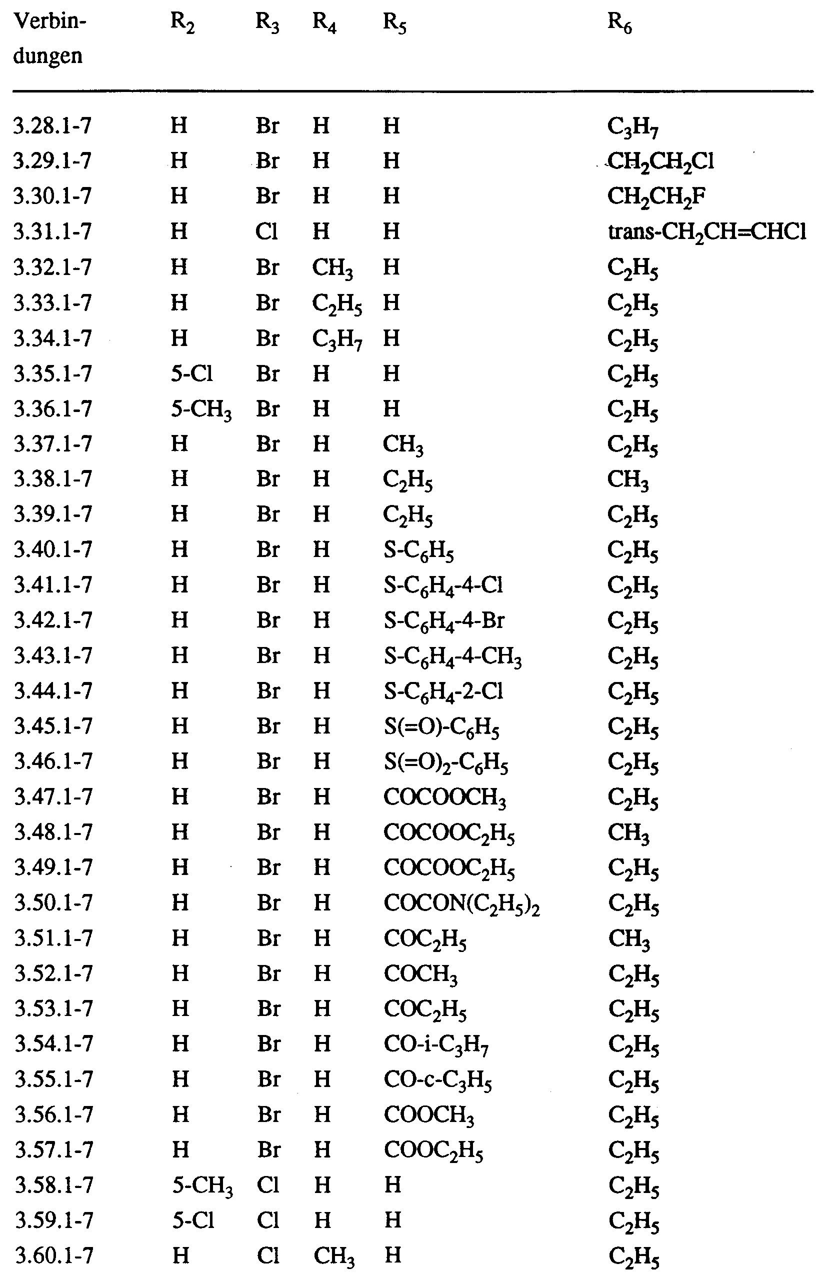

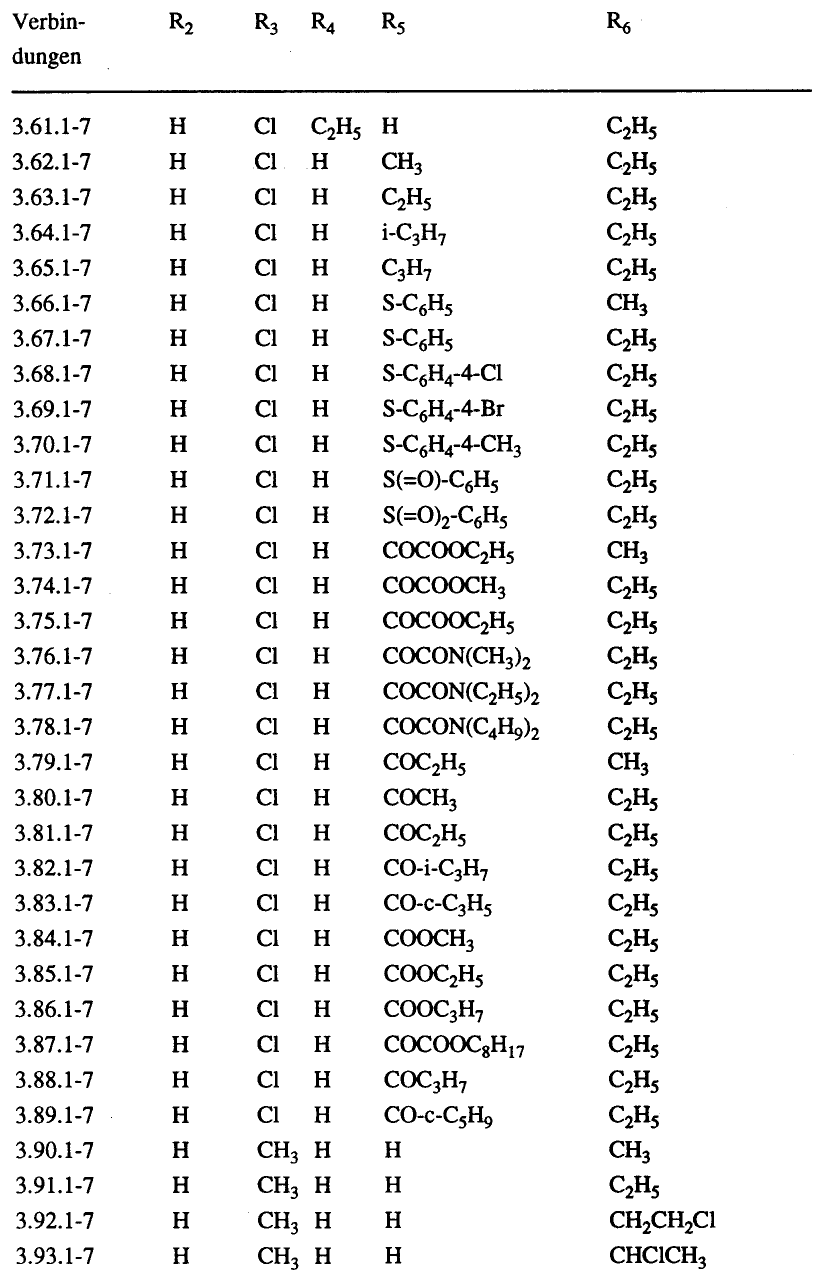

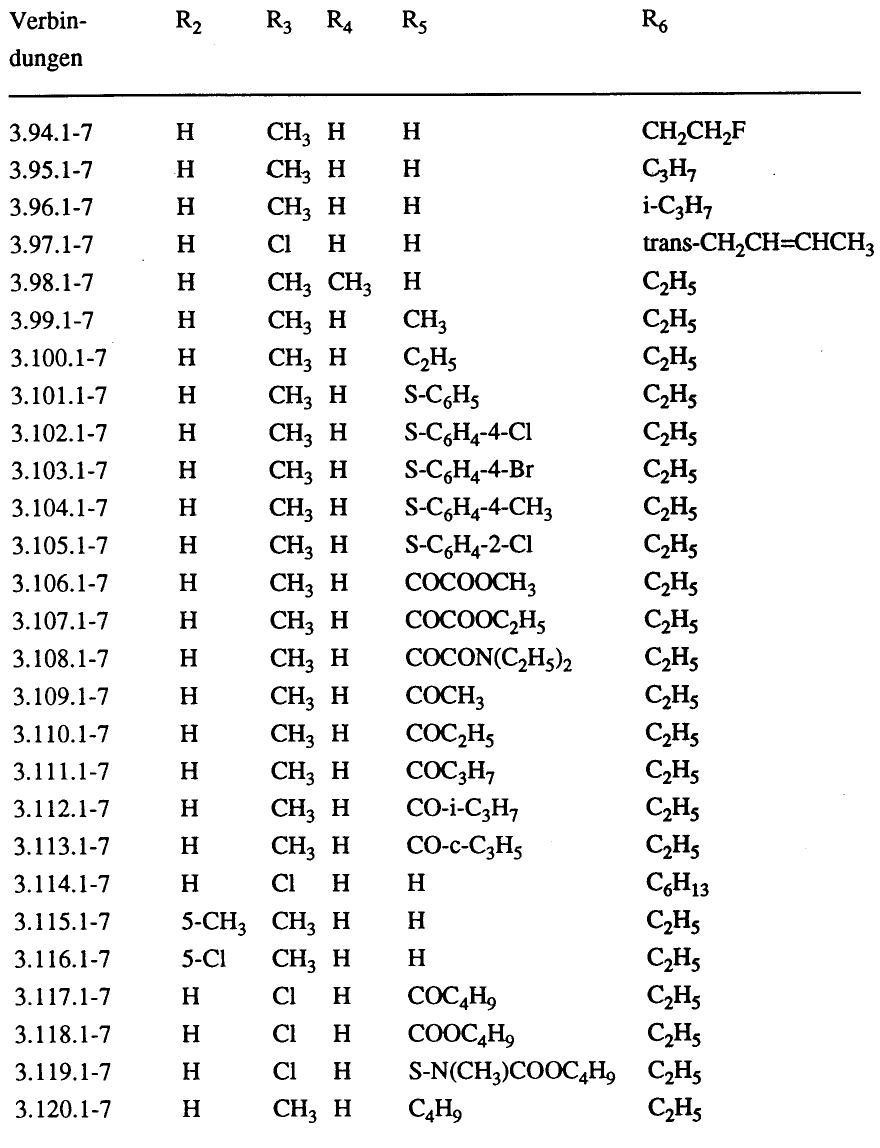

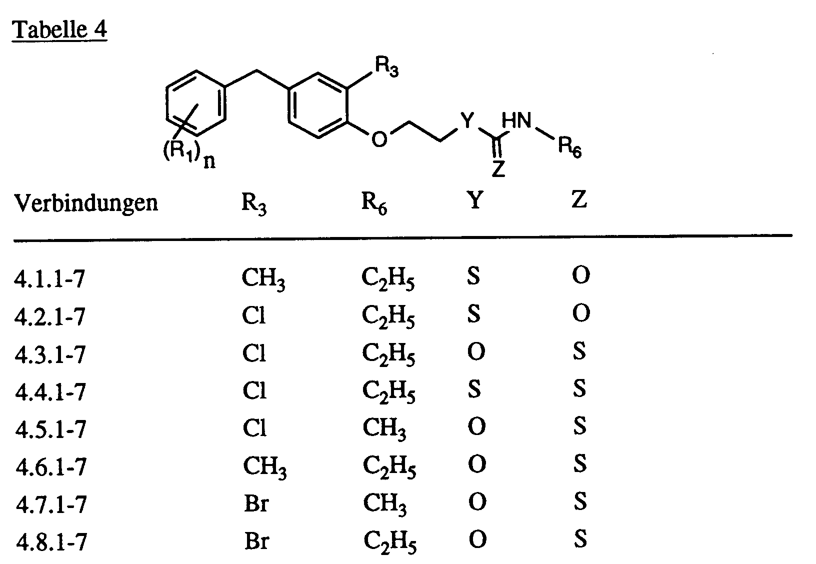

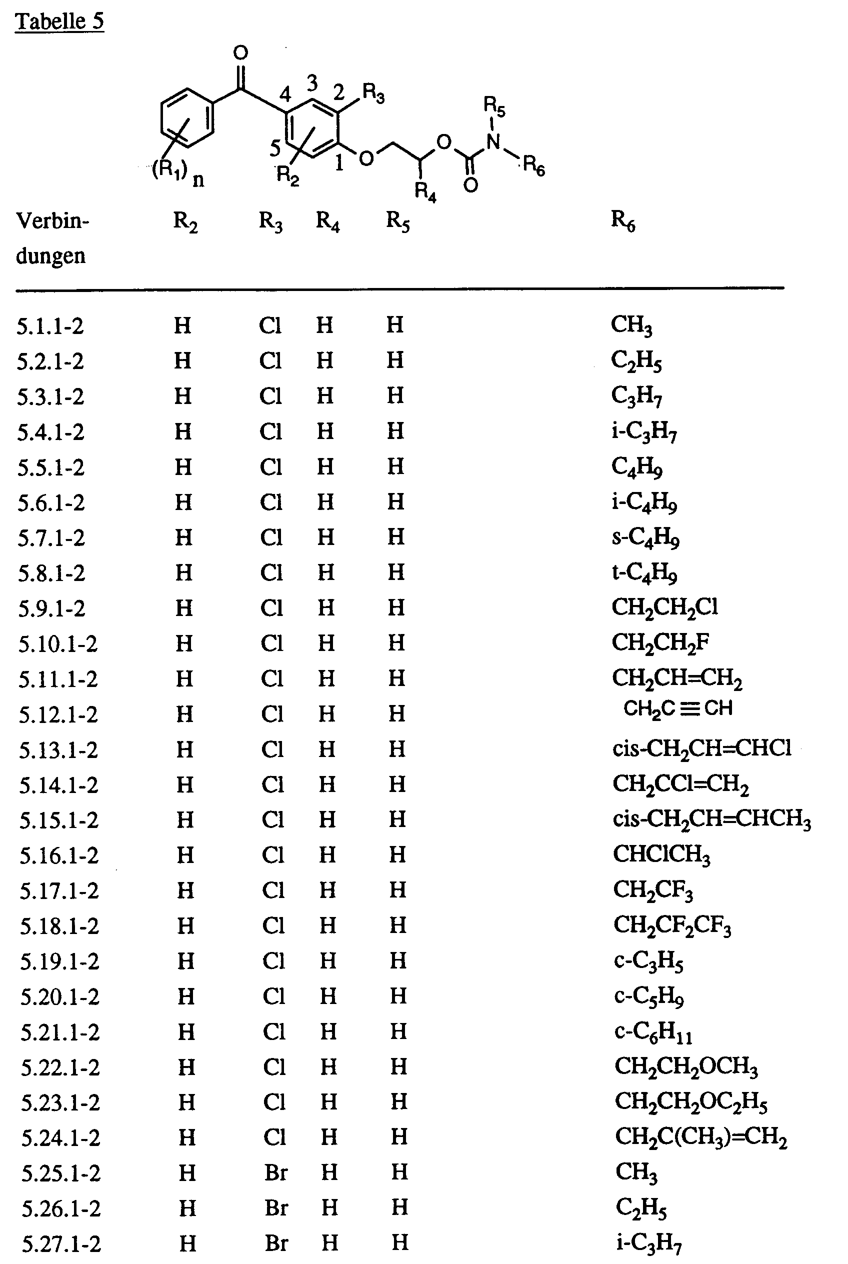

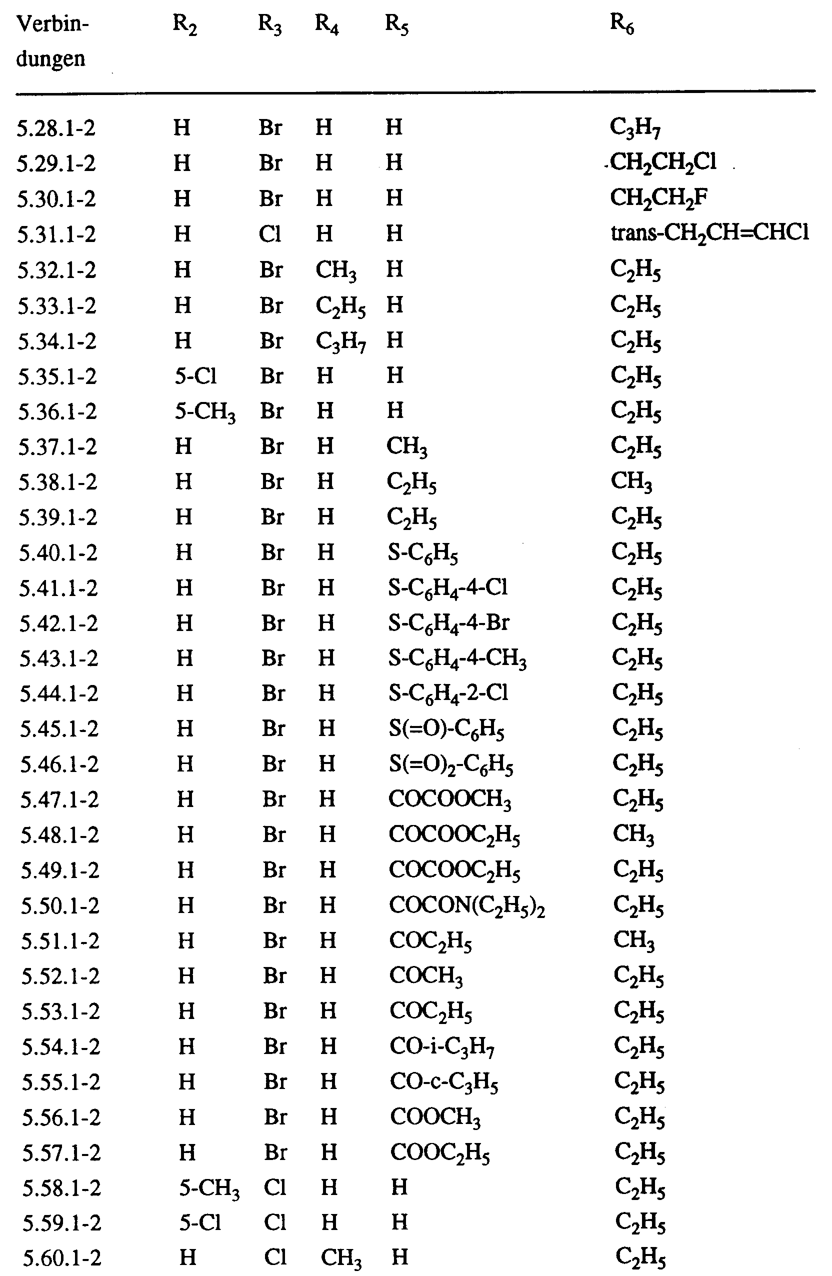

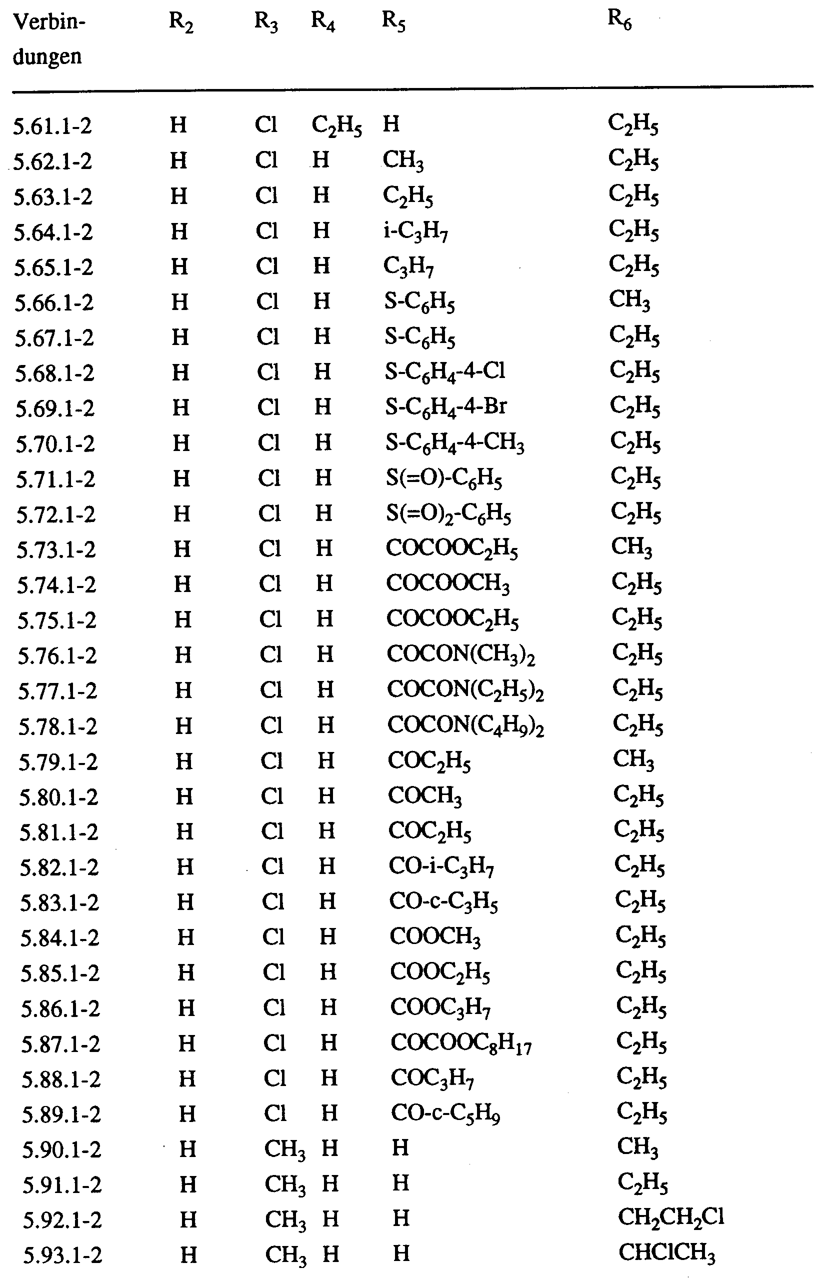

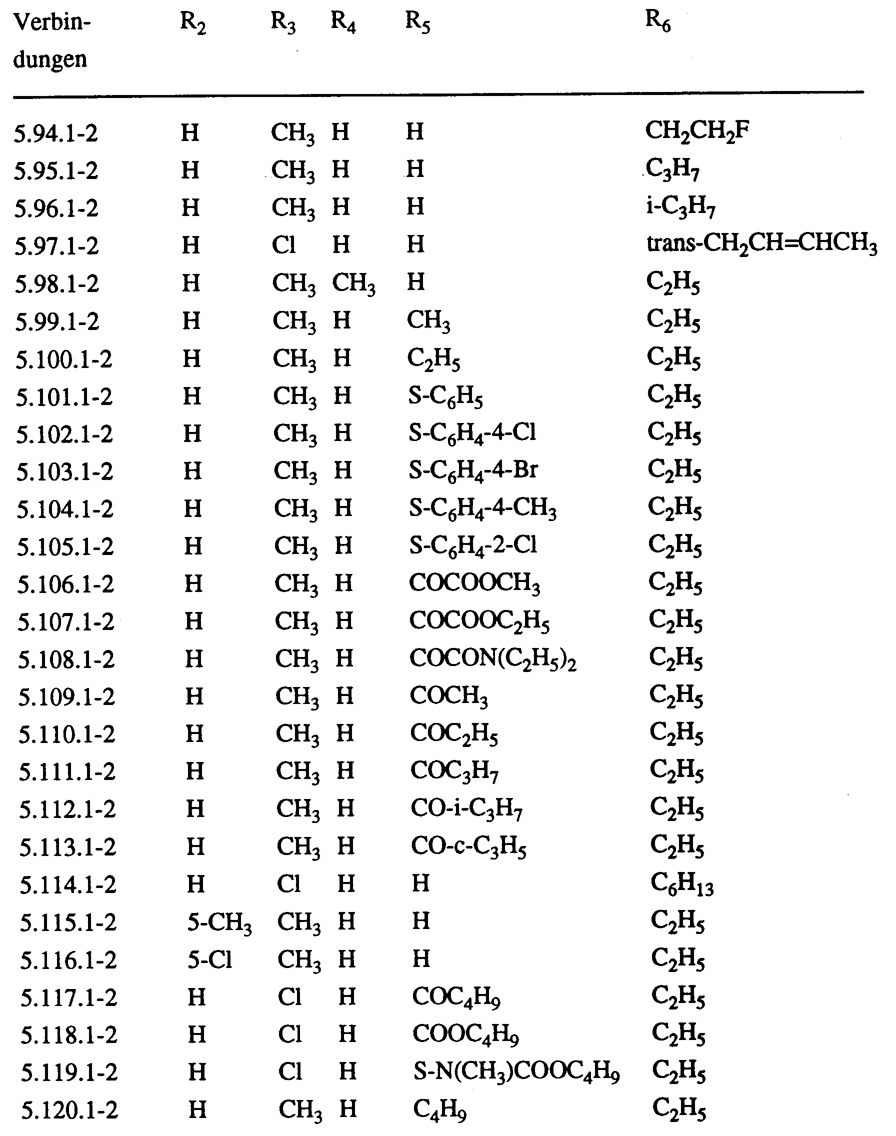

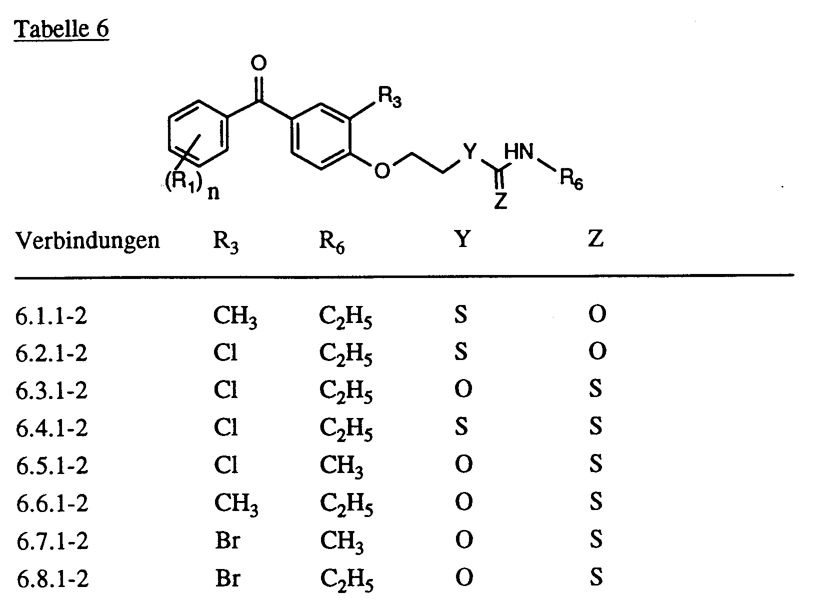

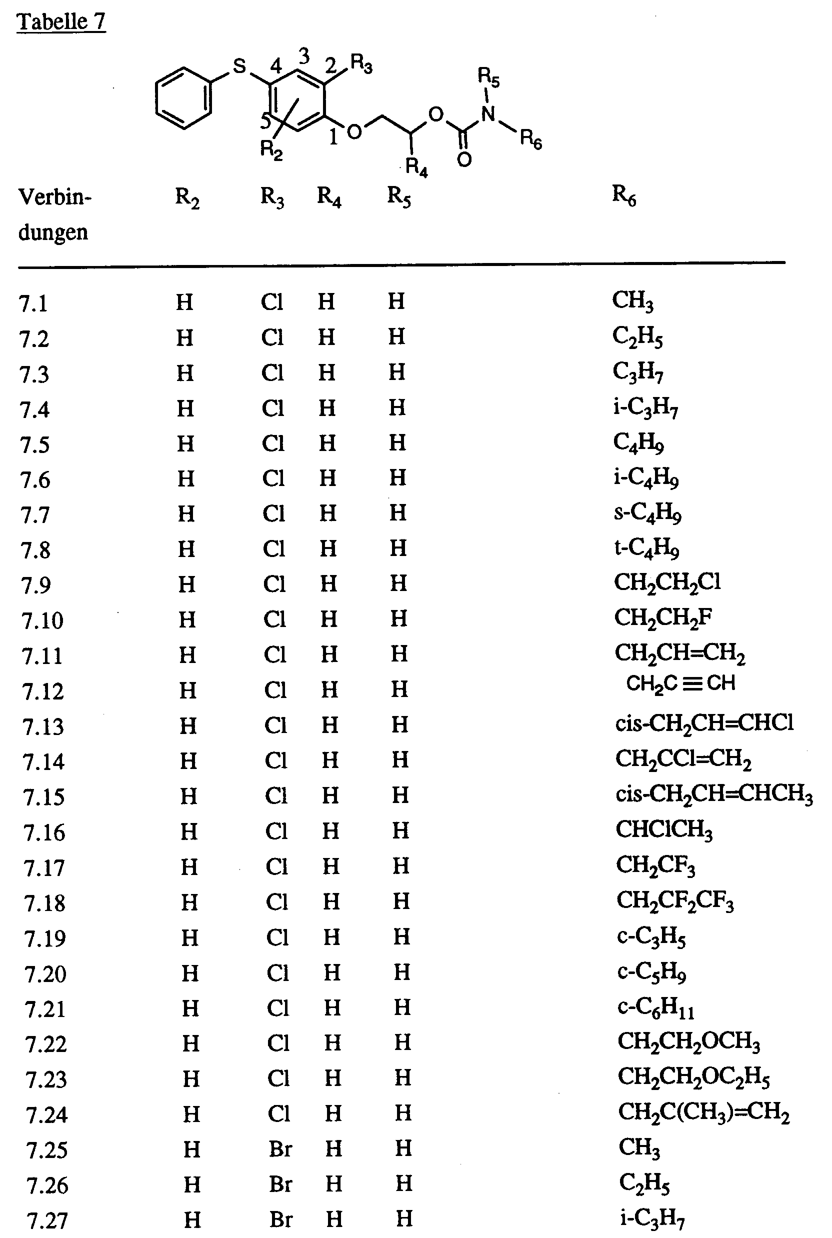

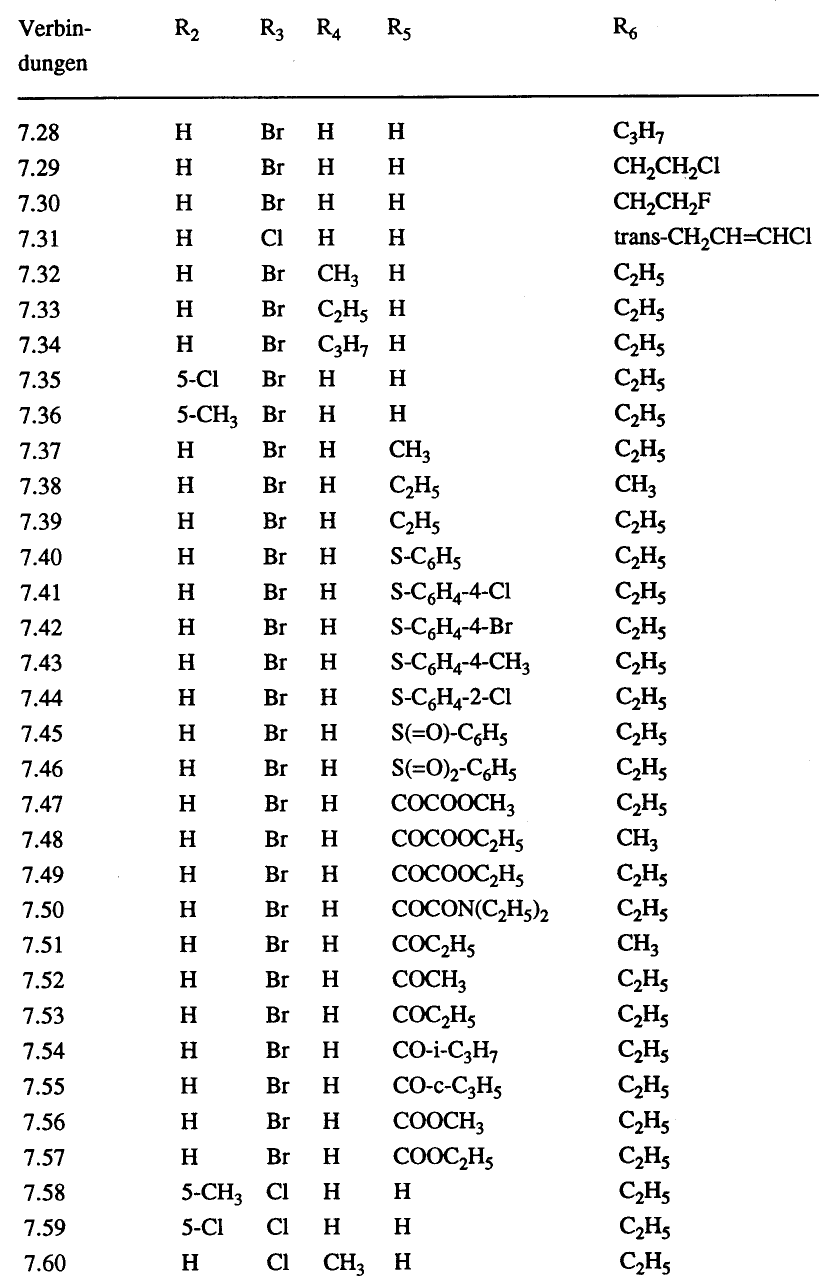

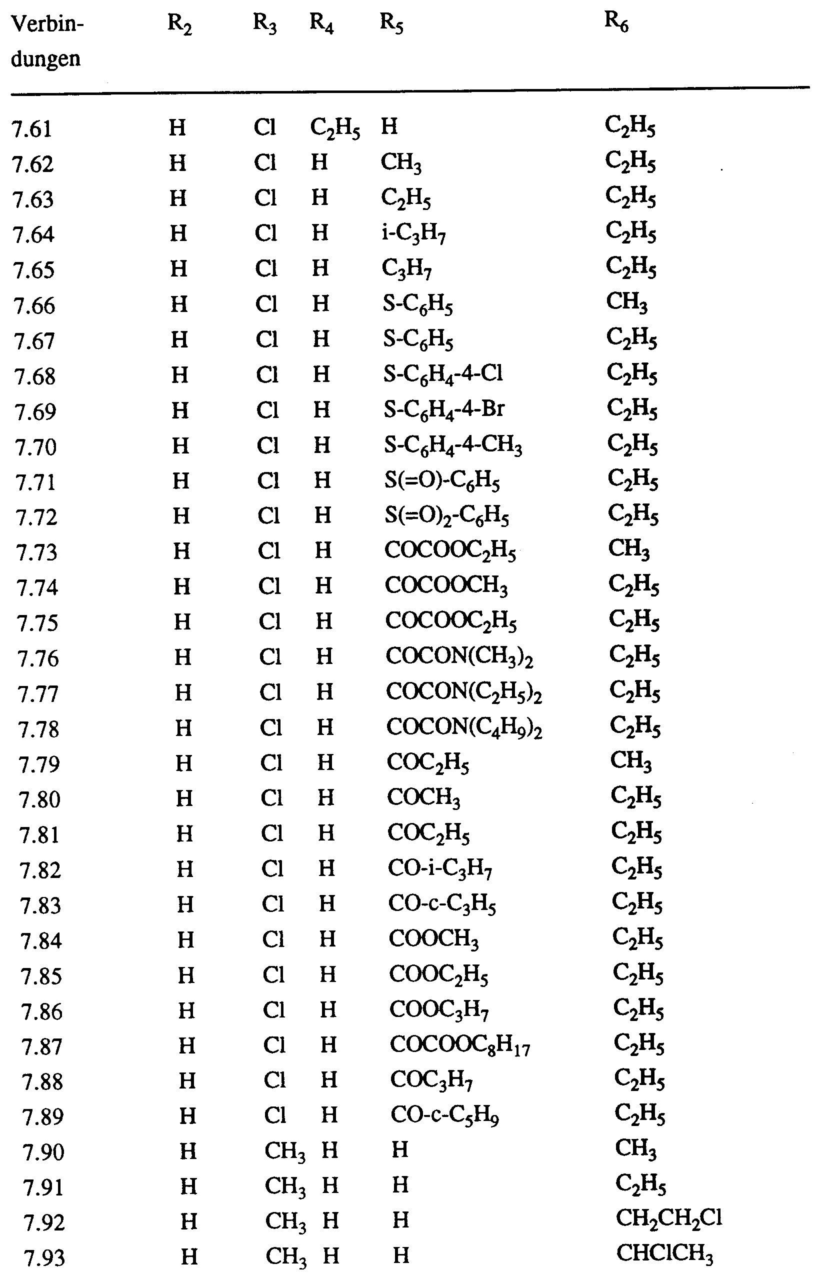

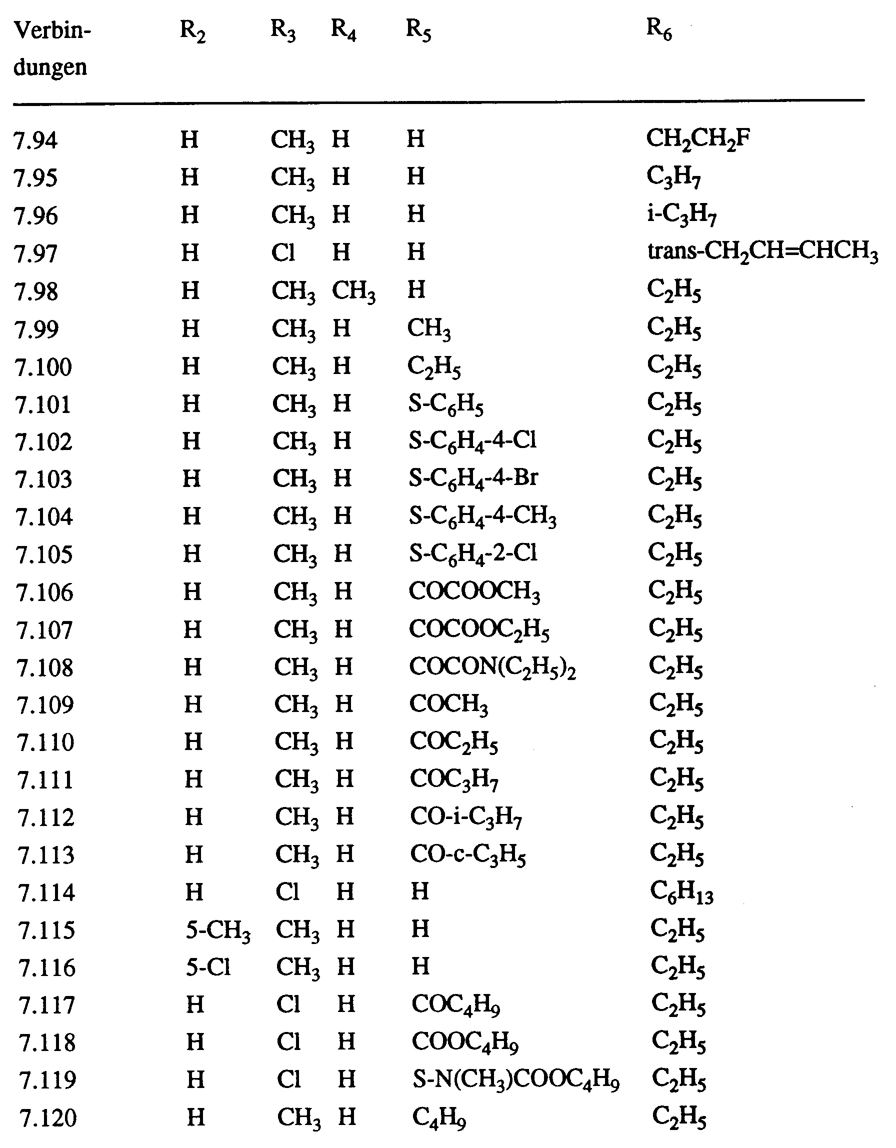

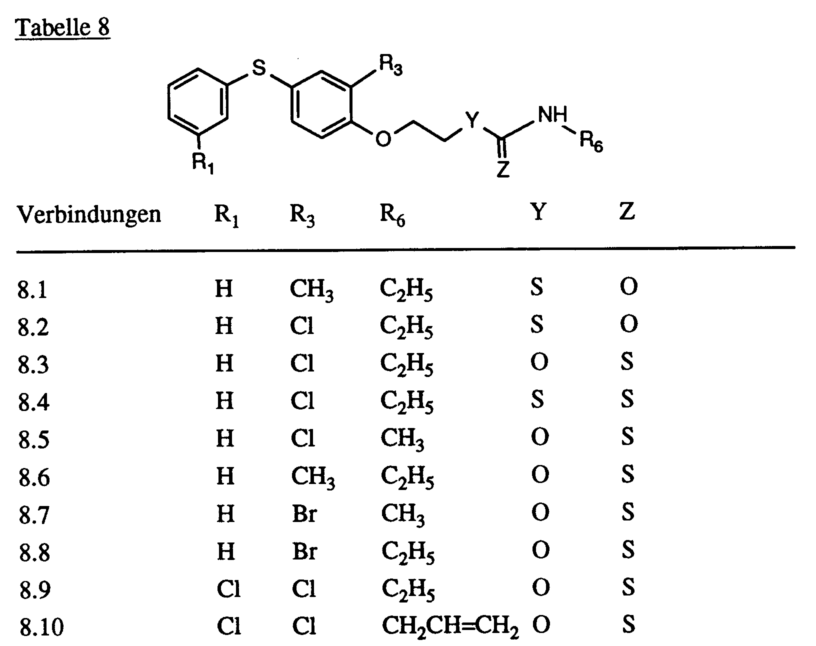

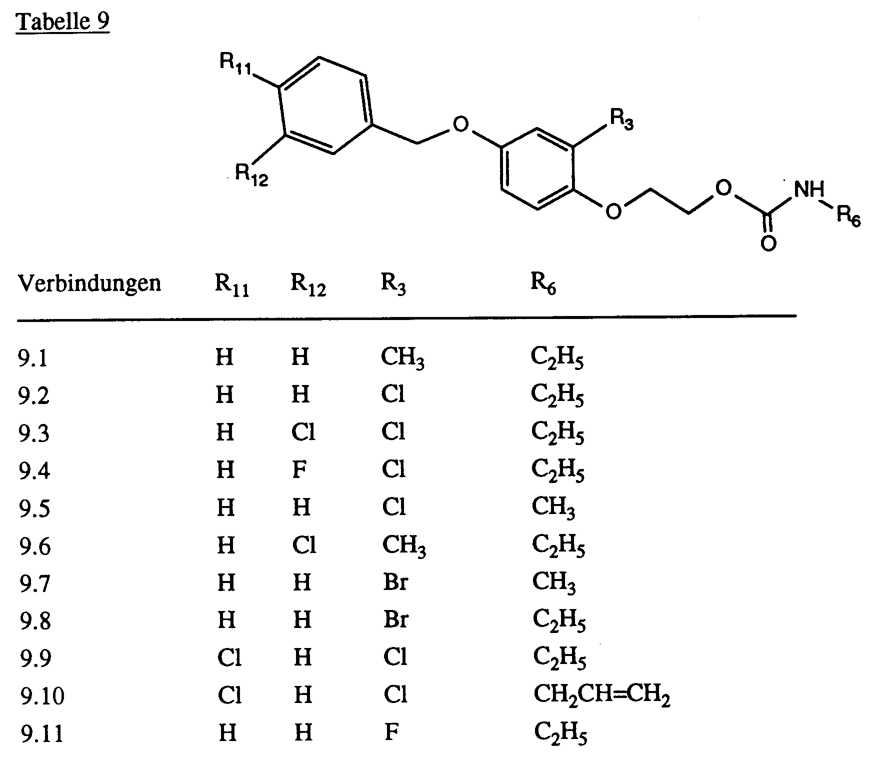

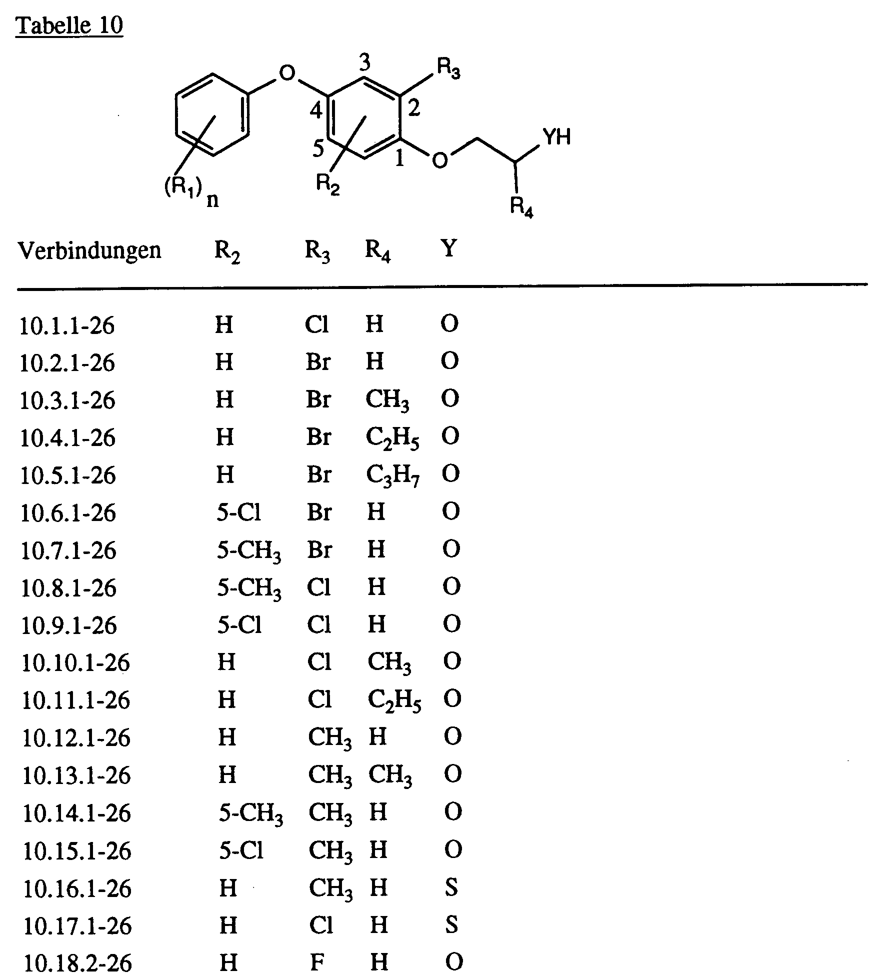

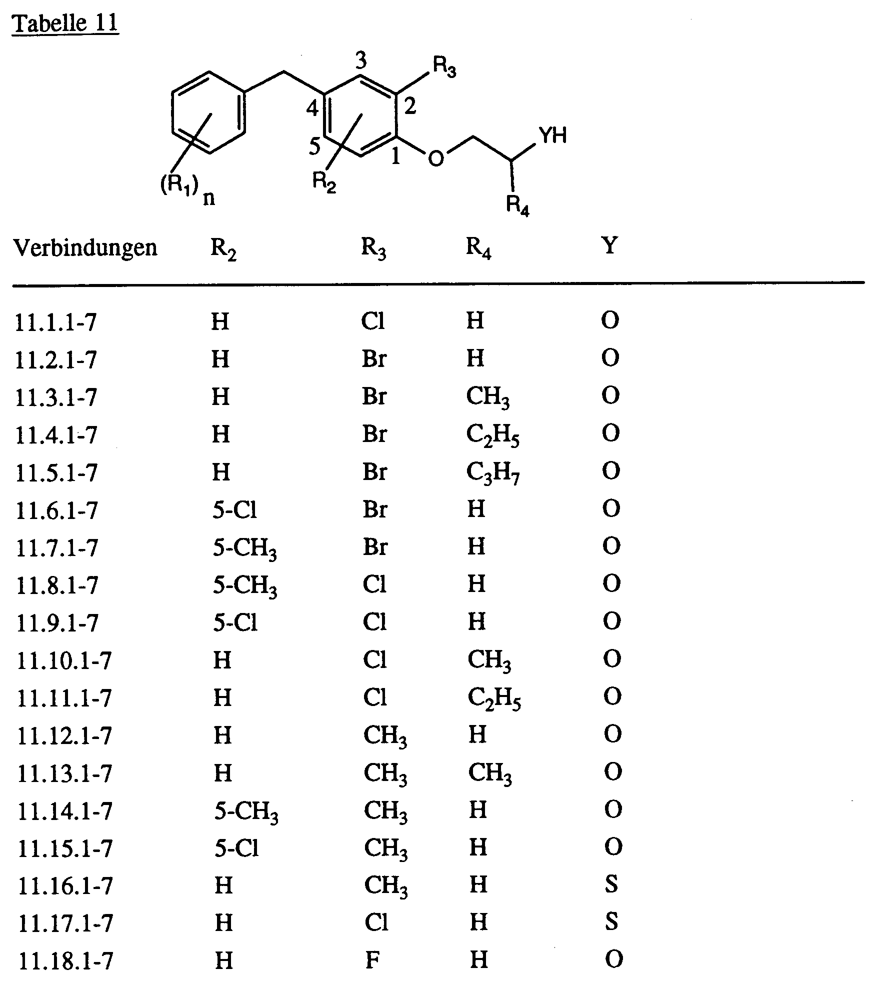

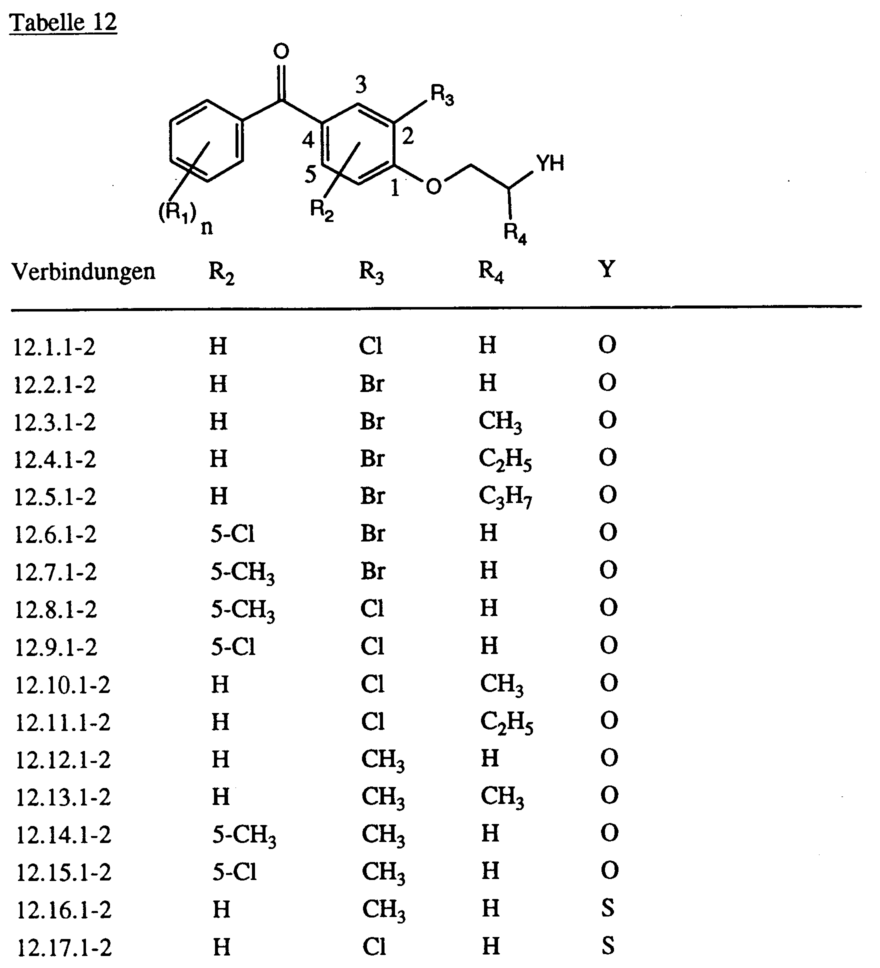

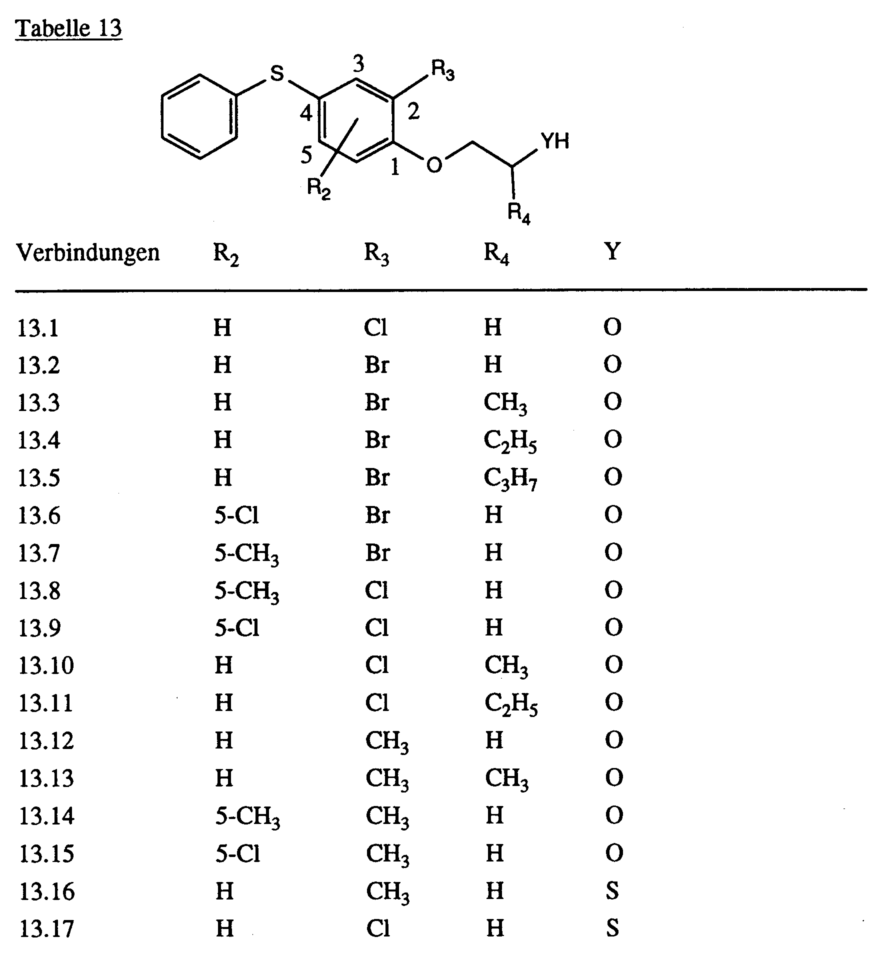

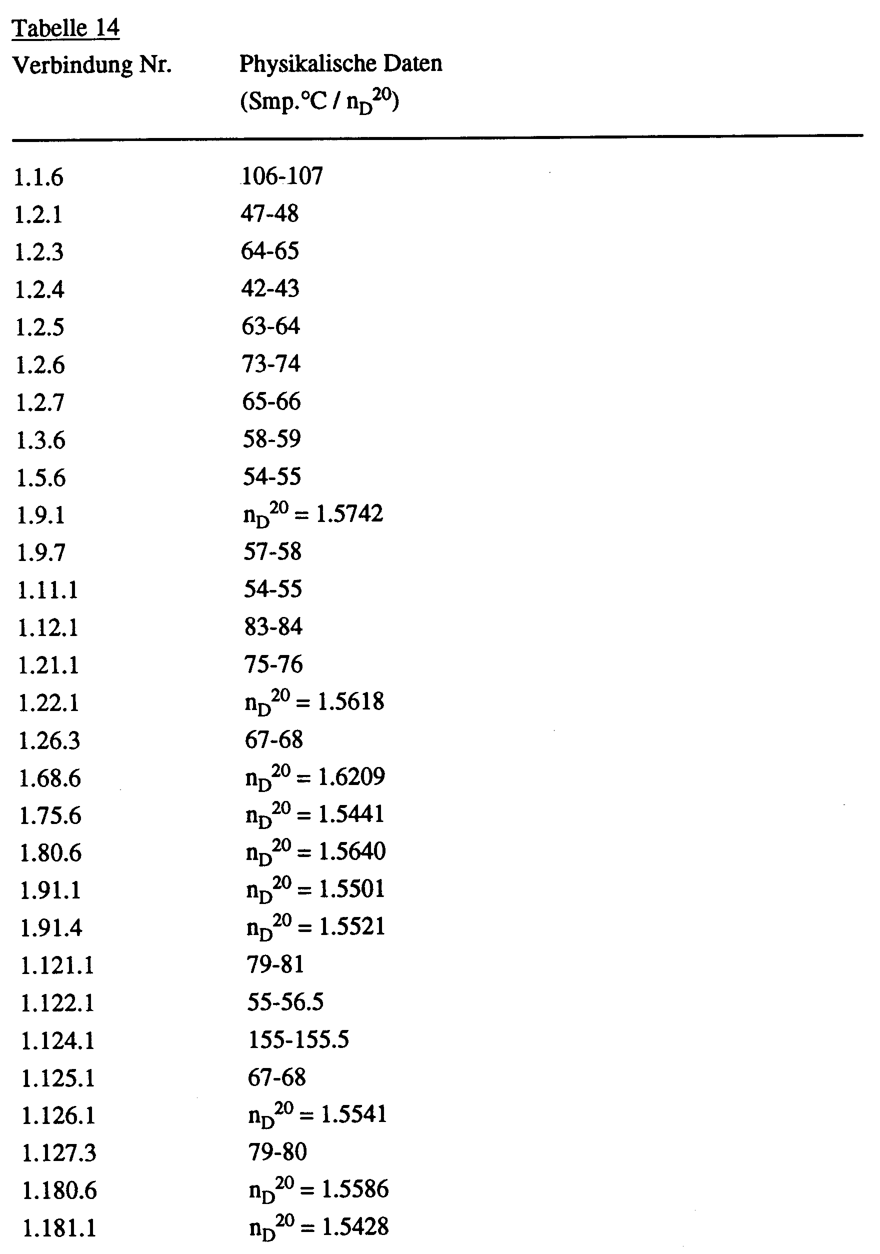

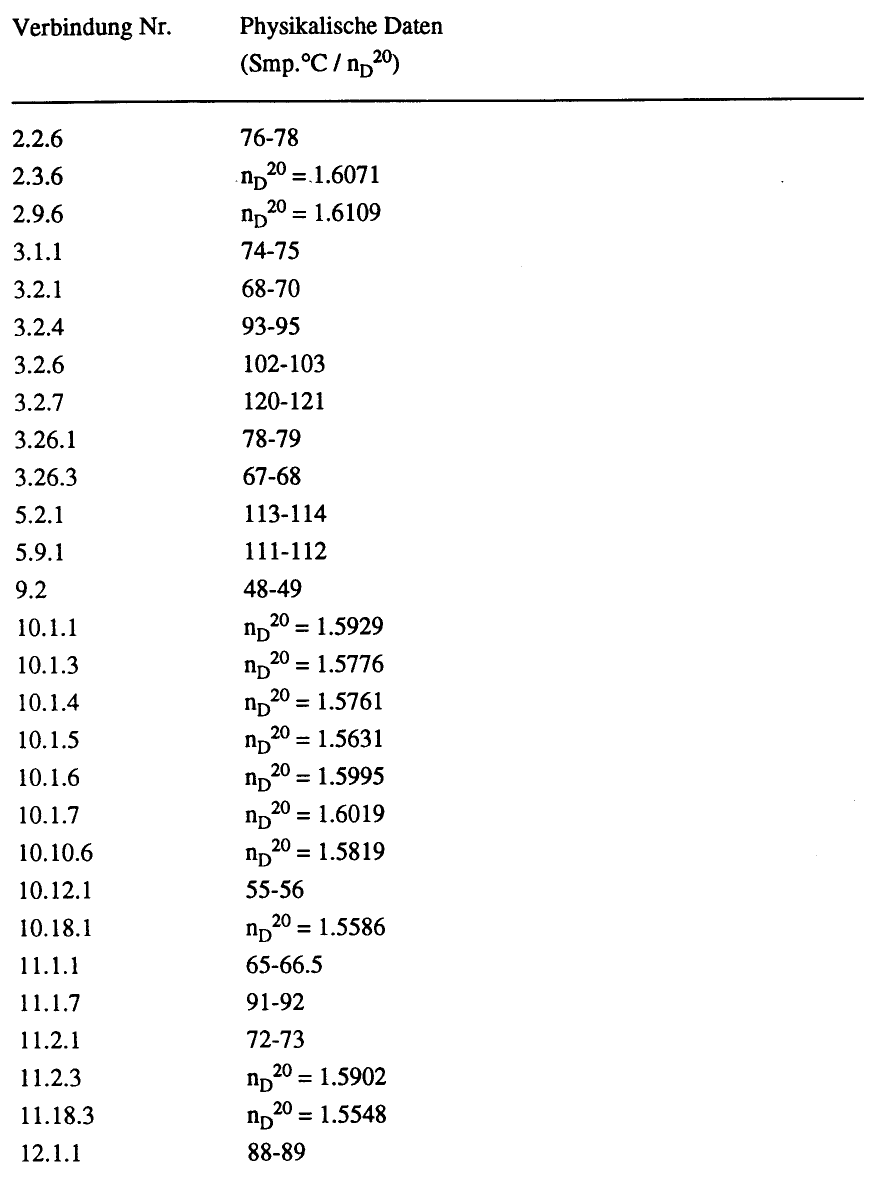

### Formulierungsbeispiele (% = Gewichtsprozent)

### Beispiel F1: Emulsions-Konzentrate

| | a) | b) | c) |
|---|---|---|---|
| Wirkstoff | 25 % | 40 % | 50 % |
| Ca-Dodecylbenzolsulfonat | 5 % | 8 % | 6 % |
| Ricinusölpolyethylenglykolether (36 Mol EO) | 5 % | - | - |
| Tributylphenolpolyethylenglykolether (30 Mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

### Beispiel F2: Lösungen

| | a) | b) | c) | d) |
|---|---|---|---|---|
| Wirkstoff | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

### Beispiel F3: Granulate

| | a) | b) | c) | d) |
|---|---|---|---|---|
| Wirkstoff | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

### Beispiel F4: Stäubemittel

| | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

### Beispiel F5: Spritzpulver

| | a) | b) | c) |
|---|---|---|---|
| Wirkstoff | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

### Beispiel F6: Emulsions-Konzentrat

| | |
|---|---|
| Wirkstoff | 10 % |
| Octylphenolpolyethylenglykolether (4-5 Mol EO) | 3 % |
| Ca-Dodecylbenzolsulfonat | 3 % |
| Ricinusölpolyglykolether (36 Mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

### Beispiel F7: Stäubemittel

| | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

### Beispiel F8: Extruder-Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

### Beispiel F9: Umhüllungs-Granulat

| | |
|---|---|
| Wirkstoff | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

### Beispiel F10: Suspensions-Konzentrat

| | |
|---|---|
| Wirkstoff | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele (% = Gewichtsprozent, sofern nichts anderes angegeben)

### A. Insektizide Wirkung

### Beispiel B1: Wirkung gegen Adoxophyes reticulana (ovizid)

Auf Filterpapier abgelegte Eier von Adoxophyes reticulana werden kurzzeitig in eine acetonisch-wässrige Testlösung, die 400 ppm Wirkstoff enthält, eingetaucht. Nach dem Antrocknen der Testlösung werden die Eier in Petrischalen inkubiert. Nach 6 Tagen wird der prozentuale Schlupf der Eier, verglichen mit unbehandelten Kontrollansätzen, ausgewertet (% Schlupfreduktion).
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### Beispiel B2: Wirkung gegen Aonidiella aurantii

Kartoffelknollen werden mit Wanderlarven ("Crawlern") von Aonidiella aurantii besiedelt. Nach etwa 2 Wochen werden die Kartoffeln in eine wässrige Emulsions- bzw. Suspensions-Spritzbrühe getaucht, die 400 ppm Wirkstoff enthält. Nach dem Abtrocknen der Knollen werden diese in einem Plastikbehälter inkubiert. Zur Auswertung wird 10 bis 12 Wochen später die Ueberlebensrate der Wanderlarven der ersten Folgegeneration der behandelten Population mit derjenigen von unbehandelten Kontrollansätzen verglichen.
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### Beispiel B3: Wirkung gegen Bemisia tabaci

Buschbohnenpflanzen werden in Gazekäfige gestellt und mit Adulten von Bemisia tabaci besiedelt. Nach erfolgter Eiablage werden alle Adulten entfernt. 10 Tage später werden die Pflanzen mit den darauf befindlichen Nymphen mit einer wässrigen Emulsionsspritzbrühe, die 400 ppm Wirkstoff enthält, besprüht. Nach weiteren 14 Tagen wird der prozentuale Schlupf der Eier im Vergleich zu unbehandelten Kontrollansätzen ausgewertet.
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### Beispiel B4: Wirkung gegen Bemisia tabaci

Buschbohnenpflanzen werden in Gazekäfige gestellt und mit Adulten von Bemisia tabaci besiedelt. Nach erfolgter Eiablage werden alle Adulten entfernt. 2 Tage später werden die Pflanzen mit den darauf befindlichen Nymphen mit einer wässrigen Emulsionsspritzbrühe, die 400 ppm Wirkstoff enthält, besprüht. Nach weiteren 10 Tagen wird der prozentuale Schlupf der Eier im Vergleich zu unbehandelten Kontrollansätzen ausgewertet.
Verbindungen der Tabellen 1 bis 9 und 14 zeigen gute Wirkung in diesem Test. Insbesondere zeigen die Verbindungen Nr. 1.2.1, 1.2.3, 1.2.4, 1.2.6, 1.2.7, 3.1.1 und 3.26.3 eine Wirkung von mehr als 80%.

### Beispiel B5: Wirkung gegen Cydia pomonella (ovizid)

Auf Filterpapier abgelegte Eier von Cydia pomonella werden kurzzeitig in eine acetonisch-wässrige Testlösung, die 400 ppm Wirkstoff enthält, eingetaucht. Nach dem Antrocknen der Testlösung werden die Eier in Petrischalen inkubiert. Nach 6 Tagen wird der prozentuale Schlupf der Eier, verglichen mit unbehandelten Kontrollansätzen, ausgewertet (% Schlupfreduktion).
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### Beispiel B6: Wirkung gegen Diabrotica balteata (ovizid)

20 bis 50 auf einem Tuchfilter abgelegte Eier von Diabrotica balteata werden in eine Petrischale gegeben und mit einer wässrigen Emulsionsspritzbrühe, die 400 ppm Wirkstoff enthält, behandelt. Die Petrischale wird bei 24° inkubiert. Nach 7 Tagen wird der prozentuale Schlupf der Eier, verglichen mit unbehandelten Kontrollansätzen, ausgewertet (% Schlupfreduktion).
Verbindungen der Tabellen 1 bis 9 und 14 zeigen gute Wirkung in diesem Test.

### Beispiel B7: Wirkung gegen Heliothis virescens (ovi-/larvizid)

Auf Baumwolle abgelegte Eier von Heliothis virescens werden mit einer wässrigen Emulsionsspritzbrühe, die 400 ppm Wirkstoff enthält, besprüht. Nach 8 Tagen werden der prozentuale Schlupf der Eier und die Ueberlebensraten der Raupen im Vergleich zu unbehandelten Kontrollansätzen ausgewertet (% Reduktion der Population).
Verbindungen der Tabellen 1 bis 9 und 14 zeigen gute Wirkung in diesem Test. Insbesondere zeigen die Verbindungen Nr. 1.2.1, 1.2.3, 1.2.4, 1.2.5, 1.2.6, 1.26.3 und 3.26.3 eine Wirkung von mehr als 80%.

### Beispiel B8: Wirkung gegen Heliothis virescens (ovizid)

Auf Filterpapier abgelegte Eier von Heliothis virescens werden kurzzeitig in eine acetonisch-wässrige Testlösung, die 400 ppm Wirkstoff enthält, eingetaucht. Nach dem Antrocknen der Testlösung werden die Eier in Petrischalen inkubiert. Nach 6 Tagen wird der prozentuale Schlupf der Eier, verglichen mit unbehandelten Kontrollansätzen, ausgewertet (% Schlupfreduktion).
Verbindungen der Tabellen 1 bis 9 und 14 zeigen gute Wirkung in diesem Test. Insbesondere zeigen die Verbindungen Nr. 1.1.6, 1.2.1, 1.2.3, 1.2.4, 1.2.5, 1.2.6, 1.26.3, 1.3.6, 1.9.1, 2.2.6, 2.9.6, 3.26.3, 5.2.1 und 5.9.1 eine Wirkung von mehr als 80%.

### Beispiel B9: Wirkung gegen Lobesia botrana (ovizid)

Auf Filterpapier abgelegte Eier von Lobesia botrana werden kurzzeitig in eine acetonisch-wässrige Testlösung, die 400 ppm Wirkstoff enthält, eingetaucht. Nach dem Antrocknen der Testlösung werden die Eier in Petrischalen inkubiert. Nach 6 Tagen wird der prozentuale Schlupf der Eier, verglichen mit unbehandelten Kontrollansätzen, ausgewertet (% Schlupfreduktion).
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### Beispiel B10: Wirkung gegen Nilaparvata lugens

Reispflanzen werden mit einer wässrigen Emulsionsspritzbrühe, die 400 ppm Wirkstoff enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten und auf unbehandelten Pflanzen wird die prozentuale Reduktion der Population (% Wirkung) bestimmt.
Verbindungen der Tabellen 1 bis 9 und 14 zeigen gute Wirkung in diesem Test. Insbesondere zeigen die Verbindungen Nr. 1.2.1, 1.2.3 und 1.2.6 eine Wirkung von mehr als 80%.

### B. Akarizide Wirkung

### Beispiel B11: Wirkung gegen Boophilus microplus

Vollgesogene adulte Zeckenweibchen werden auf eine PVC-Platte aufgeklebt und mit einem Wattebausch überdeckt. Zur Behandlung werden die Testtiere mit 10 ml einer wässrigen Testlösung, die 125 ppm Wirkstoff enthält, übergossen. Anschliessend wird der Wattebausch entfernt und die Zecken werden 4 Wochen zur Eiablage inkubiert. Die Wirkung gegen Boophilus microplus zeigt sich entweder beim Weibchen als Mortalität oder Sterilität oder bei den Eiern als ovizide Wirkung.
Verbindungen der Tabellen 1 bis 9 und 14 zeigen gute Wirkung in diesem Test.

## Patentansprüche

1. Eine Verbindung der Formel 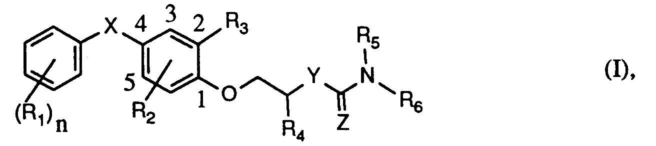 worin
R₁ Halogen, C₁-C₃-Alkyl, Halogen-C₁-C₃-alkyl, C₁-C₃-Alkoxy, Halogen-C₁-C₃-alkoxy oder Cyano und/oder zwei an benachbarte C-Atome des Phenylrings gebundene Substituenten R₁ gemeinsam -O-CH₂-O-;
R₂ Wasserstoff, Halogen oder Methyl;
R₃ Fluor, Chlor, Brom oder C₁-C₃-Alkyl;
R₄ Wasserstoff oder C₁-C₃-Alkyl;
entweder R₅ Wasserstoff, C₁-C₄-Alkyl, 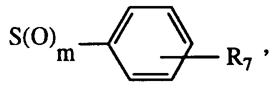 -COCO-R₈, -CO-R₉ oder S(O)ₘ-N(R₁₀)-COO-R₁₁ und
R₆ C₁-C₆-Alkyl, Halogen-C₁-C₄-alkyl, C₂-C₆-Alkenyl, Halogen-C₃-C₄-alkenyl, C₃-C₅-Alkinyl, Halogen-C₃-C₅-alkinyl, C₁-C₃-Alkoxy-C₁-C₃-alkyl, C₃-C₆-Cycloalkyl, unsubstituiertes Phenyl oder Phenyl welches ein- bis dreifach substituiert ist mit gleichen oder verschiedenen Substituenten ausgewählt aus der Gruppe bestehend aus Halogen, C₁-C₄-Alkyl und C₁-C₄-Alkoxy;
oder R₅ und R₆ gemeinsam -(CH₂)₄- oder -(CH₂)₅-;
R₇ Wasserstoff, Halogen oder C₁-C₃-Alkyl;
R₈ C₁-C₈-Alkoxy oder -N(R₁₂)₂;
R₉ C₁-C₄-Alkyl, C₃-C₆-Cycloalkyl, -N(R₁₃)₂ oder C₁-C₄-Alkoxy;
R₁₀ C₁-C₃-Alkyl;
R₁₁ C₁-C₆-Alkyl;
R₁₂ unabhängig voneinander C₁-C₈-Alkyl;
R₁₃ unabhängig voneinander C₁-C₄-Alkyl;
m die Zahl 0, 1 oder 2;
n die Zahl 0, 1, 2 oder 3, wobei, wenn n 2 oder 3 ist, die Reste R₁ gleich oder verschieden sein können;
X O, S, CH₂, CO oder -O-CH₂-;
Y O oder S; und
Z O oder S bedeuten,
mit Ausnahme von 1-(2-Fluor-4-phenoxy-phenoxy)-2-ethylaminocarbonyloxy-ethan, jeweils in freier Form oder in Salzform.

2. Eine Verbindung gemäss Anspruch 1 der Formel I, worin (R₁)ₙ (R₁)₀, Monofluor, Monochlor, Monobrom, Difluor, Dichlor, Monochlor-monofluor, Monobrom-monofluor, Monomethyl, Monoethyl, Dimethyl, Monomethoxy, Monocyano, Monotrifluormethyl, Monotrifluormethoxy oder Monomethylendioxy bedeutet.

3. Eine Verbindung gemäss Anspruch 1 oder 2 der Formel I, worin R₂ Wasserstoff, Chlor oder Methyl ist.

4. Eine Verbindung gemäss einem der Ansprüche 1 bis 3 der Formel I, worin R₃ Chlor, Brom oder Methyl ist.

5. Eine Verbindung gemäss einem der Ansprüche 1 bis 3 der Formel I, worin R₃ Fluor ist.

6. Eine Verbindung gemäss einem der Ansprüche 1 bis 5 der Formel I, worin R₄ Wasserstoff ist.

7. Eine Verbindung gemäss einem der Ansprüche 1 bis 6 der Formel I, worin R₅ Wasserstoff, C₁-C₄-Alkyl, -S-C₆H₄-Cl, -COCO-R₈, worin R₈ C₁-C₄-Alkoxy ist, oder -CO-R₉, worin R₉ C₁-C₄-Alkyl, C₁-C₄-Alkoxy oder C₃-C₆-Cycloalkyl ist.

8. Eine Verbindung gemäss einem der Ansprüche 1 bis 7 der Formel I, worin R₆ C₁-C₆-Alkyl, Halogen-C₁-C₃-alkyl, C₂-C₄-Alkenyl, Halogen-C₃-C₄-alkenyl, C₃-C₄-Alkinyl, C₁-C₂-Alkoxy-C₁-C₂-alkyl oder C₃-C₆-Cycloalkyl ist.

9. Eine Verbindung gemäss einem der Ansprüche 1 bis 6 der Formel I, worin R₅ und R₆ gemeinsam -(CH₂)₄- oder -(CH₂)₅- bedeuten.

10. Eine Verbindung gemäss einem der Ansprüche 1 bis 9 der Formel I, worin X O, CH₂, CO oder -O-CH₂- bedeutet.

11. Eine Verbindung gemäss einem der Ansprüche 1 bis 10 der Formel I, worin Y O ist.

12. Eine Verbindung gemäss einem der Ansprüche 1 bis 11 der Formel I, worin Z O ist.

13. Eine Verbindung gemäss Anspruch 1 der Formel I, worin (R₁)ₙ (R₁)₀, 2-, 3- oder 4-Fluor, 3- oder 4-Chlor, 4-Brom, 2,4- oder 3,5-Difluor, 3,4- oder 3,5-Dichlor, 3-Chlor-4-fluor, 4-Brom-2-fluor, 3- oder 4-Methyl, 3- oder 4-Ethyl, 3,5-Dimethyl, 3- oder 4-Methoxy, 4-Cyano, 3- oder 4-Trifluormethyl, 3- oder 4-Trifluormethoxy oder 3,4-Methylendioxy, R₂ Wasserstoff, Chlor oder Methyl, R₃ Fluor, Chlor, Brom oder Methyl, R₄ Wasserstoff, entweder R₅ Wasserstoff, C₁-C₄-Alkyl, 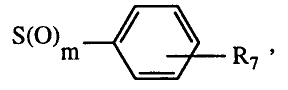 -COCO-R₈ oder -CO-R₉ und R₆ C₁-C₆-Alkyl, Halogen-C₁-C₃-alkyl, C₂-C₄-Alkenyl, C₃-C₄-Alkinyl, C₁-C₂-Alkoxy-C₁-C₂-alkyl oder einfach mit Halogen substituiertes Phenyl; oder R₅ und R₆ gemeinsam -(CH₂)₄- oder -(CH₂)₅-; X O, CH₂, CO oder -O-CH₂-; Y O oder S, Z O oder S, R₇ Wasserstoff oder Halogen; R₈ C₁-C₄-Alkoxy ; R₉ C₁-C₄-Alkyl oder C₃-C₆-Cycloalkyl und m 0 oder 1 bedeuten.

14. Eine Verbindung gemäss Anspruch 13 der Formel I, worin (R₁)ₙ (R₁)₀, 3- oder 4-Fluor, 3-Chlor, 3,5-Difluor oder 3,4-Dichlor, R₂ Wasserstoff, R₃ Chlor, R₄ Wasserstoff, entweder R₅ Wasserstoff und R₆ C₁-C₃-Alkyl, Halogen-C₁-C₂-alkyl, C₂-C₄-Alkenyl, C₃-C₄-Alkinyl, C₁-C₂-Alkoxy-C₁-C₂-alkyl oder einfach mit Chlor substituiertes Phenyl; oder R₅ und R₆ gemeinsam -(CH₂)₄-; X O, CH₂, CO oder -O-CH₂-; Y O oder S und Z O oder S bedeuten.

15. Eine Verbindung gemäss Anspruch 14 der Formel I, worin (R₁)ₙ (R₁)₀, R₂ Wasserstoff, R₃ Fluor, R₄ Wasserstoff, R₅ Wasserstoff, R₆ C₁-C₃-Alkyl und X, Y und Z jeweils O bedeuten.

16. Eine Verbindung gemäss Anspruch 15 der Formel I, worin (R₁)ₙ (R₁)₀, 3-Chlor oder 3,5-Difluor, R₂ Wasserstoff, R₃ Chlor, R₄ Wasserstoff, R₅ Wasserstoff, R₆ C₁-C₂-Alkyl oder Chlor-C₁-C₂-alkyl und X, Y und Z jeweils O bedeuten.

17. Eine Verbindung gemäss Anspruch 1 der Formel I, ausgewählt aus der Gruppe von Verbindungen bestehend aus
1-[2-Chlor-4-(3-chlorphenoxy)-phenoxyl-2-ethylaminocarbonyloxy-ethan,
1-(2-Chlor-4-phenoxy-phenoxy)-2-ethylaminocarbonyloxy-ethan und
1-[2-Chlor-4-(3,5-difluorphenoxy)-phenoxyl-2-ethylaminocarbonyloxy-ethan.

18. Verfahren zur Herstellung einer Verbindung der Formel I, mit Ausnahme von 1-(2-Fluor-4-phenoxy-phenoxy)-2-ethylaminocarbonyloxy-ethan, jeweils in freier Form oder in Salzform dadurch gekennzeichnet dass man
(a) eine Verbindung der Formel 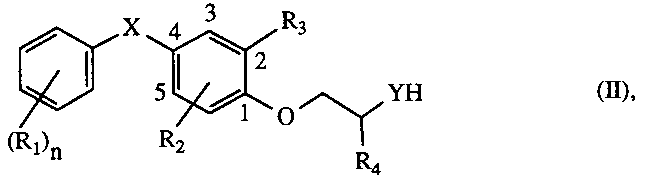 worin R₁, R₂, R₃, R₄, n, X und Y die für die Formel I angegebenen Bedeutungen haben, oder ein Salz davon, mit einer Verbindung der Formel
L-C(=Z)-N(R₅)R₆ (III),
worin R₅, R₆ und Z die für die Formel I angegebenen Bedeutungen haben und L für eine Abgangsgruppe steht, oder einem Salz davon umsetzt, oder
b) zur Herstellung einer Verbindung der Formel I, worin R₅ Wasserstoff ist, oder eines Salzes davon eine Verbindung der Formel (II) oder ein Salz davon, mit einer Verbindung der Formel
R₆N=C=Z (IV),
worin R₆ und Z die für die Formel I angegebenen Bedeutungen haben, umsetzt, oder
c) eine Verbindung der Formel 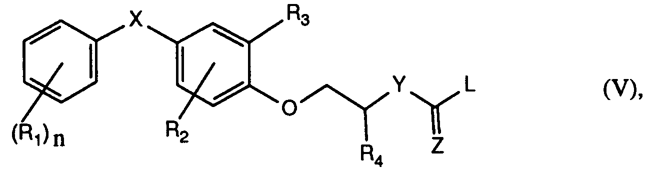 worin R₁, R₂, R₃, R₄, n, X, Y und Z die für die Formel I angegebenen Bedeutungen haben und L für eine Abgangsgruppe steht, oder ein Salz davon, mit einer Verbindung der Formel
H-N(R₅)R₆ (VI),
worin R₅ und R₆ die für die Formel I angegebenen Bedeutungen haben, oder einem Salz davon umsetzt, oder
d) eine Verbindung der Formel 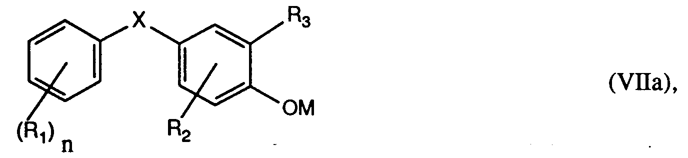 worin R₁, R₂, R₃, n und X die für die Formel I angegebenen Bedeutungen haben und M für ein Kation steht, mit einer Verbindung der Formel 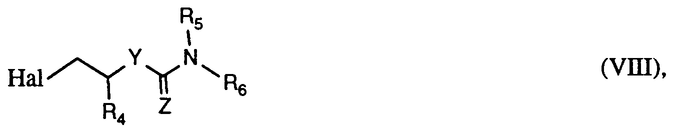 worin R₄, R₅, R₆, Y und Z die in Formel 1 angegebene Bedeutung besitzen und Hal ein Halogenatom bedeutet, umsetzt, und/oder, wenn erwünscht, eine verfahrensgemäss oder auf andere Weise erhältliche Verbindung der Formel I, in freier Form oder in Salzform, in eine andere Verbindung der Formel I überführt, ein verfahrensgemäss erhältliches Gemisch von Isomeren auftrennt und das gewünschte Isomere isoliert und/oder eine verfahrensgemäss oder auf andere Weise erhältliche freie Verbindung der Formel I in ein Salz oder ein verfahrensgemäss oder auf andere Weise erhältliches Salz einer Verbindung der Formel I in die freie Verbindung der Formel I oder in ein anderes Salz überführt.

19. Schädlingsbekämpfungsmittel, dadurch gekennzeichnet, dass es mindestens eine Verbindung gemäss Anspruch 1 der Formel I, mit Ausnahme von 1-(2-Fluor-4-phenoxy-phenoxy)-2-ethylaminocarbonyloxy-ethan, jeweils in freier Form oder in agrochemisch verwendbarer Salzform, als Wirkstoff und gegebenenfalls mindestens einen Hilfsstoff enthält.

20. Mittel gemäss Anspruch 19 zur Bekämpfung von Insekten und Vertretern der Ordnung Akarina.

21. Verfahren zur Herstellung eines mindestens einen Hilfsstoff enthaltenden Mittels gemäss Anspruch 19 oder 20, dadurch gekennzeichnet, dass man den Wirkstoff mit dem (den) Hilfsstoff(en) innig vermischt und/oder vermahlt.

22. Verfahren zur Bekämpfung von Schädlingen, dadurch gekennzeichnet, dass man ein Mittel gemäss Anspruch 19 oder 20 auf die Schädlinge oder ihren Lebensraum appliziert.

23. Verfahren gemäss Anspruch 22 zur Bekämpfung von Insekten und Vertretern der Ordnung Akarina.

24. Verfahren gemäss Anspruch 22 oder 23 zum Schutz von pflanzlichem Vermehrungsgut, dadurch gekennzeichnet, dass man das Vermehrungsgut oder den Ort der Ausbringung des Vermehrungsguts behandelt.

25. Pflanzliches Vermehrungsgut, behandelt gemäss dem in Anspruch 24 beschriebenen Verfahren.

26. Eine Verbindung der Formel 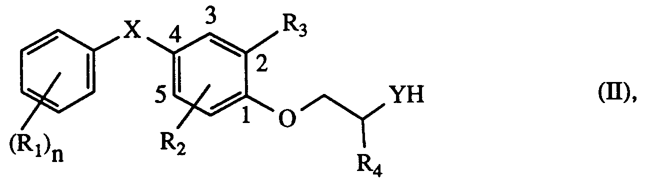 worin R₁, R₂, R₃, R₄, n, X und Y die für die Formel I angegebenen Bedeutungen haben, mit Ausnahme von 2-(2-Fluor-4-phenoxy-phenoxy)ethanol, jeweils in freier Form oder in Salzform.

27. Ein Verfahren zur Herstellung einer Verbindung gemäss Anspruch 26 der Formel II, in freier Form oder in Salzform, dadurch gekennzeichnet, dass man
e) eine Verbindung der Formel (VIIa) oder 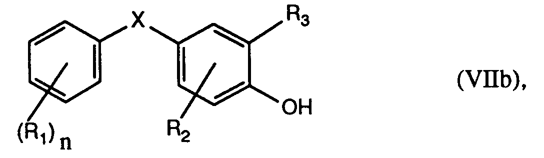 worin die R₁, R₂, R₃, X und n die in Formel I angegebene Bedeutung haben, in Gegenwart eines Alkylierungskatalysators mit einer Verbindung der Formel 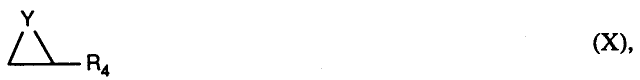 worin R₄ und Y die für die Formel I angegebenen Bedeutungen haben, umsetzt, oder
f) zur Herstellung einer Verbindung der Formel II, worin R₄ Wasserstoff und Y O ist, oder eines Salzes davon, eine Verbindung der Formel (VIIa) oder (VIIb) mit 2-Oxo-1,3-dioxolan umsetzt, oder
g) zur Herstellung einer Verbindung der Formel II, worin R₄ Wasserstoff und Y O ist, oder eines Salzes davon eine Verbindung der Formel 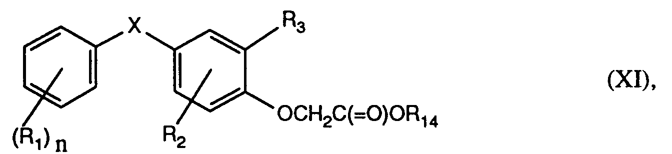 worin R₁, R₂, R₃, n und X die für die Formel I angegebenen Bedeutungen haben und R₁₄ C₁-C₈-Alkyl ist, mit einem Reduktionsmittel umsetzt
und/oder, wenn erwünscht, eine verfahrensgemäss oder auf andere Weise erhältliche Verbindung der Formel II, in freier Form oder in Salzform, in eine andere Verbindung der Formel II überführt, ein verfahrensgemäss erhaltliches Gemisch von Isomeren auftrennt und das gewünschte Isomere isoliert und/oder eine verfahrensgemäss oder auf andere Weise erhältliche freie Verbindung der Formel II in ein Salz oder ein verfahrensgemäss oder auf andere Weise erhältliches Salz einer Verbindung der Formel II in die freie Verbindung der Formel II oder in ein anderes Salz überführt.

## Claims

1. A compound of the formula 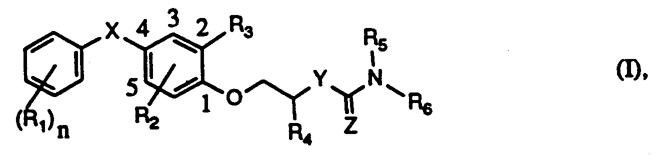 in which
R₁ is halogen, C₁-C₃alkyl, halo-C₁-C₃alkyl, C₁-C₃alkoxy, halo-C₁-C₃alkoxy or cyano and/or two substituents R₁ which are bonded to adjacent C atoms of the phenyl ring together are -O-CH₂-O-;
R₂ is hydrogen, halogen or methyl;
R₃ is fluorine, chlorine, bromine or C₁-C₃alkyl;
R₄ is hydrogen or C₁-C₃alkyl;
either R₅ is hydrogen, C₁-C₄alkyl, 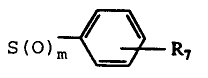 -COCO-R₈, -CO-R₉ or S(O)ₘ-N(R₁₀)-COOR₁₁ and
R₆ is C₁-C₆alkyl, halo-C₁-C₄alkyl, C₂-C₆alkenyl, halo-C₃-C₄alkenyl, C₃-C₅alkynyl, halo-C₃-C₅alkynyl, C₁-C₃alkoxy-C₁-C₃alkyl, C₃-C₆cycloalkyl, unsubstituted phenyl or phenyl which is monosubstituted to trisubstituted by identical or different substituents selected from the group consisting of halogen, C₁-C₄alkyl and C₁-C₄alkoxy;
or R₅ and R₆ together are -(CH₂)₄- or -(CH₂)₅-;
R₇ is hydrogen, halogen or C₁-C₃alkyl;
R₈ is C₁-C₈alkoxy or -N(R₁₂)₂;
R₉ is C₁-C₄alkyl, C₃-C₆cycloalkyl, -N(R₁₃)₂ or C₁-C₄alkoxy;
R₁₀ is C₁-C₃alkyl;
R₁₁ is C₁-C₆alkyl;
R₁₂ radicals independently of one another are C₁-C₈alkyl;
R₁₃ radicals independently of one another are C₁-C₄alkyl;
m is the number 0, 1 or 2;
n is the number 0, 1, 2 or 3 where, if n is 2 or 3, the radicals R₁ can be identical or different;
X is O, S, CH₂, CO or -O-CH₂-;
Y is O or S; and
Z is O or S,
with the exception of 1-(2-fluoro-4-phenoxyphenoxy)-2-ethylaminocarbonyloxyethane in each case in free form or in salt form.

2. A compound according to claim 1 of the formula I in which (R₁)ₙ is (R₁)₀, monofluoro, monochloro, monobromo, difluoro, dichloro, monochloromonofluoro, monobromomonofluoro, monomethyl, monoethyl, dimethyl, monomethoxy, monocyano, monotrifluoromethyl, monotrifluoromethoxy or monomethylenedioxy.

3. A compound according to claim 1 or 2 of the formula I in which R₂ is hydrogen, chlorine or methyl.

4. A compound according to any one of claims 1 to 3 of the formula I in which R₃ is chlorine, bromine or methyl.

5. A compound according to any one of claims 1 to 3 of the formula I in which R₃ is fluorine.

6. A compound according to any one of claims 1 to 5 of the formula I in which R₄ is hydrogen.

7. A compound according to any one of claims 1 to 6 of the formula I in which R₅ is hydrogen, C₁-C₄alkyl, -S-C₆H₄-Cl, -COCO-R₈ is C₁-C₄alkoxy, or -CO-R₉, in which R₉ is C₁-C₄alkyl, C₁-C₄alkoxy or C₃-C₆cycloalkyl.

8. A compound according to any one of claims 1 to 7 of the formula I in which R₆ is C₁-C₆alkyl, halo-C₁-C₃alkyl, C₂-C₄alkenyl, halo-C₃-C₄alkenyl, C₃-C₄alkynyl, C₁-C₂alkoxy-C₁-C₂alkyl or C₃-C₆cycloalkyl.

9. A compound according to any one of claims 1 to 6 of the formula I in which R₅ and R₆ together are -(CH₂)₄- or -(CH₂)₅-.

10. A compound according to any one of claims 1 to 9 of the formula I in which X is O, CH₂, CO or -O-CH₂-.

11. A compound according to any one of claims 1 to 10 fo the formula I in which Y is O.

12. A compound according to any one of claims 1 to 11 of the formula I in which Z is O.

13. A compound according to claim 1 of the formula I in which (R₁)ₙ is (R₁)₀, 2-, 3- or 4-fluoro, 3- or 4-chloro, 4-bromo, 2,4- or 3,5-difluoro, 3,4- or 3,5-dichloro, 3-chloro-4-fluoro, 4-bromo-2-fluoro, 3- or 4-methyl, 3- or 4-ethyl, 3,5-dimethyl, 3- or 4-methoxy, 4-cyano, 3- or 4-trifluoromethyl, 3- or 4-trifluoromethoxy or 3,4-methylenedioxy, R₂ is hydrogen, chlorine or methyl, R₃ is fluorine, chlorine, bromine or methyl, R₄ is hydrogen, either R₅ is hydrogen, C₁-C₄alkyl, 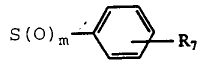 -COCO-R₈ or -CO-R₉ and R₆ is C₁-C₆alkyl, halo-C₁-C₃alkyl, C₂-C₄alkenyl, C₃-C₄-alkynyl, C₁-C₂alkoxy-C₁-C₂alkyl or phenyl which is monosubstituted by halogen; or R₅ and R₆ together are -(CH₂)₄- or -(CH₂)₅-; X is O, CH₂, CO or -O-CH₂-; Y is O or s, Z is O or S, R₇ is hydrogen or halogen; R₈ is C₁-C₄alkoxy; R₉ is C₁-C₄alkyl or C₃-C₆cycloalkyl and m is 0 or 1.

14. A compound according to claim 13 of the formula I in which (R₁)ₙ is (R₁)₀, 3- or 4-fluoro, 3-chloro, 3,5-difluoro or 3,4-dichloro, R₂ is hydrogen, R₃ is chlorine, R₄ is hydrogen, either R₅ is hydrogen and R₆ is C₁-C₃alkyl, halo-C₁-C₂alkyl, C₂-C₄alkenyl, C₃-C₄alkynyl, C₁-C₂alkoxy-C₁-C₂alkyl or phenyl which is monosubstituted by chlorine; or R₅ and R₆ together are -(CH₂)₄-; X is O, CH₂, CO or -O-CH₂-; Y is O or s and Z is O or S.

15. A compound according to claim 14 of the formula I in which (R₁)ₙ is (R₁)₀, R₂ is hydrogen, R₃ is fluorine, R₄ is hydrogen, R₅ is hydrogen, R₆ is C₁-C₃alkyl and X, Y and Z are in each case O.

16. A compound according to claim 15 of the formula I in which (R₁)ₙ is (R₁)₀, 3-chloro or 3,5-difluoro, R₂ is hydrogen, R₃ is chlorine, R₄ is hydrogen, R₅ is hydrogen, R₆ is C₁-C₂alkyl or chloro-C₁-C₂alkyl and X, Y and Z are in each case O.

17. A compound according to claim 1 of the formula I selected from the group of compounds consisting of
1-[2-chloro-4-(3-chlorophenoxy)phenoxy]-2-ethylaminocarbonyloxyethane,
1-(2-chloro-4-phenoxyphenoxy)-2-ethylaminocarbonyloxyethane and
1-[2-chloro-4-(3,5-difluorophenoxy)phenoxy]-2-ethylaminocarbonyloxyethane.

18. A process for the preparation of a compound of the formula I, with the exception of 1-(2-fluoro-4-phenoxyphenoxy)-2-ethylaminocarbonyloxyethane in each case in free form or in salt form, which comprises
a) reacting a compound of the formula 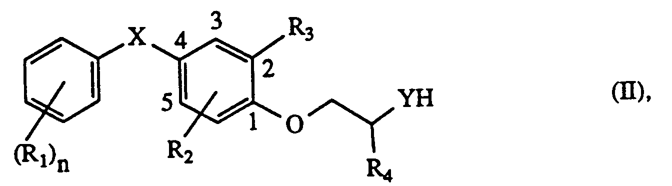 in which R₁, R₂, R₃, R₄, n, X and Y are as defined in formula I, or a salt thereof, with a compound of the formula
L-C(=Z)-N(R₅)R₆ (III),
in which R₅, R₆ and Z are as defined in formula I and L is a leaving group, or a salt thereof, or
b) to prepare a compound of the formula I in which R₅ is hydrogen, or a salt thereof, reacting a compound of the formula (II) or a salt thereof with a compound of the formula
R₆N=C=Z (IV),
in which R₆ and Z are as defined in formula I, or
c) reacting a compound of the formula 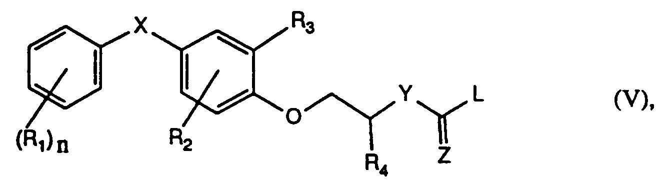 in which R₁, R₂, R₃, R₄, n, X, Y and Z are as defined in formula I and L is a leaving group,
or a salt thereof, with a compound of the formula
H-N(R₅)R₆ (VI),
in which R₅ and R₆ are as defined in formula I, or a salt thereof, or
d) reacting a compound of the formula 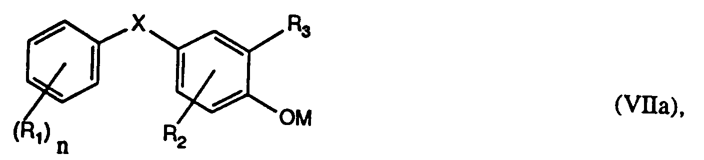 in which R₁, R₂, R₃, n and X are as defined in formula I and M is a cation, with a compound of the formula 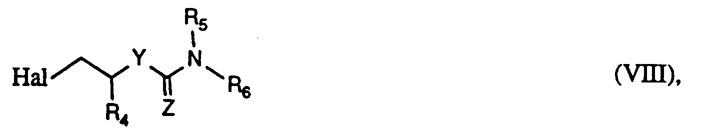 in which R₄, R₅, R₆, Y and Z are as defined in formula I and Hal is a halogen atom, and/or, if desired, converting a compound of the formula I which can be obtained according to the process or via a different route, in free form or in salt form, into a different compound of the formula I, separating an isomer mixture which can be obtained according to the process and isolating the desired isomer, and/or converting a free compound of the formula I which can be obtained according to the process or via a different route into a salt or converting a salt of a compound of the formula I which can be obtained according to the process or via a different route into the free compound of the formula I or into a different salt.

19. A pesticide which comprises at least one compound according to claim 1 of the formula I, with the exception of 1-(2-fluoro-4-phenoxyphenoxy)-2-ethylaminocarbonyloxyethane, in each case in free form or in agrochemically utilizable salt form, as active ingredient and, if desired, at least one auxiliary.

20. A composition according to claim 19 for controlling insects and representatives of the order Acarina.

21. A process according to claim 19 or 20 for the preparation of a composition which comprises at least one auxiliary, which process comprises intimately mixing and/or grinding the active ingredient with the auxiliary or auxiliaries.

22. A method of controlling pests, which comprises applying a composition according to claim 19 or 20 to the pests or their environment.

23. A method according to claim 22 of controlling insects and representatives of the order Acarina.

24. A method according to claim 22 or 23 of protecting of plant propagation material, which comprises treating the propagation material or the site where the propagation material is planted.

25. a plant propagation material treated according to the method described in claim 24.

26. A compound of the formula 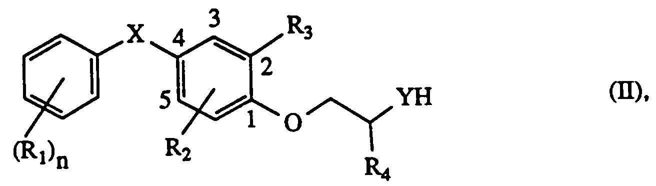 in which R₁, R₂, R₃, R₄, n, X and Y are as defined in formula I, with the exception of 2-(2-fluoro-4-phenoxyphenoxy)ethanol, in each case in free form or in salt form.

27. A process for the preparation of a compound according to claim 26 of the formula II, in free form or in salt form, which comprises
e) reacting a compound of the formula (VIIa) or 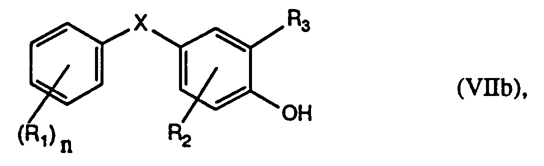 in which R₁, R₂, R₃, X and n are as defined in formula I, with a compound of the formula 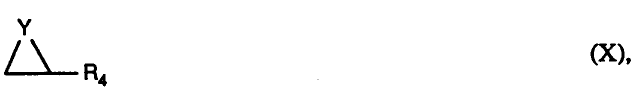 in which R₄ and Y are as defined in formula I, in the presence of alkylation catalyst, or
f) to prepare a compound of the formula II in which R₄ is hyrogen and Y is O, or a salt thereof, reacting a compound of the formula (VIIa) or (VIIb) with 2-oxo-1,3-dioxolane, or
g) to prepare a compound of the formula II in which R₄ is hydrogen and Y is O, or a salt thereof, reacting a compound of the formula 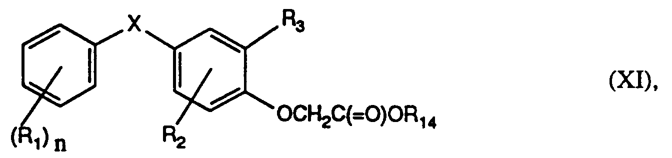 in which R₁, R₂, R₃, n and X are as defined in formula I and R₁₄ is C₁-C₈alkyl, with a reducing agent,
and/or, if desired, converting a compound of the formula II which can be obtained according to the process or via a different route, in free form or in salt form, into a different compound of the formula II, separating an isomer mixture which can be obtained according to the process and isolating the desired isomer, and/or converting a free compound of the formula II which can be obtained according to the process or via a different route into a salt, or converting a salt of a compound of the formula II whwich can be obtained according to the process of via a different route into the free compound of the formula Ii or into a different salt.

## Revendications

1. Un composé de formule 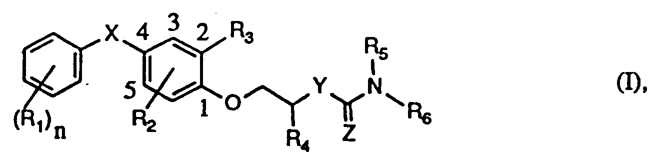 où
R₁ signifie un halogène, C₁-C₃-alkyle, halogène-C₁-C₃-alkyle, C₁-C₃-alcoxy, halogène-C₁-C₃-alcoxy ou cyano et/ou 2 substituants R₁ liés à l'atome de carbone adjacent du cycle phényle signifient ensemble -O-CH₂-O-;
R₂ signifie l'hydrogène, un halogène ou un groupe méthyle;
R₃ signifie le fluor, le chlore, le brome ou un groupe C₁-C₃-alkyle;
R₄ signifie l'hydrogène ou un groupe C₁-C₃ -alkyle;
R₅ signifie soit l'hydrogène, un groupe C₁-C₄-alkyle, 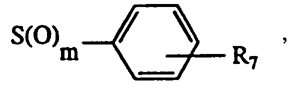 -COCO-R₈, -COR₉ ou bien S(O)ₘ-N(R₁₀)-COO-R₁₁ et
R₆ signifie un groupe C₁-C₆-alkyle, halogène-C₁-C₄-alkyle, C₂-C₆-alcényle, halogène-C₃-C₄-alcényle, C₃-C₅-alcynyle, halogène-C₃-C₅-alcynyle, C₁-C₃-alcoxy-C₁-C₃-alkyle, C₃-C₆-cycloalkyle,
phényle non substitué ou phényle qui est mono- à trisubstitué par des substituants identiques ou différents choisis parmi un groupe comprenant un halogène, un groupe C₁-C₄-alkyle et C₁-C₄-alcoxy;
ou bien R₅ et R₆ signifient ensemble -(CH₂)₄- ou -(CH₂)₅-;
R₇ signifie l'hydrogène, un halogène ou un groupe C₁-C₃-alkyle;
R₈ signifie un groupe C₁-C₈-alcoxy ou -N(R₁₂)₂;
R₉ signifie un groupe C₁-C₄-alkyle, C₃-C₆-cycloalkyle, -N(R₁₃)₂ ou C₁-C₄-alcoxy;
R₁₀ signifie un groupe C₁-C₃-alkyle;
R₁₁ signifie un groupe C₁-C₆-alkyle;
les symboles R₁₂ signifient indépendamment l'un de l'autre un groupe C₁-C₈-alkyle;
les symboles R₁₃ signifient indépendamment l'un de l'autre un groupe C₁-C₄-alkyle;
m signifie 0, 1 ou 2;
n signifie 0, 1, 2 ou 3, lorsque n signifie 2 ou 3, le reste R₁ pouvant être identique ou différent;
X signifie O, S, CH₂, CO ou -O-CH₂-;
Y signifie O ou S; et
Z signifie O ou S,
à l'exception du 1-(2-fluoro-4-phénoxy-phénoxy)-2-éthylaminocarbonyloxy-éthane, sous forme libre ou sous forme d'un sel.

2. Un composé de formule I selon la revendication 1, où (R₁)ₙ signifie (R₁)₀, monofluoro, monochloro, monobromo, difluoro, dichloro, monochloromonofluoro, monobromo-monofluoro, monométhyle, monoéthyle, diméthyle, monométhoxy, monocyano, monotrifluorométhyle, monotrifluorométhoxy ou monométhylènedioxy.

3. Un composé de formule I selon la revendication 1 ou 2, où R₂ signifie l'hydrogène le chlore ou un groupe méthyle.

4. Un composé de formule I selon l'une des revendications 1 à 3, où R₃ signifie le chlore, le brome ou un groupe méthyle.

5. Un composé de formule I selon l'une des revendications 1 à 3, où R₃ signifie le fluor.

6. Un composé de formule I selon l'une des revendications 1 à 5, où R₄ signifie l'hydrogène.

7. Un composé de formule I selon l'une des revendications 1 à 6, où R₅ signifie l'hydrogène, un groupe C₁-C₄-alkyle, -S-C₆H₄-Cl, -COCO-R₈, où R₈ signifie un groupe C₁-C₄-alcoxy, ou -CO-R₉ où R₉ signifie un groupe C₁-C₄-alkyle, C₁-C₄-alcoxy ou C₃-C₆-cycloalkyle.

8. Un composé de formule I selon l'une des revendications 1 à 7, où R₆ signifie un groupe C₁-C₆-alkyle, halogène-C₁-C₃-alkyle, C₂-C₄-alcényle, halogène- C₃-C₄-alcényle, C₃-C₄-alcynyle, C₁-C₂-alcoxy-C₁-C₂-alkyle ou C₃-C₆-cycloalkyle.

9. Un composé de formule I selon l'une des revendications 1 à 6, où R₅ et R₆ signifient ensemble -(CH₂)₄- ou (CH₂)₅-.

10. Un composé de formule I selon l'une des revendications 1 à 9, où X signifie O, CH₂, CO ou -O-CH₂-.

11. Un composé de formule I selon l'une des revendications 1 à 10, où Y signifie O.

12. Un composé de formule I selon l'une des revendications 1 à 11, où Z signifie O.

13. Un composé de formule I selon la revendication 1, où (R₁)ₙ signifie (R₁)₀, 2-, 3- ou 4-fluoro, 3- ou 4-chloro, 4-bromo, 2,4- ou 3,5-difluoro, 3,4- ou 3,5-dichloro, 3-chloro-4-fluoro, 4-bromo-2-fluoro, 3- ou 4-méthyle, 3- ou 4-éthyle, 3,5-diméthyle, 3- ou 4-méthoxy, 4-cyano, 3- ou 4-trifluorométhyle, 3- ou 4-trifluorométhoxy ou 3,4-méthylènedioxy, R₂ signifie l'hydrogène, le chlore ou un groupe méthyle, R₃ signifie le fluor, le chlore, le brome ou un groupe méthyle, R₄ signifie l'hydrogène, R₅ signifie soit l'hydrogène, un groupe C₁-C₄-alkyle, 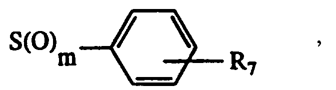 -COCO-R₈ ou -CO-R₉ et R₆ signifie un groupe C₁-C₆-alkyle, halogène-C₁-C₃-alkyle, C₂-C₄-alcényle, C₁-C₄-alcynyle, C₁-C₂-alcoxy-C₁-C₂-alkyle ou signifie simplement un groupe phényle substitué par un halogène; ou bien R₅ et R₆ signifient ensemble -(CH₂)₄- ou -(CH₂)₅-; X signifie O, CH₂, CO ou bien -O-CH₂-; Y signifie O ou S, Z signifie O ou S, R₇ signifie l'hydrogène ou un halogène; R₈ signifie un groupe C₁-C₄-alcoxy; R₉ signifie un groupe C₁-C₄-alkyle ou C₃-C₆-cycloalkyle et m signifie 0 ou 1.

14. Un composé de formule I selon la revendication 13, où (R₁)ₙ signifie (R₁)₀, 3- ou 4-fluoro, 3-chloro, 3,5-difluoro ou 3,5-dichloro, R₂ signifie l'hydrogène, R₃ signifie le chlore, R₄ signifie l'hydrogène, R₅ signifie soit l'hydrogène et R₆ signifie un groupe C₁-C₃-alkyle, halogène-C₁-C₂-alkyle, C₂-C₄-alcényle, C₃-C₄-alcynyle, C₁-C₂-alcoxy-C₁-C₂-alkyle, ou signifie simplement un groupe phényle substitué par le chlore; ou bien R₅ et R₆ signifient ensemble -(CH₂)₄-; X signifie O, CH₂, CO ou bien -O-CH₂-; Y signifie O ou S et Z signifie O ou S.

15. Un composé de formule I selon la revendication 14, où (R₁)ₙ signifie (R₁)₀, R₂ signifie l'hydrogène, R₃ signifie le fluor, R₄ signifie l'hydrogène, R₅ signifie l'hydrogène, R₆ signifie un groupe C₁-C₃-alkyle et X, Y et Z signifient chacun O.

16. Un composé de formule I selon la revendication 15, où (R₁)ₙ signifie (R₁)₀, 3-chloro ou 3,5-difluoro, R₂ signifie l'hydrogène, R₃ signifie le chlore, R₄ signifie l'hydrogène, R₅ signifie l'hydrogène, R₆ signifie un groupe C₁-C₂-alkyle ou chloro-C₁-C₂-alkyle et X, Y et Z signifient chacun O.

17. Un composé de formule I selon la revendication 1, caractérisé en ce qu'il est choisi parmi le groupe de composés suivant:
le 1-[2-chloro-4-(3-chlorophénoxy)-phénoxy]-2-éthylaminocarbonyloxy-éthane,
le 1-(2-chloro-4-phénoxy-phénoxy)-2-éthylaminocarbonyloxy-éthane, et
le 1-[2-chloro-4-(3,5-difluorophénoxy)-phénoxy]-2-ethylaminocarbonyloxy-éthane.

18. Un procédé de préparation d'un composé de formule I, à l'exception du 1-(2-fluoro-4-phénoxy-phénoxy)-2-éthylaminocarbonyloxy-éthane, sous forme libre ou sous forme d'un sel, caractérisé en ce qu'on fait réagir
(a) un composé de formule 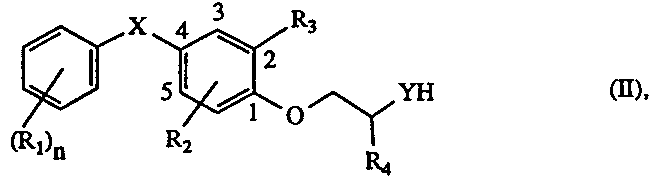 où R₁,R₂, R₃, R₄, n, X et Y ont les significations indiquées dans la formule I, ou un de ses sels, avec un composé de formule
L-C(=Z)-N(R₅)R₆ (III),
où R₅, R₆ et Z ont les significations indiquées dans la formule I et L signifie un groupe éliminable, ou un de ses sels, ou bien
(b) pour préparer un composé de formule I où R₅ signifie l'hydrogène, ou un de ses sels, on fait réagir un composé de formule (II) ou un de ses sels, avec un composé de formule
R₆N=C=Z (IV),
où R₆ et Z ont les significations indiquées dans la formule I, ou bien
(c) on fait réagir un composé de formule 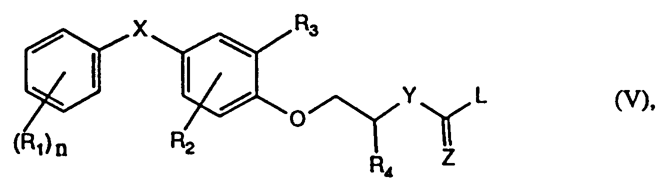 où R₁,R₂, R₃, R₄, n, X, Y et Z ont les significations indiquées dans la formule I et L signifie un groupe éliminable, ou un de ses sels, avec un composé de formule
H-N(R₅)R₆ (VI),
où R₅ et R₆ ont les significations indiquées dans la formule I, ou un de ses sels, ou bien
(d) on fait réagir un composé de formule 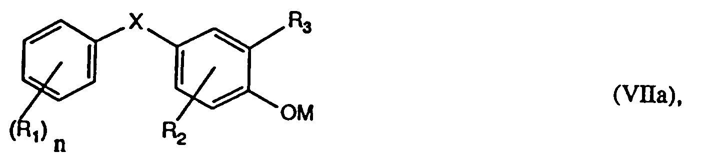 où R₁,R₂, R₃, n et X ont les significations indiquées dans la formule I et M signifie un cation, avec un composé de formule 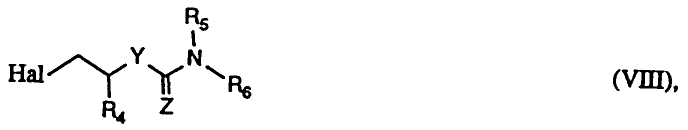 où R₄, R₅, R₆, Y et Z ont la signification indiquée dans la formule I et Hal signifie un atome d'halogène,
et/ou, lorsque cela est nécessaire, on transforme un composé de formule I, sous forme libre ou sous forme d'un sel, obtenu selon le procédé ou d'une autre manière, en un autre composé de formule I, on sépare un mélange d'isomères obtenu selon le procédé et on isole l'isomère souhaité et/ou on transforme un composé libre de formule I obtenu selon le procédé ou d'une autre manière, en un sel, ou bien on transforme un sel d'un composé de formule I obtenu selon le procédé ou d'une autre manière, en un composé libre de formule I ou en un autre sel.

19. Un pesticide, caractérisé en ce qu'il contient comme matière active au moins un composé de formule I selon la revendication 1, à l'exception du 1-(2-fluoro-4-phénoxy-phénoxy)-2-éthylaminocarbonyloxy-éthane, sous forme libre ou sous forme d'un sel acceptable en chimie agricole et éventuellement au moins un produit auxiliaire.

20. Un pesticide selon la revendication 19, pour lutter contre les insectes et les représentants de l'ordre des acariens.

21. Un procédé de préparation d'un pesticide selon la revendication 19 ou 20 contenant au moins un produit auxiliaire, caractérisé en ce qu'on mélange intimement et/ou on broie la matière active avec le ou les produits auxiliaires.

22. Un procédé de lutte contre des organismes nuisibles, caractérisé en ce qu'on applique un pesticide selon la revendication 19 ou 20 sur les organismes nuisibles ou sur leur environnement.

23. Un procédé selon la revendication 22 de lutte contre les insectes et les représentants de l'ordre des acariens.

24. Un procédé selon la revendication 22 ou 23, pour la protection des organes de propagation des plantes, caractérisé en ce qu'on traite l'organe de propagation ou la zone dans laquelle l'organe de propagation est planté.

25. Un organe de propagation des plantes traité selon le procédé décrit à la revendication 24.

26. Un composé de formule 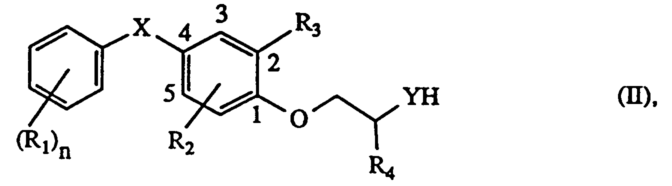 où R₁,R₂, R₃, R₄, n, X et Y ont les significations indiquées dans la formule I, à l'exception du 2-(2-fluoro-4-phénoxy-phénoxy)-éthanol, sous forme libre ou sous forme d'un sel.

27. Un procédé de préparation d'un composé de formule II selon la revendication 26, sous forme libre ou sous forme d'un sel, caractérisé en ce que
(e) on fait réagir un composé de formule (VIIa) ou 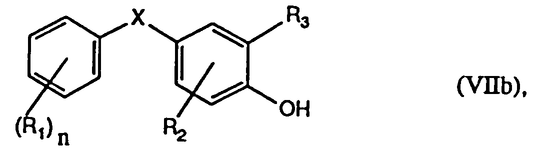 où R₁,R₂, R₃, X et n ont la signification indiquée dans la formule I, en présence d'un catalyseur d' alkylation avec un composé de formule 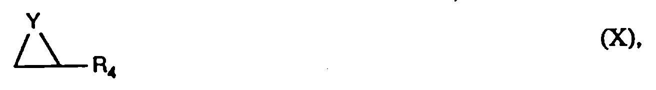 où R₄ et Y ont les significations indiquées dans la formule I, ou bien
(f) pour la préparation d'un composé de formule II où R₄ signifie l'hydrogène et Y signifie O ou bien d'un de ses sels, on fait réagir un composé de formule (VIIa) ou (VIIb) avec du 2-oxo-1,3-dioxolane, ou bien
g) pour la préparation d'un composé de formule II où R₄ signifie l'hydrogène et Y signifie O, ou d'un de ses sels, on fait réagir un composé de formule 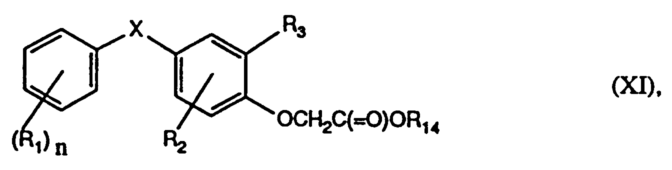 où R₁,R₂, R₃, n et X ont les significations indiquées dans la formule I et R₁₄ signifie un groupe C₁-C₈-alkyle, avec un agent réducteur,
et/ou, lorsque cela est nécessaire, on transforme un composé de formule II sous forme libre ou sous forme d'un sel obtenu selon le procédé ou d'une autre manière, en un autre composé de formule II, on sépare un mélange d' isomères obtenu selon le procédé et on isole l'isomère souhaité et/ou on transforme un composé libre de formule II obtenu selon le procédé ou d'une autre manière, en un sel, ou bien on transforme un sel d'un composé de formule II obtenu selon le procédé ou d'une autre manière, en un composé libre de formule II ou en un autre sel.
